# EUROPEAN PATENT APPLICATION

(11) **EP 3 750 989 A1**
(43) Date of publication of application: **16.12.2020**
(21) Application number: 19209826.7
(22) Date of filing: 18.11.2019
(51) Int. Cl.: C12N 9/04, C12N 9/12, C12N 9/88, C12N 9/90, C12N 15/52, C12P 7/22, C12P 7/42, C12P 17/06

(54) **PRODUCTION OF PLANT-BASED ACTIVE SUBSTANCES (E.G. CANNABINOIDS) BY RECOMBINANT MICROORGANISMS**

(30) Priority: 20.02.2019 EP 19158324; 14.03.2019 WO PCT/EP2019/056412
(71) Applicant: Synbionik GmbH, 60314 Frankfurt am Main (DE)
(72) Inventor: SCHMITT, Patrick, 55122 Mainz (DE); TABOADA, Andrea, 60439 Frankfurt am Main (DE)
(74) Representative: Kilger, Ute

(57) **Abstract**

A genetically modified bacteria transformed to have an enhanced glycolosis pathway achieved through incorporation of promoters and specific sequences for the simultaneous utilization of the Entner-Doudoroff (ED) pathway and Embden-Meyerhof-Parnas (EMP) pathway. The resultant molecules from both pathways are redirected, as a consequence of additional genetical modifications to the Zymomonas species, for the production of cannabinoids. Therefore, the resulting non-naturally occurring bacterium comprises a set of sequences for an enhanced and efficient production of a multitude of cannabinoids. Also, a method for the utilization of the non-naturally modified bacterium for the production of different cannabinoids in a batch form for a wider range of productions sizes.

## Description

### A) TECHNICAL FIELD

The present invention describes the genetic modification of the gram-negative bacterium *Zymomonas mobilis*, for the biosynthetic production of cannabinoids, cannabinoid acids or precursors thereof from D-glucose. The invention also concerns methods for the production of cannabinoids, cannabinoid acids or precursors thereof, that make use of the bacterial strain according to the invention.

### B) BACKGROUND

The demand for cannabinoids for medical purposes is steadily increasing worldwide. A large part of the cannabinoids on the market is currently being obtained by carbon dioxide extraction after cultivation of plants of the genus *Cannabis* as described in DE 100 51 427 C1. The high costs of cultivation in compliance with regulatory requirements, low yields and complex separation procedures are reflected in enormous treatment costs for patients with standardized cannabinoid therapies. This development establishes a need for alternative production methods for medically relevant cannabinoids. In addition to pure extraction from plant material, a large number of processes for the chemical synthesis of cannabinoids are therefore also being used. Exemplarily, reference is made to US 10/479,021, US 12/770,056 and US 10/954,345. Complete chemical synthesis on an industrial scale, however, requires the purchase of partly cost-intensive specialty chemicals as well as the construction of highly specific production lines. In contrast, the biosynthesis of cannabinoids is currently the most cost-effective and practicable solution. Cannabinoids were already able to be produced in this way in yeast (cf. US 14/795,816 and Zirpel et al. (2017, doi: 10.1016/j.jbiotec.2017.07.008) "Engineering yeasts as platform organisms for cannabinoid biosynthesis") and plant cell culture as described in WO 2018/176055 A2. According to the current state of the art, brewer's yeast, *Saccharomyces cerevisiae,* is regarded as platform organism for the synthesis of cannabinoids (cf. Carvalho et al. (2017, doi: 10.1093/femsyr/fox037) "Designing microorganisms for heterologous biosynthesis of cannabinoids". Biosynthesis strategies of cannabinoids are based on the synthesis from the intermediate products geranyl pyrophosphate (GPP) and olivetolic acid (OA); in connection with the invention, these and other intermediate products of the steps of cannabinoid biosynthesis described in more detail below are also referred to as precursors of cannabinoids. The accumulation of the two intermediate products (precursors) GPP and olivetolic acid and their enzymatic conversion to cannabinoids by enzymes from the cannabis plant are decisive for the efficiency and yield of the biosynthesis.

### C) BRIEF DESCRIPTION OF THE INVENTION

The subject matter of the present invention is a modified strain of the bacterial species *Zymomonas mobilis*, which in comparison to the already-established synthesis organism, provides a higher yield in the synthesis of cannabinoids, cannabinoid acids or precursors thereof. Since it is a prokaryote it is easier to cultivate than eukaryotic organisms, such as *Saccharomyces cerevisiae.* In addition, the invention includes methods for the production of cannabinoids, cannabinoid acids or precursors thereof from D-glucose that make use of the bacterial strain according to the invention. Said strain is a non-naturally occurring strain.

Aspects of the present invention are summarized in the following list of embodiments 1 to according to the invention, with the designation of each of the enzymes in all of these embodiments including any isoforms of the enzyme:
1. Modified non-naturally occurring strain of the bacterial species *Zymomonas mobilis*, characterized in that the strain
   a) Is modified to contain the genes coding for the enzymes *aldolase* and *triose-phosphate isomerase,*
   b) has an inactivation of the gene coding for the enzyme *pyruvate decarboxylase* (preferably *pdc*)*,*
   c) has an inactivation of the gene coding for the enzyme *lactate dehydrogenase* (preferably *ddh*)*,*
   d) has an inactivation of the gene coding for the enzyme *phosphoenolpyruvate carboxylase* (preferably *ppc*)*,* and
   e) contains the gene coding for the enzyme *hexokinase* (preferably *glucokinase)* and has an increased activity of the enzyme *hexokinase* (preferably *glucokinase)* compared to a non-modified strain.

In the modified non-naturally occurring strain, the genes coding for the enzymes *aldolase* and *triose-phosphate isomerase* result in two simultaneously occurring glycolysis pathways taking place, namely via the Entner-Doudoroff (ED) pathway as well as via the Embden-Meyerhof-Parnas (EMP) pathway, in which the intermediate products pyruvate (CAS No. 127-17-3), glyceraldehyde 3-phosphate (CAS No. 591-59-3) and acetyl-CoA (CAS-No. 85-61-0) are formed from D-glucose (CAS No. 50-99-7). Preferably, the enzyme aldolase is EC No 4.1.2.13 and/or the enzyme triose-phosphate isomerase is EC No. 5.3.1.1. In a particularly preferred embodiment, the gene coding for the enzyme aldolase is the gene with the gene ID: 33950769 and/or the gene coding for the enzyme triose-phosphate isomerase is the gene having the gene ID: 29953556.

In the modified non-naturally occurring strain, the inactivation of the gene coding for the enzyme *pyruvate decarboxylase* (preferably *pdc*) results in no enzymatic conversion of pyruvate (CAS No. 127-17-3) to acetaldehyde (CAS No. 75-07-0) taking place. Preferably, the enzyme pyruvate decarboxylase is EC No: 4.1.1.1. In a particularly preferred embodiment, the pdc gene coding for the enzyme pyruvate decarboxylase is the gene with the gene ID: 33073732.

In the modified non-naturally occurring strain, the inactivation of the gene coding for the enzyme *lactate dehydrogenase* (preferably *ddh*) results in no enzymatic conversion of pyruvate (CAS No. 127-17-3) to lactate (CAS No. 113-21-3) taking place. Preferably, the enzyme *lactate dehydrogenase* is EC No. 1.1.1.28. In a particularly preferred embodiment, the ddh gene coding for the enzyme *lactate dehydrogenase* is the gene with the gene ID: 33072845.

In the modified non-naturally occurring strain, the inactivation of the gene coding for the enzyme *phosphoenolpyruvate carboxylase* (preferably *ppc*) results in no enzymatic conversion of phosphoenolpyruvate (CAS No. 73-89-2) to oxaloacetate (CAS No. 328-42-7) taking place. Preferably, the enzyme phosphoenolpyruvate carboxylase is EC No. 4.1.1.31. In a particularly preferred embodiment, the ppc gene coding for the enzyme phosphoenolpyruvate carboxylase is the gene with the gene ID: 33072979.

In the modified non-naturally occurring strain, the gene coding for the enzyme *glucokinase* or the increased activity of the enzyme *glucokinase* results in a quantitatively increased conversion of D-glucose (CAS No. 50-99-7) to glucose 6-phosphate (CAS No. 56-73-5) taking place, compared to a non-modified strain. Preferably, the enzyme hexokinase is EC No. 2.7.1.1 or the enzyme glucokinase is EC No. 2.7.1.2. In a particularly preferred embodiment, the gene coding for the enzyme *glucokinase* is the gene with the gene ID: 33073808.
2. Modified non-naturally occurring strain according to embodiment 1, characterized in that the inactivation of the genes is induced by genetic engineering or generated by selection, the inactivation preferably being a permanent inactivation.
3. Modified non-naturally occurring strain according to embodiment 1 or 2, characterized in that the strain is transformed with the genes coding for the enzymes *aldolase, triose-phosphate isomerase* and/or *glucokinase.*
4. Modified non-naturally occurring strain according to one of the embodiments 1 to 3, characterized in that the modified strain, in comparison with a non-modified strain, has an overexpression of the gene coding for the enzyme *hexokinase* (preferably *glucokinase),* preferably mediated by transformation with the gene and/or modification of an expression regulation sequence of the gene.
5. Modified non-naturally occurring strain according to one of the embodiments 1 to 4, characterized in that the strain contains the genes coding for the enzymes
   a) *Deoxy-xylulose-phosphate synthase*
   *b) 1-deoxy-D-xylulose-5-phosphate reductoisomerase*
   *c) Cytidine diphosphate methylerythritolsynthase*
   *d) Cytidylmethyl kinase*
   *e) Methyl-erythritol cyclo-diphosphate synthase*
   *f) Hydroxy-methyl-butenyl diphosphate synthase*
   *g) IPP*/*DMAPP synthase*
   *h) Isopentenyl-diphosphate isomerase* and
   *i) Geranyl pyrophosphate synthase.*
      In the modified non-naturally occurring strain, these genes coding for the enzymes a) to i) result in an enzymatic conversion of pyruvate (CAS No. 127-17-3) and glyceraldehyde 3-phosphate (CAS No. 591-59-3) to geranyl pyrophosphate (CAS No. 763-10-0) taking place via several intermediate steps. The enzymes a) to i) are preferably the following enzymes:
      a) *Deoxy-xylulose-phosphate synthase:* EC No: 2.2.1.7
      *b) 1-deoxy-D-xylulose-5-phosphate reductoisomerase:* EC No. 1.1.1.267
      *c) Cytidine diphosphate methylerythritolsynthase:* EC No. 2.7.7.60
      *d) Cytidylmethyl kinase:* EC No. 2.7.1.148
      *e) Methyl-erythritol cyclo-diphosphate synthase:* EC No. 4.6.1.12
      *f) Hydroxy methyl butenyl diphosphate synthase:* EC No. 1.17.7.1
      *g) IPP*/*DMAPP synthase:* EC No. 1.17.1.2
      *h) Isopentenyl-diphosphate isomerase:* EC No. 5.3.3.2 and/or
      *i) Geranyl pyrophosphate synthase:* EC No. 2.5.1.1.
      In a particularly preferred embodiment, the genes coding for the enzymes a) to i) are the genes with the following gene ID numbers:
      a) Gene ID: 29954322 for *deoxy-xylulose-phosphate synthase*
      *b)* Gene ID: 29954627 for *1-deoxy-D-xylulose-5-phosphate reductoisomerase*
      c) Gene ID: 29952840 for *cytidine diphosphate-methylerythritol synthase*
      *d)* Gene ID: 29954344 for *cytidylmethyl kinase*
      e) Gene ID: 29952840 for *methyl-erythritol cyclo-diphosphate synthase*
      *f)* Gene ID: 29952365 for *hydroxy-methyl-butenyl-diphosphate synthase*
      *g)* Gene ID: 17279806 for the *IPP*/*DMAPP synthase*
      *h)* Gene ID: 8154321 for *isopentenyl-diphosphate isomerase* and/or
      *i)* Gene ID: 888334 for *geranyl pyrophosphate synthase.*
6. Modified non-naturally occurring strain according to one of the embodiments 1 to 5, characterized in that the strain is transformed with the genes coding for the enzymes
   a) *Deoxy-xylulose-phosphate synthase*
   *b) 1-deoxy-D-xylulose-5-phosphate reductoisomerase*
   *c) Cytidine diphosphate methylerythritolsynthase*
   *d) Cytidylmethyl kinase*
   *e) Methyl-erythritol cyclo-diphosphate synthase*
   *f) Hydroxy-methyl-butenyl diphosphate synthase*
   *g) IPP*/*DMAPP Synthase*
   *h) Isopentenyl-diphosphate isomerase* and/or
   i) *Geranyl pyrophosphate synthase.*
7. Modified non-naturally occurring strain according to one of embodiments 1 to 6, characterized in that the strain contains the genes coding for the enzymes
   *a) Acetyl-CoA carboxylase*
   *b) Acetyl-CoA acetyltransferase*
   *c) FadB hydroxyacyl-CoA dehydrogenase*
   *d) FadB enoyl-CoA hydratase*
   *e) Trans-2-enoyl-CoA reductase*
   *f) Olivetolsynthase* and
   *g) Olivetolic acid cyclase.*
      In the modified non-naturally occurring strain, these genes coding for enzymes a) to g) result in an enzymatic conversion of acetyl-CoA (CAS No. 85-61-0) to olivetolic acid (CAS No. 491-72-5) taking place via several intermediate steps. Preferably, the enzymes a) to g) are the following enzymes:
      *a) Acetyl-CoA carboxylase:* EC No. 6.4.1.2
      *b) Acetyl-CoA acetyltransferase:* EC No. 2.3.1.9
      *c) FadB Hydroxyacyl-CoA Dehydrogenase:* EC No. 5.1.2.3
      d) *FadB Enoyl-CoA Hydratase:* EC No. 4.2.1.17
      *e) Trans-2-enoyl-CoA Reductase:* EC No. 1.3.1.44
      *f) Olivetol Synthase:* EC No. 2.3.1.206 and/or
      *g) Olivetolic Acid Cyclase:* EC No. 4.4.1.26.
      In a particularly preferred embodiment, the genes coding for the enzymes a) to g) are the genes with the following gene ID numbers:
      a) Gene ID: 33073499 and 33072760 for the *acetyl-CoA carboxylase*
      *b)* Gene ID: 34309143 for the *acetyl-CoA acetyltransferase*
      c) Gene ID: 948336 for the *FadB hydroxyacyl-CoA dehydrogenase*
      *d)* Gene ID: 4714532 for the *FadB Enoyl-CoA hydratase*
      e) GeneBank: AY741582.1 for the trans-2-enoyl-CoA reductase
      *f)* GeneBank: AB164375.1 for *olivetol synthase* and/or
      *g)* GeneBank: AFN42527.1 for *olivetolic acid cyclase.*
8. Modified non-naturally occurring strain according to one of the embodiments 1 to 7, characterized in that the strain is transformed with the genes coding for the enzymes
   *a) Acetyl-CoA Carboxylase*
   *b) Acetyl-CoA acetyltransferase*
   *c) FadB Hydroxyacyl-CoA Dehydrogenase*
   *d) FadB Enoyl-CoA Hydratase*
   *e) Trans-2-enoyl-CoA reductase*
   *f) Olivetol synthase* and/or
   g) *Olivetolic acid cyclase.*
9. Modified non-naturally occurring strain according to one of the embodiments 1 to 8, characterized in that the strain contains the gene coding for the enzyme *geranyl-pyrophosphate-olivetolic acid geranyltransferase,* the strain preferably being transformed with this gene. In the modified strain, the gene coding for the enzyme *geranyl-pyrophosphate-olivetolic acid geranyltransferase* results in a conversion of olivetolic acid (CAS No. 491-72-5) and geranyl pyrophosphate (CAS No. 763-10-0) to cannabigerolic acid (CAS No. 25555-57-1) taking place. Preferably, the enzyme geranyl-pyrophosphate-olivetolic acid geranyltransferase is EC No. 2.5.1.102. In a particularly preferred embodiment, the gene coding for the enzyme *geranyl-pyrophosphate-olivetolic acid geranyltransferase* is the gene with the GenBank ID: AWW43729.1.
10. Modified non-naturally occurring strain according to one of the embodiments 1 to 9, characterized in that the strain contains the gene coding for the enzyme *cannabidiolic acid synthase,* the strain preferably being transformed with this gene. In the modified strain, the gene coding for the enzyme *cannabidiolic acid synthase* results in an enzymatic conversion of cannabigerolic acid (CAS No 25555-57-1) to cannabidiolic acid (CAS No 1244-58-2) taking place. Preferably, the enzyme cannabidiolic acid synthase is EC No. 1.21.3.8. In a particularly preferred embodiment, the gene coding for the enzyme *cannabidiolic acid synthase* is the gene with the GenBank ID: AB292682.1.
11. Modified non-naturally occurring strain according to one of the embodiments 1 to 10, characterized in that the strain contains the gene coding for the enzyme *tetrahydrocannabinolic acid synthase,* the strain preferably being transformed with this gene. In the modified strain, the gene coding for the enzyme *tetrahydrocannabinolic acid synthase* results in an enzymatic conversion of cannabigerolic acid (CAS No. 25555-57-1) to tetrahydrocannabinolic acid (CAS No. 23978-85-0) taking place. Preferably, the enzyme tetrahydrocannabinolic acid synthase is EC No. 1.21.3.7. In a particularly preferred embodiment, the gene coding for the enzyme *tetrahydrocannabinolic acid synthase* is the gene with the GenBank ID: AB057805.1.
12. Modified non-naturally occurring strain according to one of the embodiments 1 to 11, characterized in that the strain is transformed with the genes coding for one or more of the enzymes by means of a genetic vector system or several genetic vector systems.
13. Modified non-naturally occurring strain according to one of the embodiments 1 to 12, characterized in that the strain is a genetically engineered strain.
14. Modified non-naturally occurring strain according to one of the embodiments 1 to 13, characterized in that the strain is a strain for biosynthesis of cannabinoids, cannabinoid acids or precursors thereof from D-glucose, the biosynthesis preferably being *de-novo* biosynthesis.
   In the context of the invention, the intermediate products of the biosynthesis of cannabinoids or cannabinoid acids from D-glucose described here for the modified strain are referred to as precursors of cannabinoids or cannabinoid acids; in particular, olivetolic acid, geranyl pyrophosphate are precursors of cannabigerolic acid or other cannabinoids or cannabinoid acids.
15. Method for the production of cannabinoids, cannabinoid acids or precursors thereof from D-glucose which comprises the cultivation of a modified strain of the bacterial species *Zymomonas mobilis* according to one of the embodiments 1 to 14 as well as obtaining the cannabinoids, cannabinoid acids or precursors thereof from the bacterial culture.
16. Method according to embodiment 15 for the production of the cannabinoid cannabidiol, which comprises the cultivation of a modified strain according to embodiment 10 as well as obtaining the cannabinoid from the bacterial culture.
17. Method according to embodiment 15 for the production of the cannabinoid tetrahydrocannabinol, which comprises the cultivation of a modified strain according to embodiment 11 as well as obtaining the cannabinoid from the bacterial culture.
18. Method according to embodiment 16 or 17, wherein obtaining the cannabinoid comprises the disruption of the cultivated bacterium, the purification of an acid of the cannabinoid as well as the decarboxylation of the acid.

### D) DETAILED DESCRIPTION OF THE INVENTION

The subject matter of the present invention is a modified non-naturally occurring strain of the bacterial species *Zymomonas mobilis*, which provides a higher yield in the synthesis of cannabinoids, cannabinoid acids or precursors thereof and, it has the technical feature of double glycolysis, which is achieved by the simultaneous usage of the Entner-Doudoroff (ED) and the Embden-Meyerhof-Parnas (EMP) glycolysis pathways. In addition, the invention also includes processes for the production of cannabinoids, cannabinoid acids or precursors thereof from D-glucose that make use of the bacterial strain according to the invention.

### I. Zymomonas mobilis as Synthesis Organism

*Zymomonas mobilis* is a rod-shaped, gram-negative bacterium. *Zymomonas mobilis* is facultatively anaerobic and can be cultivated at pH values of 3.5 to 7.5. Also, it is tolerant to high ethanol concentrations (up to 16% ethanol concentration in the medium). Its energy is generated in the form of fermentation. In contrast to Saccharomyces cerevisiae, *Zymomonas mobilis* uses glucose via the Entner-Doudoroff (ED) pathway. Fermentation with *Zymomonas mobilis* is faster than with *Saccharomyces cerevisiae. Zymomonas mobilis* has a 5% higher ethanol yield due to lower sugar incorporation in the cell wall and only small amounts of carbon substrates are used for competing pathways. Moreover, the anaerobic ethanol yield of *Zymomonas mobilis* is 97 % of theoretical yield of glucose as carbon substrate and less than 1 % of the yield goes to solvent products other than ethanol or CO₂ (Yang 2009). By blocking the ethanol production in *Zymomonas mobilis*, a production rate of products increases. In *Zymomonas mobilis*, sugar transport does not take place via the phosphoenolpyruvate-dependent sugar phosphotransferase system as it is usual in other bacteria, but rather via a special hydrogen ion symport system. *Zymomonas mobilis* is a prokaryote that can take up the substrate glucose by facilitated diffusion into the cell.

In the following, exemplary modifications according to the invention of a bacterium of the species *Zymomonas mobilis* are described.

### II. Simultaneous Double Glycolysis and Triple-gene-knockout

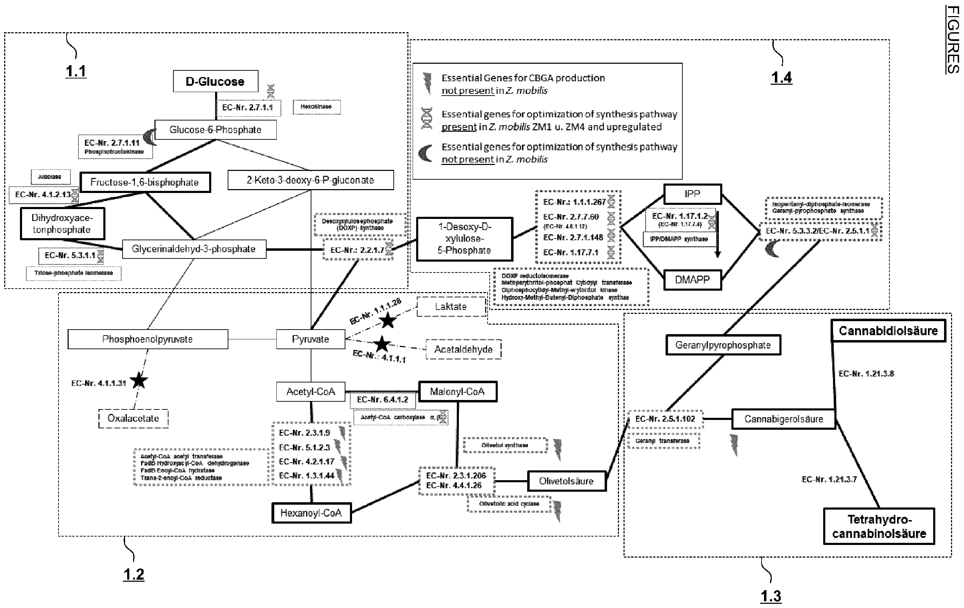

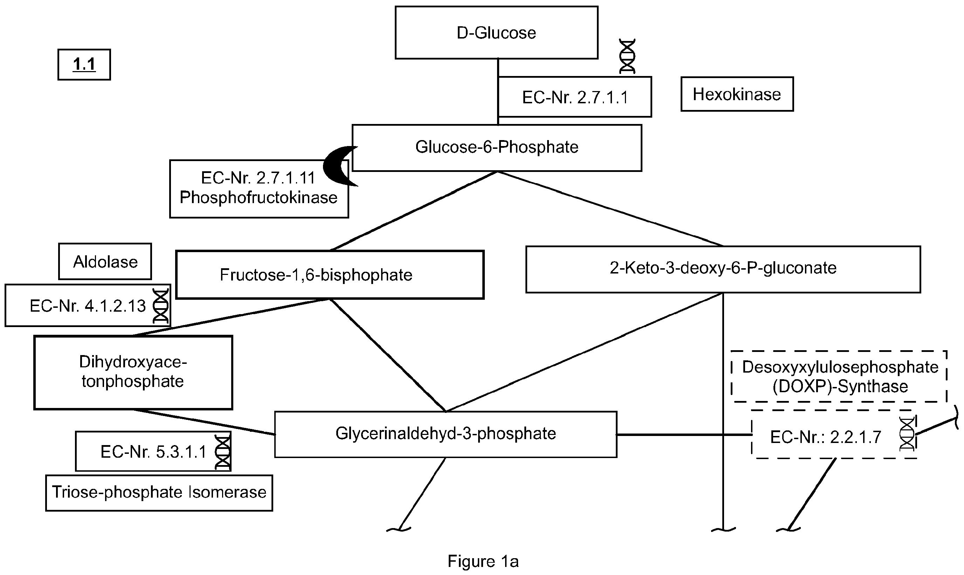

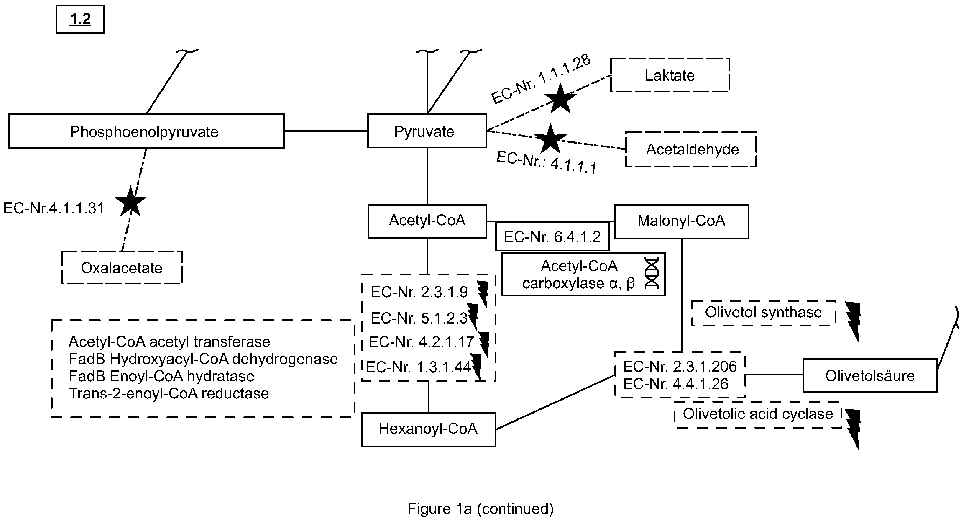

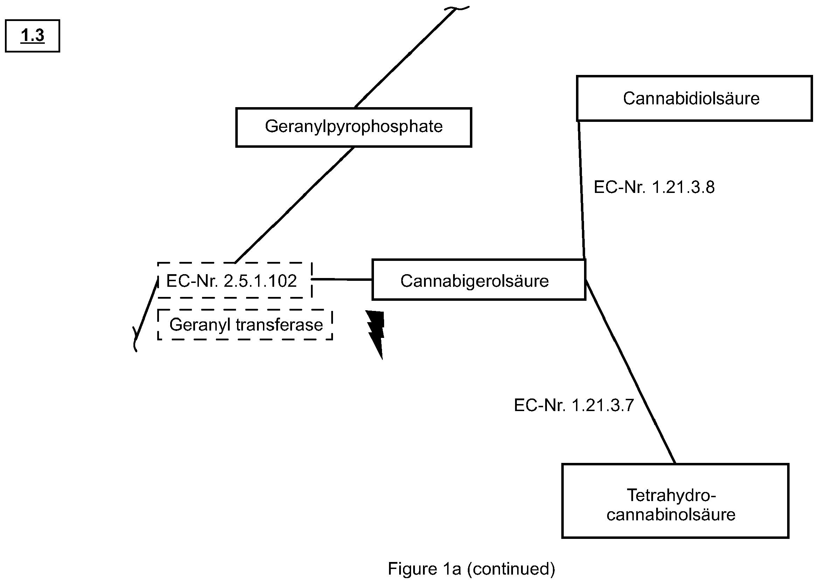

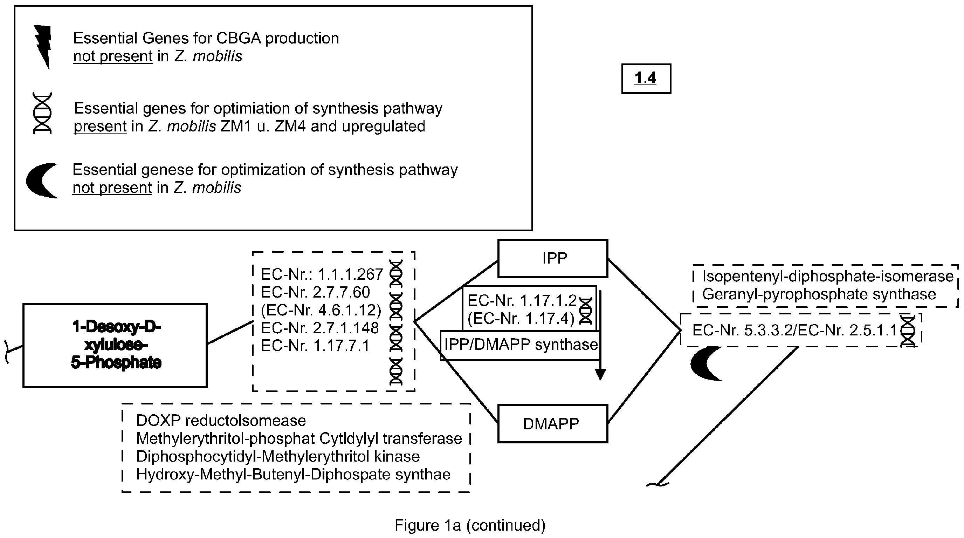

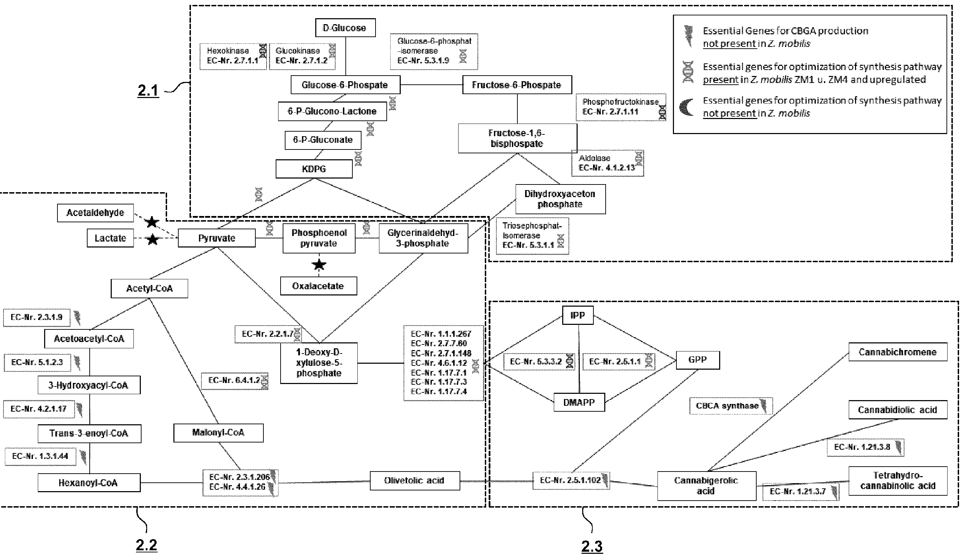

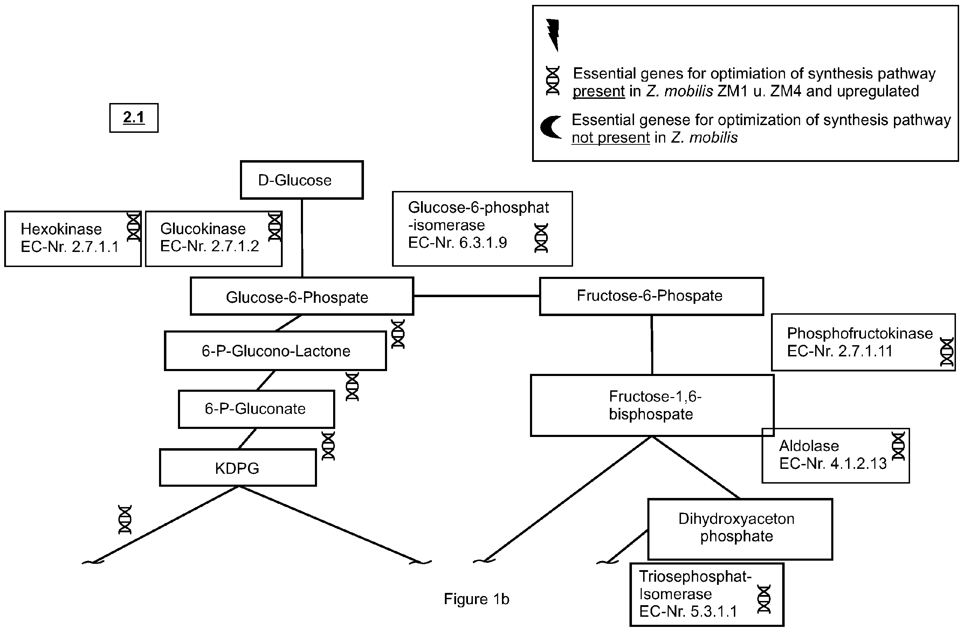

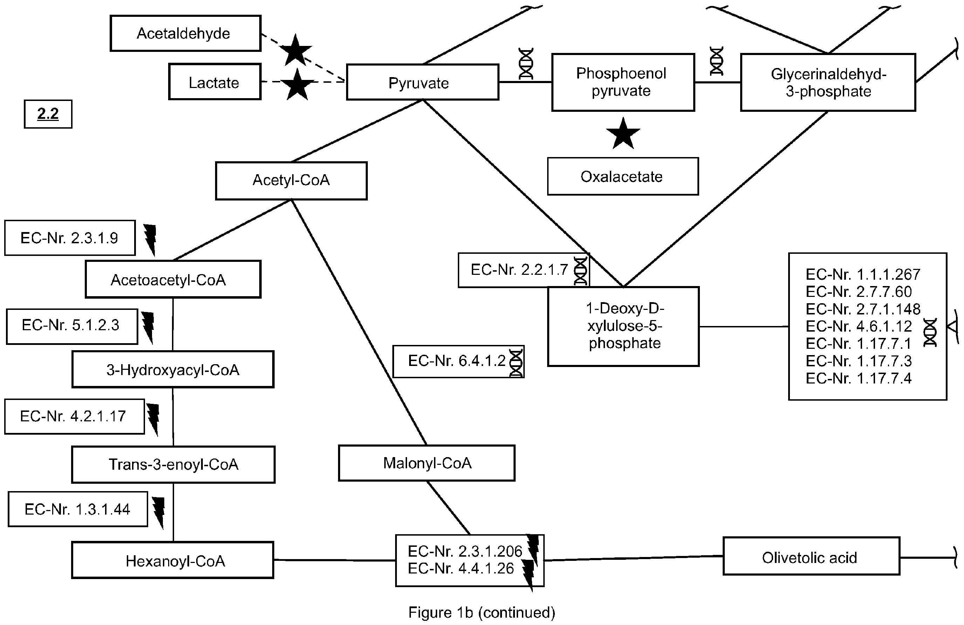

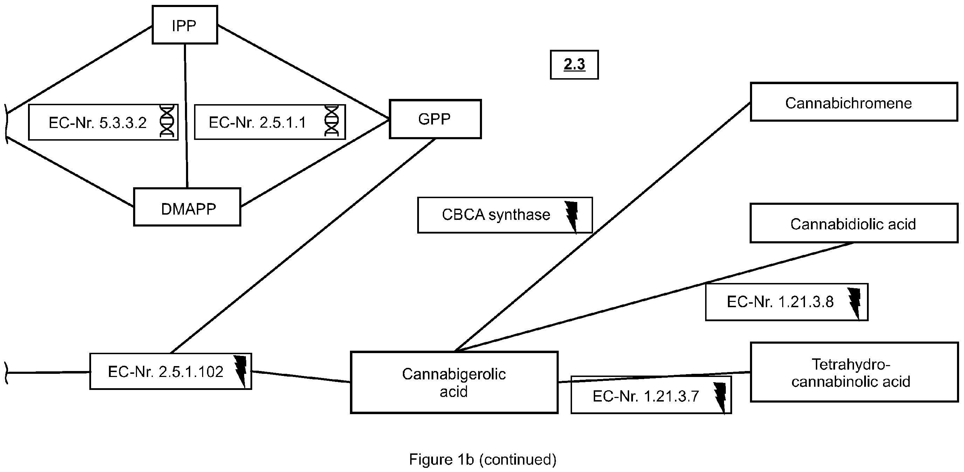

The modified non-naturally occurring bacterium is characterized by having the technical feature of a genetically engineered double glycolysis pathway, namely the Entner-Doudoroff (ED) pathway occurring in the wild type of *Zymomonas mobilis* and the Emden-Meyerhoff-Parnas (EMP) pathway, which take place simultaneously. The conversion of D-glucose via the Emden-Meyerhoff-Parnas (EMP) pathway was achieved by the introduction of the genes coding for the enzymes aldolase and triose-phosphate isomerase and the enzymatic conversion of fructose-1,6-bisphosphate to glyceraldehyde 3-phosphate and the enzymatic conversion of dihydroxyacetone phosphate to glyceraldehyde 3-phosphate enabled thereby, respectively, using genetic engineering. Since the two glycolysis pathways branch off from one another at the intermediate product glucose 6-phosphate, the enzymatic conversion of glucose to glucose-6-phosphate is quantitatively increased by an overexpression of the enzyme hexokinase (preferably glucokinase) through the introduction of an genetic material. In addition, a triple knockout (TKO) was performed. To achieve a triple knockout, multiple genetic tools and protocols can be used in order to perform such modifications; for example, a bacteriophage lambda red (Khandelwal et al., 2018), enterobacteriophage RecET (Wu et al., 2017), homologous recombination, insertional mutation, Flp recombinase based methods, fusion PCR based methods and transposon mutagenesis (He et al., 2014), CRISPR/Cas9 Type II (Coa et al., 2016) and/or endogenous CRISPR/Cas system (Type I-F) (Zheng et al., 2019). Moreover, multiple protocols to modify *Zymomonas mobilis* are nowadays considered common knowledge such as the procedures described in K.-M. Pappas et al. 1997. Consequently, the genes coding for the enzymes phosphoenolpyruvate carboxylase, pyruvate decarboxylase and lactate dehydrogenase are thereby deleted from the genome of *Zymomonas mobilis.* As a result, no enzymatic conversions of phosphoenolpyruvate to oxaloacetate, of pyruvate to lactate and of pyruvate to acetaldehyde take place, compared to the wild type. The described modifications lead to an increased synthesi, or accumulation of the intermediate products (precursors) glyceraldehyde 3-phosphate, pyruvate and acetyl-CoA, which are involved for the synthesis of cannabinoids, cannabinoid acids or precursor. These and other features of the bacterium according to the invention described below are illustrated schematically in Figure 1a and Figure 1b.

### III. Geranyl Pyrophosphate (GPP) Synthesis

Terpenoid synthesis takes place in the plastids of the cannabis plant via the methylerythritol phosphate pathway and in the cytosol via the mevalonate pathway. *Zymomonas mobilis* contains the endogenous methylerythritol phosphate pathway (MEP-pathway) for GPP biosynthesis this can be further enhanced by the overexpression of MEP-pathway genes, knockout of negative regulators of the MEP-pathway or the suppression of competitive pathways. The synthesis of isopentenyl pyrophosphate (IPP; also known as isopentenyl diphosphate) and dimethylallyl pyrophosphate (DMAPP) takes place via the methylerythritol phosphate pathway according to the synthesis scheme found in *Plasmodium malariae* from the accumulated metabolic intermediate products (precursors) pyruvate and glyceraldehyde 3-phosphate. The modified non-naturally occurring strain is able to generate higher amounts of intermediate products, for example GPP, in the production strain. This ensures a higher flux of intermediate products and a more efficient biosynthesis of the end product (cannabinoids), resulting in higher yields.

The bacterial GPP synthesis is enhanced in the modified non-naturally occurring strain by introducing an additional GPP biosynthesis pathway. The additional GPP pathway is incorporated to produce enhanced levels of GPP, which is needed as a biosynthetic intermediate product for the biosynthesis of cannabinoids in further reactions.

The enhancement can either be based on the prokaryotic MEP pathway or the predominantly eukaryotic mevalonate pathway. It is ensured by the introduction of additional genes encoding for enzymes as part of the MEP-biosynthetic-pathway or for enzymes as part of the Mevalonate-Pathway into modified non-naturally occurring strain.

The genes coding for the enzymes deoxy-xylulose-phosphate synthase, 1-deoxy-D-xylulose 5-phosphate reductoisomerase, cytidine diphosphate methylerythritol synthase, cytidyl methyl kinase, methyl-erythritol cyclo-diphosphate synthase, hydroxy methyl butenyl diphosphate synthase and IPP/DMAPP synthase are introduced using genetic engineering, such that a conversion of pyruvate and glyceraldehyde 3-phosphate to isopentenyl pyrophosphate and dimethylallyl pyrophosphate takes place via the intermediate products 1-deoxy-D-xylulose-5-phosphate (DXP), 2C-methyl-D-erythritol (MEP), 4-diphosphocytidyl-2C-methyl-erythritol (CDP-ME), 4-diphosphocytidyl-2C-methyl-erythritol-2-phosphate (CDP-MEP), 2C-methyl-D-erythritol-2,4-cyclodiphosphate (MEcPP) and (E)-4-hydroxy-3-methylbut-2enyl diphosphate (HMB-PP). In the course of this conversion pathway, there is a concentration ratio of 5:1 for the products isopentenyl pyrophosphate and dimethylallyl pyrophosphate. In order to prevent a disproportionate accumulation of isopentenyl pyrophosphate and to simultaneously achieve a sufficient accumulation of dimethylallyl pyrophosphate, a gene coding for the enzyme IPP isomerase is introduced using genetic engineering, thereby inducing an enzymatic conversion of isopentenyl pyrophosphate to dimethylallyl pyrophosphate. For the conversion of isopentenyl pyrophosphate and dimethylallyl pyrophosphate to geranyl pyrophosphate (GPP), a gene coding for the enzyme geranyl pyrophosphate synthase (GPPS) is introduced using genetic engineering. The genetic material transformed into the modified non-naturally occurring bacterium comprises the genes for the MEP pathway: 1-deoxy-D-xylulose-5-phosphate synthase (Gene ID: 29954322 (EC-Nr: 2.2.1.7)), 1-deoxy-D-xylulose-5-phosphate (Gene ID: 29954627 (EC-Nr: 1.1.1.267)), bifunctional, 2-C-methyl-D-erythritol 2,4-cyclodiphosphate synthase (Gene ID: 29952840 (EC-Nr: 2.7.7.60; EC-Nr: 4.6.1.12)), 4-(cytidine 5'-diphospho)-2-C-methyl-D-erythritol kinase (Gene ID: 29954344 (ED-Nr: 2.7.1.148)), bifunctional 2-C-methyl-D-erythritol 4-phosphate cytidylyltransferase/2-C-methyl-D-erythritol 2,4-cyclodiphosphate synthase (Gene ID: 29952840 (EC-Nr: 2.7.7.60; EC-Nr: 4.6.1.12)), 4-hydroxy-3-methylbut-2-en-1-yl diphosphate synthase (Gene ID: 29952365 (EC-Nr: 1.17.7.1)), 4-hydroxy-3-methylbut-2-enyl diphosphate (Gene ID: 913428 (EC-Nr: 1.17.7.4)), Isopentenyl pyrophosphate isomerase (Gene ID: 8154321 (EC-Nr: 5.3.3.2)) and/or Geranylgeranyl pyrophosphate synthetase (Gene ID: 888334 (EC-Nr: 2.5.1.1)). For the Mevalonate upper pathway, the genetic material transformed into the modified non-naturally occurring bacterium comprises the genes ERG10, ERG13, tHMGR, mvaE, mvaS and mvaE/phaA. For the Mevalonate lower pathway, the genetic material transformed into the modified non-naturally occurring bacterium comprises the genes ERG12, ERG8, ERG19, IDI1, mvaK1, MvaK2 and mvaD.

| Gene name | Gene function |
|---|---|
| gap | Glyceraldehyde-3-phosphate dehydrogenase, type I |
| pdc | Thiamine pyrophosphate protein TPP binding domain-containing protein |
| tuf | Elongation factor Tu |
| eno | Phosphopyruvate hydratase |
| eda | 2-dehyd ro-3-deoxyph osphogluconate aldolase/4-hydroxy-2-oxoglutarate aldolase |
| zwf | Glucose-6-phosphate1-dehydrogenase |
| frk | ROK family protein |
| | Hypothetical protein |
| gfo | Oxidoreductase domain-containing protein |
| gloA3 | Glyoxalase/bleomycin resistance protein/dioxygenase |
| rpsG | 30S ribosomal protein S7 |
| | Elongation factor G |
| adhB | Iron-containing alcohol dehydrogenase |
| ilvC | Ketol-acid reductoisomerase |
| atpD | FOF1 ATP synthase subunit beta |
| | Histone family protein DNA-binding protein |
| glnA | Glutamine synthetase, type I |
| | Antibiotic biosynthesis monooxygenase |
| clcD1 | Carboxymethylenebutenolidase |
| glmS | Glucosamine--fructose-6-phosphate aminotransferase |
| | Hypothetical protein |
| | Toxic anion resistance family protein |
| | S1/P1 nuclease |
| | Nucleotidyl transferase |
| | Hypothetical protein |
| | 7-cyano-7-deazaguanine reductase |
| prmA | Ribosomal L11 methyltransferase |
| recJ | Single-stranded-DNA-specific exonuclease RecJ |
| gidB | Methyltransferase GidB |
| | UvrD/REP helicase |
| | Peptidase M28 |
| | NAD-dependent epimerase/dehydratase |
| dprA | DNA protecting protein DprA |
| | DEAD/DEAH box helicase domain-containing protein |
| | Uracil-DNA glycosylase superfamily protein |
| cysD | Sulfate adenylyltransferase subunit 2 |
| xseA | Exodeoxyribonuclease VII large subunit |
| Table 1: List of endogenous wild-type *Zymomonas mobilis* promoters and the genes they regulate. | |

### IV. Olivetolic Acid Synthesis

For the production of cannabinoids in a non-naturally occurring modified *Zymomonas mobilis,* the biosynthesis of olivetolic acid is crucial as this is an intermediate product for cannabigerolic acid (CBGA) biosynthesis. In the non-naturally occurring modified bacterium, the synthesis of the intermediate product (precursor), olivetolic acid, takes place via a synthetic route from the accumulated metabolic intermediate acetyl-CoA. As a result of the triple knockout (TKO) described under section I., the enzymatic conversion of pyruvate to lactate and acetaldehyde by the enzyme pyruvate decarboxylase does not occur, contrary to the wild-type. As a consequence, pyruvate is accumulated. Therefore, it is subsequently provided both as substrate for the induced geranyl pyrophosphate (GPP) synthesis and for the enzymatic conversion by the pyruvate dehydrogenase complex. With the latter, a conversion from pyruvate to acetyl-CoA takes place. The genes coding for the enzymes acetyl-CoA acetyltransferase, FadB hydroxyacyl-CoA dehydrogenase, FadB enoyl-CoA hydratase and EgTer enoyl-CoA reductase are introduced using genetic engineering, thereby an enzymatic conversion of acetyl-CoA to the intermediate product (precursor) hexanoyl-CoA takes place. In addition, the gene coding for the enzyme acetyl-CoA carboxylase is introduced using genetic engineering, resulting in an enzymatic conversion to malonyl-CoA. Finally, the genes coding for the enzymes olivetolic acid synthase and olivetolic acid cyclase are introduced using genetic engineering. Consequently, an enzymatic conversion of hexanoyl-CoA and malonyl-CoA to olivetolic acid takes place via four conversion steps. Three of these conversion are catalyzed by the enzyme olivetolic acid synthase, wherein malonyl-CoA serves as co-substrate. For the condensation reaction between Malonyl-Coa and Hexanoyl-Coa the Olivetol synthase is introduced. The cyclisation reaction to form olivetolic acid is accomplished by the olivetolic acid cyclase (Taura et al. 2009 and Gagne et al. 2012). The stable formation for olivetolic acid must be ensured by the constant flux of precursors for hexanoyl-Coa and malonyl-Coa. The constant flux of hexanoly-Coa can be achieved by the introduction of genes from the beta-oxidation of fatty acids. The reversal of these reactions leads to the formation of hexanoyl-Coa.

Additionally, competing fermentative product pathways as the production of lactate succinate acetate and ethanol, or thioesterase pathway as the production of fatty acids, that derive from the beta oxidation pathway can be silenced; for example, by using gene deletion. This ensures the intermediate product flux towards hexanoyl-Coa and malonyl-Coa.

Enhanced levels of malonyl-Coa can be achieved via the introduction of additional acetyl-Coa-carboxylase enzymes or by increasing the expression of endogenous *Zymomonas mobilis* acety-Coa-carboxylase via strong promoters. This ensures an enhanced level of malonyl-Coa for the condensation reaction with hexanoyl-Coa. The genetic material transformed into de non-naturally occurring bacterium comprises the following genes: Acetyl-CoA carboxylase carboxyl transferase subunit alpha (Gene ID: 33073499 (EC-Nr: 6.4.1.2)), Acetyl-CoA carboxylase carboxyl transferase subunit beta (Gene ID: 33072760 (EC-Nr: 6.4.1.2)), beta-ketothiolase BktB / Acetyl-CoA acetyl transferase (Gene ID: 34309143 (EC-Nr: 2.3.1.9)), FadB 3-hydroxyacyl-CoA dehydrogenase (Gene ID: 948336 (EC-Nr: 5.1.2.3)), FadB Enoyl-CoA hydratase / multifunctional fatty acid oxidation complex subunit alpha (Gene ID: 4714532 (EC-Nr: 4.2.1.17)), Trans-2-enoyl-CoA reductase (Gene ID: AY741582.1 (EC-Nr: 1.3.1.44)), Olivetol synthase (Gene ID: AB164375.1 (EC-Nr: 2.3.1.206)), Olivetolic acid (Protein ID: AFN42527.1 (EC-Nr: 4.4.1.26)), Acetyl-coenzyme A carboxylase carboxyl transferase subunit alpha (AccA) (Gene ID: 944895), Acetyl-coenzyme A carboxylase carboxyl transferase subunit beta (AccB) (Gene ID: 947758), Biotin carboxylase (AccC) (Gene ID: 947761) and/or Acetyl-coenzyme A carboxylase carboxyl transferase subunit beta (AccD) (Gene ID: 946796)

### V. Cannabigerolic Acid (CBGA) Synthesis

In the non-naturally occurring bacterium, the synthesis of cannabigerolic acid (CBGA) takes place from the intermediate products (precursors) geranyl pyrophosphate and olivetolic acid. Cannabigerolic acid is a key intermediate (precursor) in the synthesis of cannabinoids. For this purpose, the gene coding for the enzyme geranyl-pyrophosphate-olivetolic acid geranyltransferase from the bacterium *Streptomyces sp.* was introduced into the non-naturally occurring bacterium using genetic engineering. Due to the original cannabigerolic acid synthase (CBGAS) from *Cannabis sativa* is not expressed functionally in heterologous organisms, a soluble aromatic prenyltransferse NphB from *Streptomyces sp.* is used (Prenyltransferase Protein ID: AWW43729.1 (EC-Nr: 2.5.1.102)). Besides expressing sufficient amounts of catalytically slow enzymes of secondary metabolism, the application of integral membrane proteins (like CBGAS) in a heterologous biosynthetic pathway is challenging due to problems of correct protein folding and incorrect organelle localization accompanied with additional compartmentation issues. To circumvent these unpredictable barriers, we alternatively select the soluble aromatic prenyltransferase NphB from *Streptomyces sp.* strain CL190 (Bonitz et al., 2011; Kuzuyama et al., 2005) to replace CBGAS (Zirepel 2017).

Previous studies indicated that NphB is able to prenylate olivetolic acid (Kuzuyama et al., 2005). High performance liquid chromatography (HPLC) analysis of the activity assay with GPP and olivetolic acid revealed the formation of two products with m/zof 361.23. CBGA and 2-O-GOA (Zirpel 2017).

### VI. Selective Cannabinoid Synthesis

The modified non-naturally occurring bacterium can be used for the synthesis of tetrahydrocannabinolic acid or cannabidiolic acid. The synthesis of tetrahydrocannabinolic acid and cannabidiolic acid each takes place from the accumulated cannabigerolic acid. Either a gene coding for the enzyme tetrahydrocannabinolic acid synthase for the enzymatic conversion of cannabigerolic acid (CBDA) to tetrahydrocannabinolic acid (THCA) or a gene coding for the enzyme cannabidiolic acid synthase for the conversion of cannabigerolic acid to cannabidiolic acid is introduced using genetic engineering. After disruption of the bacterium, the obtained cannabinoid acids can be purified and (to obtain the respective cannabinoid) decarboxylated. Since CBGA is the direct precursor of several cannabinoids, in the next step the targeted biosynthesis of individual cannabinoids can be achieved. Starting from the non-naturally occurring *Zymomonas mobilis* capable of producing CBGA, specific mutants are generated for the production of CBDA and THCA. For this reason, the enzymes from *Cannabis sativa* (Cannabidiolic acid synthase (Gene ID: AB292682.1 (EC-Nr: 1.21.3.8)) and Tetrahydrocannabinolic acid synthase (Gene ID: AB057805.1 (EC-Nr: 1.21.3.7)) are expressed in the non-naturally occurring *Zymomonas mobilis* strain.

### VII. Transformation of Zymomonas mobilis

### Zymomonas mobilis strains and growth conditions

Zymomonas mobilis strains ZM1, ZM4 and ZM6 were acquired from ATCC (American Type Culture Collection):

| **Strain** | **ATCC Number** |
|---|---|
| ZM4 | ATCC 31821 |
| ZM6 | ATCC 29191 |
| ZM1 | ATCC 10988 |
| Table 2: Zymomonas mobilis strains | |

| | | | |
|---|---|---|---|
| **Z. mobilis** ZM1 ZM4 | RM Medium | 20 g/l Glucose | pH 6 |
| | | 10 g/l Yeast extract | |
| | | 2 g/l KH₂PO₄ | |
| | | **Agar plates:** | |
| | | **15 g/l** Agar-Agar | |
| | alternative RM Medium (ZM6) | 20 g/l Glucose | pH 6 |
| | | 5 g/l Yeast extract | |
| | | **Agar plates:** | |
| | | 15 g/l Agar-Agar | |
| | Minimal Medium A | 20 g/l Glucose | pH 5,5 |
| | | 3,5 g/l KH₂PO₄ | |
| | | 2 g/l (NH₄)₂SO₄ | |
| | | 1g/l MgSO₄^{∗}7H₂O | |
| | | 0,01 g/l FeSO₄^{∗}7H₂O | |
| | | 19,52 g/l MES | |
| | | **after autoclaving:** | |
| | | 10 ml/l sterile vitamin mix: **0,1 mg/ml** (10 mg/100 ml biotin, 10 mg/100 ml Ca-Panthothenate) | |
| | | **Agar plates:** | |
| | | 15 g/l Agar-Agar | |
| | Minimal Medium B | 20 g/l Glucose | pH 5,7 |
| | | 1 g/l KH₂PO₄ | |
| | | 1 g/l K₂HPO₄ | |
| | | 0,5 g/l NaCl | |
| | | 1 g/l (NH₄)₂SO₄ | |
| | | 0,2 g/l MgSO₄^{∗}7H₂O, | |
| | | 0,025 g/l Na₂MoO₄.2 H₂O | |
| | | 0,025 g/l FeSO₄^{∗}7 H₂O | |
| | | 0,01 g/l CaCl₂ ^{∗}2 H₂O | |
| | | **after autoclaving:** | |
| | | 10 ml/l sterile vitamin mix: **0,1 mg/ml** (10 mg/100 ml biotin, 10 mg/100 ml Ca-Panthothenate) | |
| | | **Agar plates:** | |
| | | 15 g/l Agar-Agar | |
| | Minimal Medium C | 0,5 g/l KH₂PO₄ | pH 5,8 |
| | | 1,6 g/l NH₄Cl | |
| | | 1 g/l MgSO₄^{∗}7H2O | |
| | | 0,21 g/l Citronensäure-Hydrat | |
| | | 0,01 g/l feSO₄^{∗}7H₂O | |
| | | **after autoclaving:** | |
| | | 10 ml/l sterile vitamin mix: **0,1 mg/ml** (10 mg/100 ml biotin, 10 mg/100 ml Ca-Panthothenate) 100 g/l fructose (in H₂O, sterile filtered) | |
| | | **Agar plates:** | |
| | | 15 g/l Agar-Agar | |

| | **RM** | | **MMa** | | **MMb** | | **MMc** | |
|---|---|---|---|---|---|---|---|---|
| | **30 °C** | **37 °C** | **30 °C** | **37 °C** | **30 °C** | **37 °C** | **30 °C** | **37 °C** |
| **ZM1 Duplication time [h]** | 5,2 | 2,0 | 3,1 | 2,8 | 3,6 | 6,0 | 2,5 | 2,2 |
| **ZM4 Duplication time [h]** | 1,5 | 1,5 | 2,3 | 3,3 | 2,1 | 2,8 | 3 | 2,7 |
| Table 4: Duplication times of Z. mobilis ZM4, ZM1 and ATCC 31821 incubated at 30 °C and 37 ° C in different media. Calculation was done by nonlinear fit (model: Y = Y0 ^{*} exp (k ^{*} X)) of the growth curve in the exponential phase (ZM1: t5-t16 (RM), t4-t18 (MMa), t1-t10 (MMb), t4-t14 (MMc), ZM4: t4-t10 (RM), t4-t13 (MMa), t4-t11 (MMb), t5-t13 (MMc) in GraphPad Prism. | | | | | | | | |
| The Zymomonas mobilis growth curves for the different medias are described in Figures 11a, 11b, 11c and 11d respectively. | | | | | | | | |

Competent cells are produced as described in Yang et al. (2016, doi: 10.1111/1751-7915.12408) and transformation of plasmids is accomplished via electroporation or chemical transformation of the cells.

### Plasmid vector design

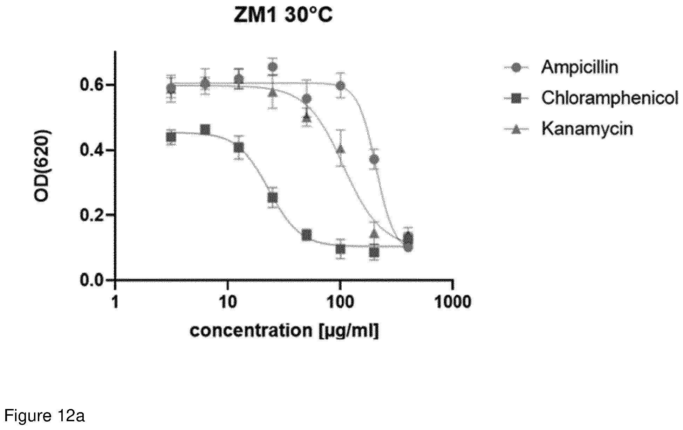

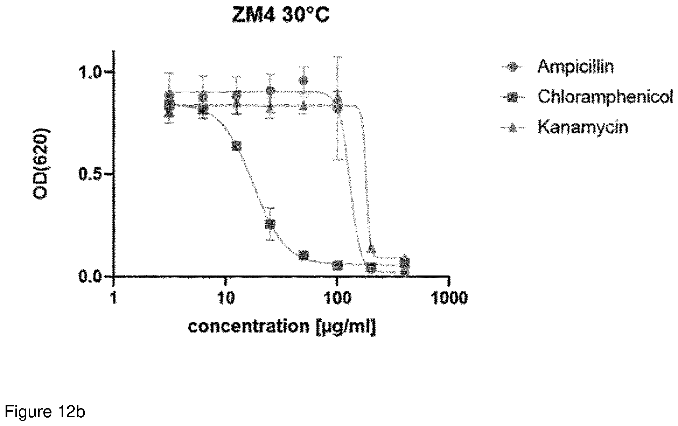

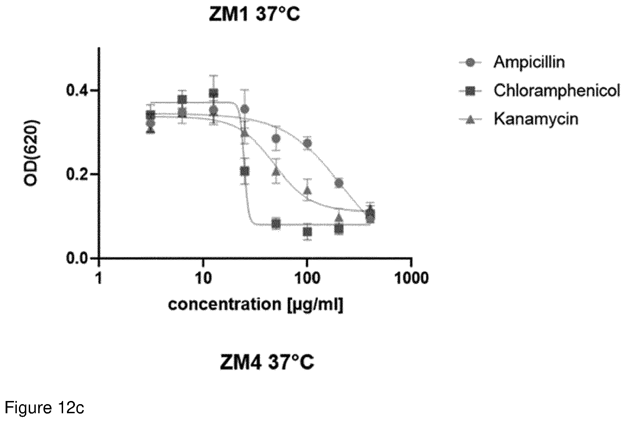

In an embodiment, the genetic insertion for cannabinoid production is accomplished via plasmid transformation. For that purpose, shuttle-vectors are designed, which can be used for the incorporation of genetic material in *Zymomonas mobilis* and *Escherichia coli* cells. Each plasmid contain an *E.coli* origin of replication identified by the sequences SEQ ID_NO 12 and SEQ ID_NO 13, a *Zymomonas mobilis* origin of replication identified by the sequences SEQ ID_NO 9, SEQ ID_NO 10 and SEQ ID_NO 11 and antibiotic resistance cassette selected from any of the sequences identified by SEQ ID_NO 14, SEQ ID_NO 15, SEQ ID_NO 16, SEQ ID_NO 17 and SEQ ID_NO 18. The Growth curves of *Zymomonas mobilis* (ZM1 and ZM4) are incubated at different ampicillin, chloramphenicol and kanamycin concentrations at an incubation temperature of 30 ° C (and 37°C) for 24 hours. The curves correspond to non-linear fit (Model: Y = Bottom + (Top-Bottom) / (1 + ((X ^ HillSlope) / (IC50 ^ HillSlope))) in GraphPad Prism. The error bar: standard deviation of the 3 technical replicates (See Figures 12a, 12b, 12c and 12d).

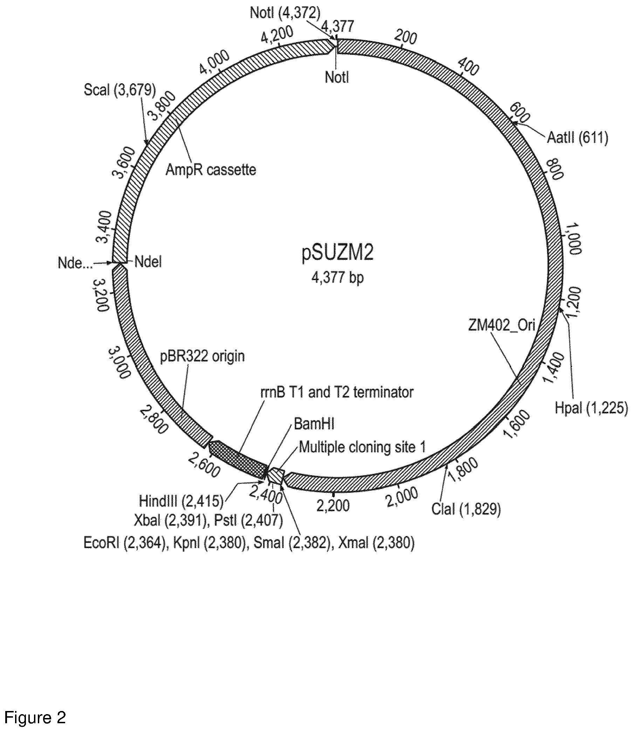

In an embodiment, the shuttle vector pSUZM2 (P1) (identified by SEQ ID_NO 1) comprises the origin of replication of the native *Zymomnas mobilis* (ZM4) Plasmid pZZM402 (identified by SEQ ID_NO 9) and it is designed analogously to the shuttle-vector described in Cao et al. (2016, doi: 10.1016/j.ejbt.2015.11.004) . P1 comprises a multiple cloning site for restriction-based insertion of DNA fragments, a rrnB T1 (identified by SEQ ID_NO 20) terminator, the E. *coli* origin of replication pBR322 (identified by SEQ ID_NO 12) and an ampicillin-resistance-cassette (AmpR) (identified by SEQ ID_NO 15). Figure 2 displays the plasmid map, the plasmid sequence containing 4377 bp (SEQ ID_NO 1) as shown below and deposited in the appendix.

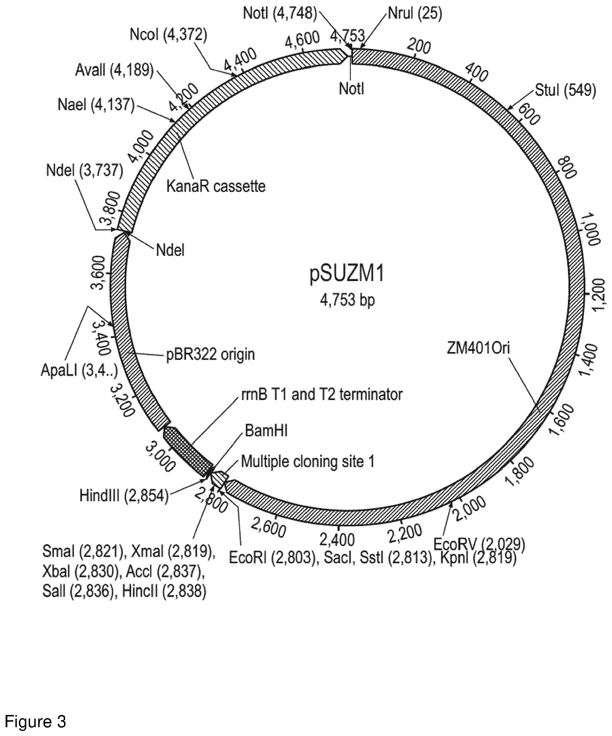

In an embodiment, the shuttle vector pSUZM1 (P2) (identified as SEQ ID_NO 2) comprises the origin of replication of the native Z. *mobilis* (ZM4) Plasmid pZZM401 (identified as SEQ ID_NO 10) and it is designed analogously to the shuttle-vector described in Cao et al referenced above. P1 comprises a multiple cloning site for restriction-based insertion of DNA fragments, a rrnB T1 terminator (identified as SEQ ID_NO 20), the E. *coli* origin of replication pBR322 (identified as SEQ ID_NO 12) and a kanamycin-resistance-cassette (KanaR) (identified as SEQ ID_NO 16). Figure 3 displays the plasmid map, the plasmid sequence containing 4753 bp (identified as SEQ ID_NO 2) as shown below and deposited in the appendix.

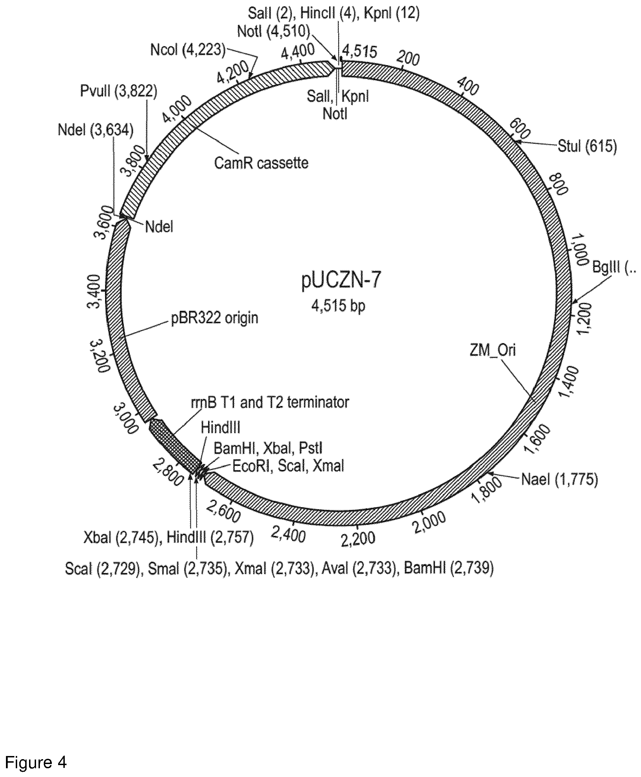

In an embodiment, the shuttle vector pUCZN-7 (P3) (identified as SEQ ID_NO 3) comprises the origin of replication of the native Z. *mobilis* (ZM4) Plasmid pZMO7 and it was designed analogously to the shuttle-vector described in So et al. (2014, doi: 10.1186/1471-2180-14-68). P1 contains the Z. *mobilis* origin of replication from pZMO7 (ZM_Ori) (SEQ ID_NO 11), a multiple cloning site for restriction-based insertion of DNA fragments, a rrnB T1 terminator (SEQ ID_NO 20), the E. coli origin of replication pBR322 (SEQ ID_NO 12) and a chloramphenicol-resistance-cassette (CamR) (SEQ ID_NO 14) . Figure 4 displays the plasmid map, the plasmid sequence containing 4515 bp (SEQ ID_NO 3) as shown below and deposited in the appendix.

### Gene cassette design

One cassette comprises two or more genes separated by a ribosome binding site and are under control of one endogenous promoter (see Table 1) and one plasmid comprises one or more cassettes each regulated by a promoter (see Table 6).

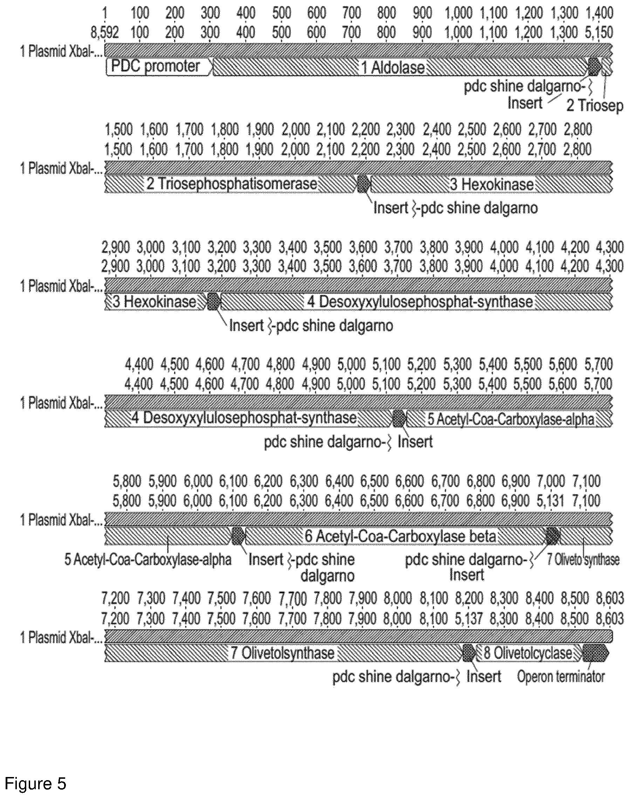

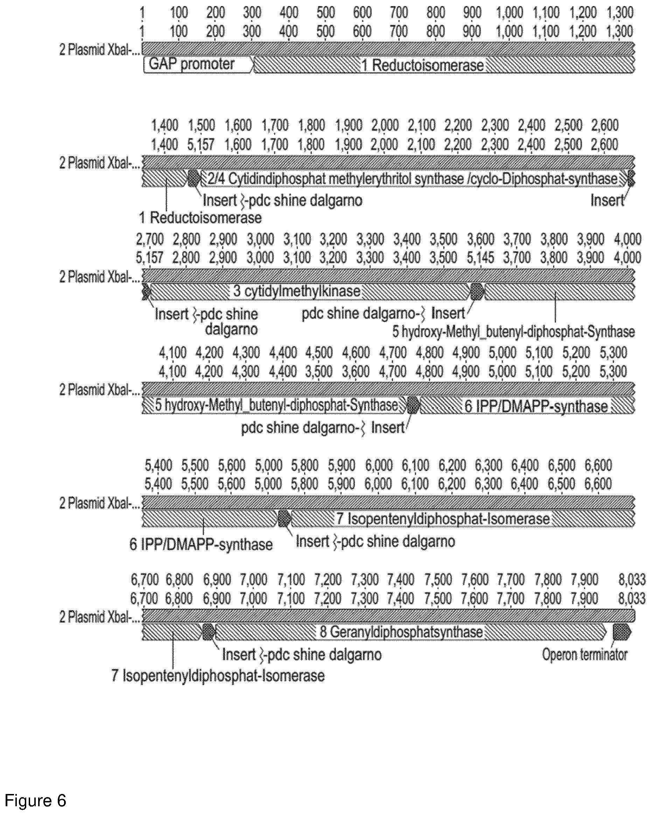

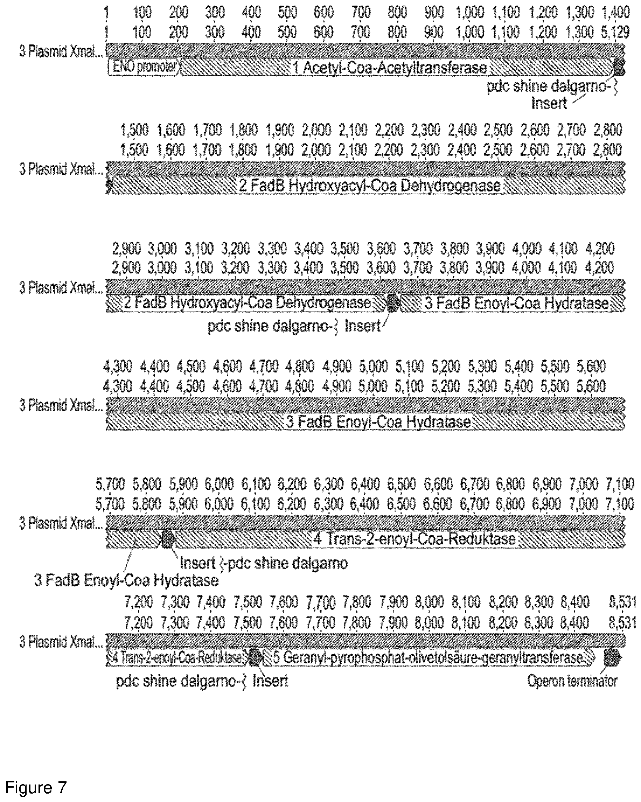

In an embodiment, three gene cassettes G1, G2 and G3 (Figure 5 - 7, respectively) (identified as SEQ ID_NO 4, SEQ ID_NO 5 and SEQ ID_NO 6, respectively) are designed. The cassettes were cloned into the P1, P2 or P3 plasmids via restriction digestion and ligation.

| | **P1** | **P2** | **P3** |
|---|---|---|---|
| **G1** | G1 is cloned into P1 | G1 is cloned into P2 | G1 is cloned into P3 |
| **G2** | G2 is cloned into P1 | G2 is cloned into P2 | G2 is cloned into P3 |
| **G3** | G3 is cloned into P1 | G3 is cloned into P2 | G3 is cloned into P3 |
| Table 5: Different combinations of cassettes and plasmids resulting in a total of 9 possible constructs. | | | |

The gene cassettes (G1-G3) are listed in Table 6 with their respective genes, Gene-ID numbers, EC (enzyme commission)-numbers and promoters.

| | | **Genes** | **Gene-ID Nr.** | **EC-Nr.** | **Promoter** |
|---|---|---|---|---|---|
| **Gene 1** | 1 | Aldolase | 33950769 | EC-Nr. 4.1.2.13 | PDC |
| **G1** | 2 | Triose-phosphate isomerase | 29953556 | EC-Nr. 5.3.1.1. | |
| | 3 | Hexokinase (Glucokinase) | 33073808 | EC-Nr. 2.7.1.1 | |
| | 4 | Desoxyxylulosephosphate (DOXP)-Synthase | 29954322 | EC-Nr.: 2.2.1.7 | |
| | 5 | Acetyl-CoA carboxylase alpha | 33073499 | EC-Nr. 6.4.1.2 | |
| | 6 | Acetyl-CoA carboxylase β | 33072760 | EC-Nr. 6.4.1.2 | |
| | 7 | Olivetol synthase | AB164375.1 | EC-Nr. 2.3.1.206 | |
| | 8 | Olivetolic acid cyclase | AFN42527.1 | EC-Nr. 4.4.1.26. | |
| | | | | | |
| **Gene 2** | 1 | Deoxy-xylulose-phosphate (DOXP)-reductoisomerase | 29954627 | EC-Nr.: 1.1.1.267 | GAP |
| **G2** | 2 | Methylerythritol-phosphate Cytidylyl-transferase (Methylerythritol-cyclodiphosphate | 29952840 | EC-Nr. 2.7.7.60 (EC-Nr. 4.6.1.12) | |
| | | synthase) | | | |
| | 3 | Diphosphocytidyl-Methyl-erythritol kinase | 29954344 | EC-Nr. 2.7.1.148 | |
| | 4 | Hydroxy-Methyl-Butenyl-Diphosphate synthase | 29952365 | EC-Nr. 1.17.7.1 | |
| | 5 | IPP/DMAPP synthase | 17279806 | EC-Nr. 1.17.1.2 | |
| | 6 | Isopentenyl-diphosphate-isomerase | 8154321 | EC-Nr. 5.3.3.2 | |
| | 7 | Geranyl-pyrophosphate synthase | 888334 | EC-Nr. 2.5.1.1. | |
| | | | | | |
| **Gene 3** | 1 | Acetyl-CoA acetyl transferase | 34309143 | EC-Nr. 2.3.1.9 | ENO |
| **G3** | 2 | FadB Hydroxyacyl-CoA dehydrogenase | 948336 | EC-Nr. 5.1.2.3 | |
| | 3 | FadB Enoyl-CoA hydratase | 4714532 | EC-Nr. 4.2.1.17 | |
| | 4 | Trans-2-enoyl-CoA reductase (EgTer enoyl-CoA reductase) | AY741582.1 | EC-Nr. 1.3.1.44 | |
| | 5 | Geranyl transferase | AWW43729.1 | EC-Nr. 2.5.1.102. | |
| Table 6: Gene cassettes G1, G2 and G3. Acetyl-CoA carboxylase (gene 5 and 6) in the gene cassette G1 consists of two subunits alpha and beta, which share the same EC-Number. Methylerythritol-phosphate cytidylyl-transferase with the EC-Nr. 2.7.7.60 and Methylerythritol-cyclodiphosphate synthase with the EC-Nr. 4.6.1.12 (gene 2) in gene cassette G2 represent two different catalytic steps that are performed by the same enzyme (Gene-ID: 29952840). Trans-2-enoyl-CoA reductase (gene 4) in gene cassette G3 is alternatively named EgTER enoyl-CoA reductase (TER gene from the organism Euglena gracilis). | | | | | |

In an embodiment, the genes in the gene cassettes G1-G3 (identified as SEQ ID_NO 4, SEQ ID_NO 5 and SEQ ID_NO 6) are transformed into *Zymomonas mobilis* to produce CBGA (cannabigerolic acid), a metabolic precursor cannabinoid of CBDA (cannabidiolic acid) and THCA (tetrahydrocannabinolic acid). To produce CBDA or THCA, the gene cannabigerolic acid synthase (EC-Nr. 1.21.3.8; Gene-ID: AB292682.1.) or tetrahydrocannabinolic acid synthase (EC-Nr. 1.21.3.7; Gene-ID: AB057805.1) are cloned into either P1, P2 or P3 vector and additionally transformed.

### Transformation and Screening

In an embodiment, the shuttle vectors with the incorporated gene cassettes are transformed into *Zymomonas mobilis* via electroporation and subsequently plated on agar plates containing one or more of the following antibiotics ampicillin, kanamycin, chloramphenicol, tetracycline and spectinomycin. Single colonies are cultivated in RMG2-medium (20 g/L glucose, 10 g/L yeast extract und 2 g/L KH2PO4) at 30°C. Detection of the successful transformation of gene cassettes is accomplished via PCR and sequencing. The gene transcription and enzyme translation and activity are determined by transcriptome, proteome and metabolome analysis.

### Genome engineering using the endogenous CRISPR-Cas system

The *Zymomonas mobilis* strains can genetically modified via the CRISPR Cas system. This technique enables deletions, insertions or point mutations in the genomic DNA of *Zymomonas mobilis.*

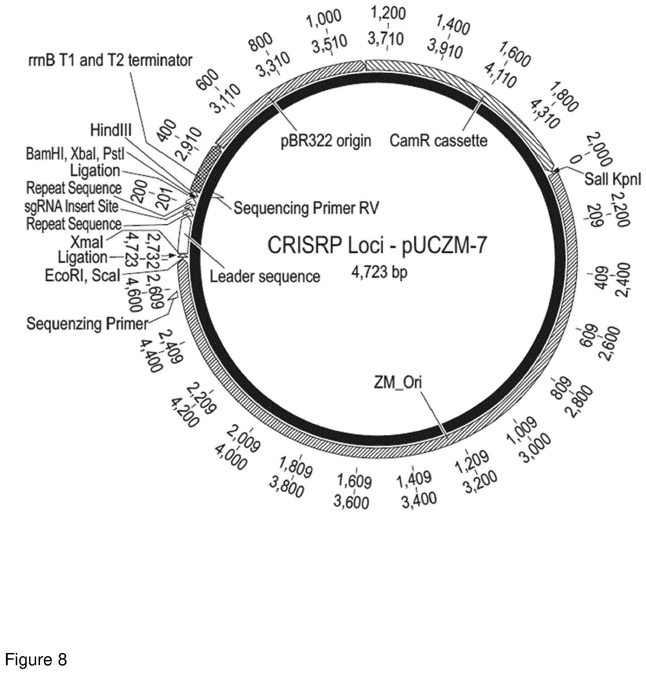

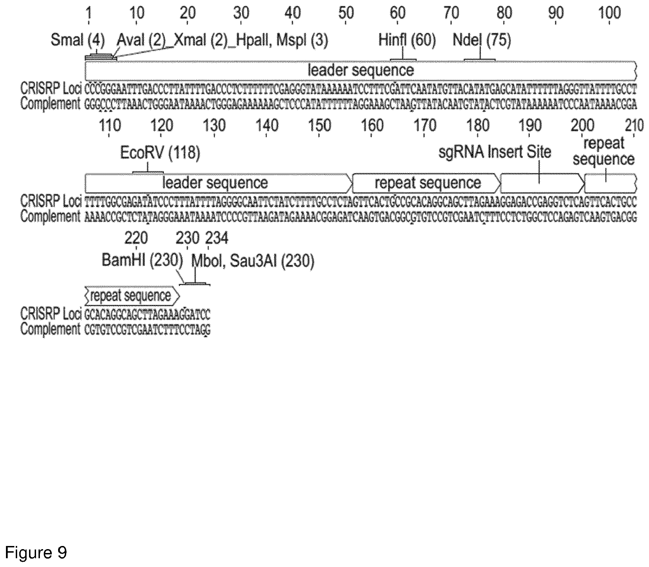

In an embodiment, the CRISPR Plasmid (CRISRP Loci - pUCZM-7) (P4) (identified as SEQ ID_NO 7) is designed according to Zheng et al. (2019, doi: 10.1101/576355) (see Figure 8). It comprises the endogenous CRISPR array (SEQ ID_NO 19), an *E. coli* (identified as SEQ ID_NO 12 and 13) and Z. *mobilis* Origin of Replication (Ori1, Ori2 and Ori3) (identified as SEQ ID_NO 9, SEQ ID_NO 10 and SEQ ID_NO 11) and a chloramphenicol resistance cassette (identified as SEQ ID_NO 14). The CRISPR array contains the endogenous leader sequence (150bp) followed by two or more repeat sequences (GTTCACTGCCGCACAGGCAGCTTAGAAA) (identified as SEQ ID_NO 8). In-between the repeat sequences, two *Bsal* restriction sites are incorporated to allow the insertion of different spacer regions (see Figure 9). The transcription product consists of a repeat region and a spacer region called guide RNA (gRNA). The repeat region connects the gRNA to the endogenous Cas protein, whereas the spacer region guides it to the complementary part of the genomic DNA followed by a subsequent double stranded break initiated by the Cas protein. By the individual construction of the spacer region, the genomic DNA can be cut at any target location under the following conditions:
1. The genomic target location must be adjacent to a PAM site (5'-CCC-3')
2. No off-target sites in the genomic DNA

The spacer sequence consists of 32 nucleotides which are synthesized as forward and reverse oligos. The oligos are constructed so that after annealing the overhangs correspond to the *Bsal* restriction sites. Thereby, the spacer region can be ligated into the CRISPR plasmid between the two repeat regions after its digestion with *Bsal.*

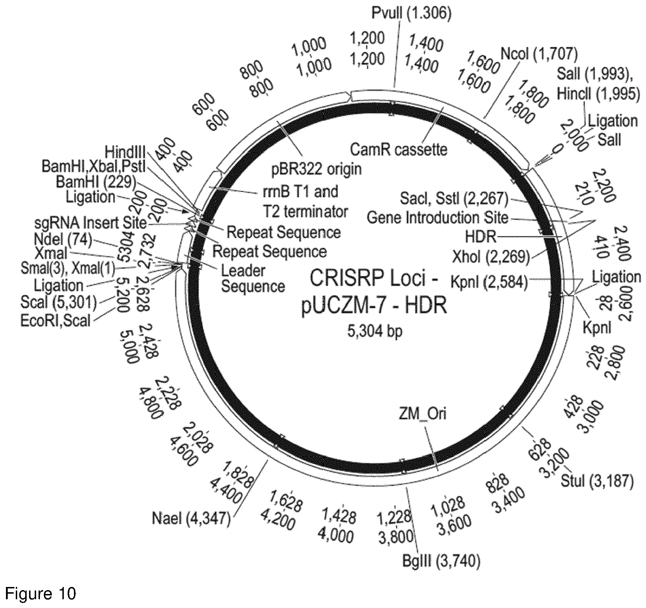

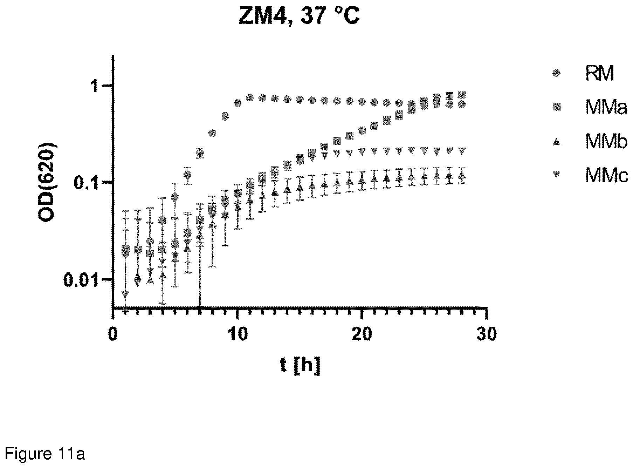

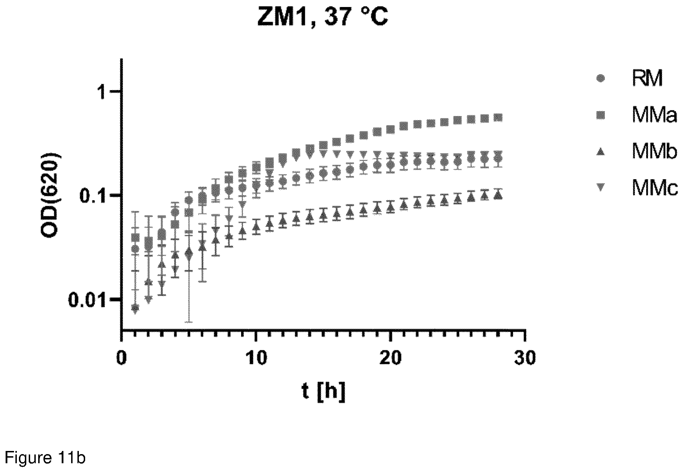

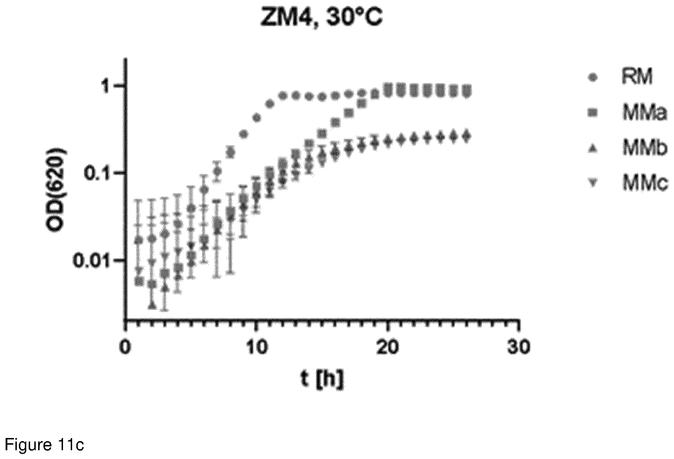

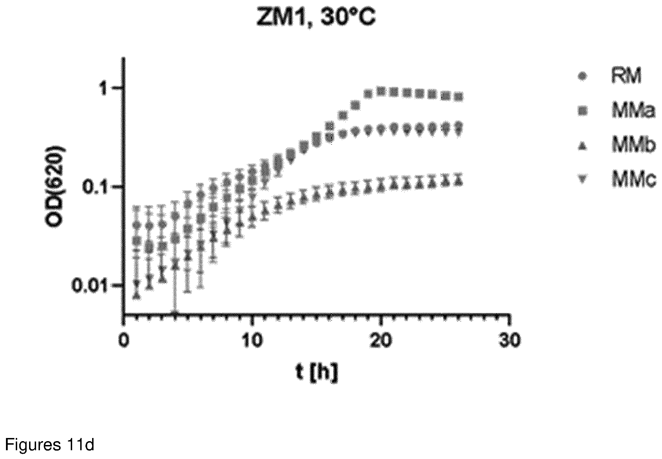

In addition, the CRISPR plasmid (P4) has a homologous directed repair (HDR) insert site with a *Sall* and a *KpNI* restriction site (see Figure 10). The HDR template is essential for DNA repair and it is composed of the desired mutation and two arms that are homologous to the genomic DNA.

### Genome Modification via Lamba Red

The *Zymomonas mobilis* strains are genetically modified via the phage Lambda-derived Red recombination system. This technique enables deletions, insertions or point mutations in the genomic DNA of *Zymomonas mobilis.* In an embodiment, a plasmid is used comprising the bacteriophage lambda red components: Gam (identified as SEQ ID 21), Beta (identified as SEQ ID 22) and Exo (identified as SEQ ID 23) as well as an antibiotic resistance cassette (identified as SEQ ID_NO 14, SEQ ID_NO 15, SEQ ID_NO 16, SEQ ID_NO 17 or SEQ ID_NO 18) and an E.coli origin of replication (identified as SEQ ID_NO 12 or SEQ ID_NO 13) and/or an Zymomonas mobilis origin of replication (identified as SEQ ID_NO 9, SEQ ID_NO 10 or SEQ ID_NO 11). The repair template essential for the homologous recombination is designed to have homologous arms to the genomic DNA of *Zymomonas mobilis* where the mutation is incorporated as well as containing the desired mutation. Both the plasmid and the repair template are transformed into *Zymomonas mobilis*, either together or after one another.

### Lambda Red Plasmid Transformation and Screening

In an embodiment, the Lambda-derived Red recombination plasmids are transformed by electroporation into the competent *Zymomonas mobilis* strain and subsequently spread on agar plates, supplemented with antibiotics. By colony PCR, the target sequence is amplified with the help of the corresponding primers and individual positive colonies are then identified with Agarose Gel electrophoresis and sequencing.

### Genome Modification via Homologous Directed Repair (HDR).

Homologous directed repair is used as a method for the introduction of genetic material in the non-natural occurring bacterial. The genetic material for the homologous directed repair contains a high degree of homology to the genetic sequence of *Zymomonas mobilis.* Through an endogenous in cell mechanism the region with a high degree of homology gets build into the genome creating a non-naturally occurring bacteria. The homologous regions are design in a way that they flank a specific sequence region. This sequence region gets incorporated into the genome of the bacteria through homologous recombination. In an embodiment, the incorporated region contains FRT-sequences from Saccharomyces cerevisiae. The FRT sequence is used as a FLP-FRT site-specific recombination system. Gen fragments flanking two FRT sequences are incorporated by the Saccharomyces cerevisiae FLT gen, coding for the FLP recombinase enzyme (Zou et al 2012)

### Gen-Knockouts

To generate gene knockout strains, the HDR arms are complementary to the DNA regions in front and behind the gene. Thereby, after successful recombination, the whole gene is cut out of the genomic DNA. In an embodiment, the incorporation of additional genes into the genomic DNA, a *Sacl* and *Xhol* restriction site are introduced between the homologous arms. The genomic DNA of *Zymomnas mobilis* is isolated and the regions before and behind the target gene are amplified by PCR. The primers are designed to match the restriction sites to those in the CRISPR Plasmid (*Sall*/*KpNI*) and the gene knockins (*Sacl*/*Xhol*) (see Table 2). Both PCR products are combined with SOE-PCR and then ligated after the digestion with *Sall* and *KpNI* into the CRISPR Plasmid.

| **Primer Name** | **Sequence (5'-3')** | **Restriction Enzymes** | **Sequence ID NO** |
|---|---|---|---|
| Pyruvate Decarboxylase_sgRNA_FW | GAAATCAATGCAGGTG AACTGAAGAAAGCCGC TCCG | *Bsal* | SEQ ID_NO 25 |
| Pyruvate Decarboxylase_sgRNA_RV | GAACCGGAGCGGCTT TCTTCAGTTCACCTGC ATTGA | *Bsal* | SEQ ID_NO 26 |
| Pyruvate Decarboxylase_DR_FW_1 | CGTCGACGTTGGTTAT GTCCTATAATC | *Sall* | SEQ ID_NO 27 |
| Pyruvate Decarboxylase_DR_RV_1 | CTCGAGGAGCTCTGCT TACTCCATATATTC | *Xhol*/*Sacl* | SEQ ID_NO 28 |
| Pyruvate Decarboxylase_HDR_FW_2 | GAGCTCCTCGAGTTTT TAAATAAACTTAGAG | *Sacl*/*Xhol* | SEQ ID_NO 29 |
| Pyruvate Decarboxylase_HDR_RV_2 | TGGTACCTTAAGGCAC TGACTTC | *KpNI* | SEQ ID_NO 30 |
| Lactatedehydrogenase_sgRNA_FW | GAAAGAAACAATTTTA AGTCGTGTTGAAAAAG GCGA | *Bsal* | SEQ ID_NO 31 |
| Lactatedehydrogenase_sgRNA_RV | GAACTCGCCTTTTTCA ACACGACTTAAAATTG TTTC SEQ | *Bsal* | SEQ ID_NO 32 |
| Lactatedehydrogenase_HDR_FW_1 | CGTCGACGAAAGATAG CTATTATTATC | *Sall* | SEQ ID_NO 33 |
| Lactatedehydrogenase_HDR_RV_1 | CTCGAGGAGCTCTATA TCGACCTTTTATTTTC | *Xhol*/*Sacl* | SEQ ID_NO 34 |
| Lactatedehydrogenase_HDR_FW_2 | GAGCTCCTCGAGTTAA GCTGCTATAAATA | *Sacl*/*Xhol* | SEQ ID_NO 35 |
| Lactatedehydrogenase_HDR_RV_2 | TGGTACCTCTGTCGCC | *KpNI* | SEQ ID_NO 36 |
| | AGATCGAC | | |
| Phosphoenolpyruvatecarboxylase_sgRNA_FW | GAAAATGAAATTTTTG GCCCTGAATCCATTAC GACT | *Bsal* | SEQ ID_NO 37 |
| Phosphoenolpyruvatecarboxylase_sgRNA_RV | GAACAGTCGTAATGGA TTCAGGGCCAAAAATT TCAT | *Bsal* | SEQ ID_NO 38 |
| Phosphoenolpyruvatecarboxylase_HDR_FW_1 | CGTCGACTTTATCGGG AAGCTG | *Sall* | SEQ ID_NO 39 |
| Phosphoenolpyruvatecarboxylase_HDR_RV_1 | CTCGAGGAGCTCAAAC CATCCCTTTGCC | *Xhol*/*Sacl* | SEQ ID_NO 40 |
| Phosphoenolpyruvatecarboxylase_HDR_FW_2 | GAGCTCCTCGAGTACA TCAGCCCTATTTTAAA G | *Sacl*/*Xhol* | SEQ ID_NO 41 |
| Phosphoenolpyruvatecarboxylase_HDR_RV_2 | TGGTACCCCGCCTTG GGCTGTCTG | *KpNI* | SEQ ID_NO 42 |
| KnockIN_G1_FW | GTCATGAGCTCATGGC GCTGGTTTCTATGCGT C SEQ | *Sacl* | SEQ ID_NO 43 |
| KnockIN_G1_RV | CAGTACTCGAGTTATT TACGCGGGGTGTAGT C | *Xhol* | SEQ ID_NO 44 |
| KnockIN_G2_FW | GTCATGAGCTCATGAA ATCTGTTACCATCCTG G | *Sac* | SEQ ID_NO 45 |
| KnockIN_G2_RV | CAGTACTCGAGTTACG CAGAACGGTTCGCCG | *Xhol* | SEQ ID_NO 46 |
| KnockIN_G3_FW | Count line1: G T C A T G A G C T C A T G A C = 16. Line2: C C G T G A A G T T G T T G T T =16.GTCATGAGCTCATGAC CCGTGAAGTTGTTGTT G | *Sacl* | SEQ ID_NO 47 |
| KnockIN_G3_RV | CAGTACTCGAGTTAGT CTTCCAGCGCGTCGAA CG | *Xhol* | SEQ ID_NO 48 |
| SequencingPrimer_ORF_FW | CTTCCTAAGCCTTTACCTGATTTAG | | SEQ ID_NO 49 |
| Sequencing Primer_ORF_RV | CGACTGAGCCTTTCGTTTTA | | SEQ ID_NO 50 |
| SequencingPrimer_HDR_FW | TCATGACCAAAATCCCTTAACGTG | | SEQ ID_NO 51 |
| SequencingPrimer_HDR_RV | CCATGTTTTGAGAAGCCGATAATC | | SEQ ID_NO 52 |
| Table 7: Primer list. | | | |

| **Gen-Knockouts** | **ID** |
|---|---|
| Pyruvate Decarboxylase | EC-Nr. 4.1.1.1. |
| Lactatedehydrogenase | EC-Nr. 1.1.1.28. |
| Phosphoenolpyruvatecarboxylase | EC-Nr. 4.1.1.31. |
| *Table 8 Gen Knockouts. The knockouts of pyruvate decarboxylase, lactatedeydrogenase and phosphoenolpyruvatecarboxylase and their corresponding Enzyme IDs.* | |

### Gen-Knockins

In an embodiment, the overhang Primers are designed to amplify the gene fragments (G1, G2 and G3) (identified as SEQ ID_NO 4, SEQ ID_NO 5 and SEQ ID_NO 6) from the expression plasmids (P1, P2 and P3) (identified as SEQ ID_NO 1, SEQ ID_NO 2 and SEQ ID_NO 3). A *Sacl* restriction site is cloned to the 5' end and a Xhol restriction site to the 3' end. After digestion of the amplified PCR product and the CRISPR plasmid (identified as SEQ ID_ NO 7), the gene fragments are cloned into the Gene Introduction Site, between the homologous directed repair (HDR) arms. This allows the gene knockout to occur simultaneously to the gene knockin after the transformation of the plasmid into *Zymomonas mobilis.*

### CRISPR Plasmid Transformation and Screening

In an embodiment, the CRISPR plasmid is transformed by electroporation into the competent *Zymomonas mobilis* strain and subsequently spread on agar plates, supplemented with antibiotics. By colony PCR, the target sequence is amplified with the help of the corresponding primers and individual positive colonies are then identified with Agarose Gel electrophoresis and sequencing.

### CRISPR Plasmid Curing

In an embodiment, the plasmid curing is performed as described by Zheng et al. (referenced above). A plasmid curation must take place between each CRISPR plasmid transformation. Individual colonies are suspended in 10 µl sterile ddH2O and then 1 µl of the cell suspension is spotted on agar plates with and without antibiotics. Cells from the same suspension that did not grow on the plate with antibiotic but on the one without antibiotic have lost the plasmid.

### Analytics

The analysis and the verification of the cannabinoids are performed by (High Performance Liquid Chromatography Mass Speck) HPLC/MS. Cannabinoids are measured using an internal standard, the retention time, the UV spectrum and the mass spectrum. If the measured values of the standard correspond to the produced compounds, it is proven that this is the natural substance confirm the structure.

In order to measure the cannabinoids produced by the modified non-natural occurring *Zymomonas mobilis*, there are several approaches. The wild type serves as a matrix and is compared with the respective mutants. Measurements of the culture filtrate after separation of *Zymomonas mobilis* cells and cell lysate after cell disruption are performed. The cell disruption is performed with a sonicator. During this treatment, the cells are buffered with a PBS lysis buffer.

In addition, fluid-fluid extraction of the culture filtrate using organic solvents (n-hexane, ethyl acetate, etc.) is performed. Further purification processes include a sand filter separation and/or solid phase extraction (SPE). The chromatograms are compared with literature or common knowledge and an interpretation is made.

### Quantification of CBGA

To determine the production rate of cannabinoids, the produced quantity of natural compounds was quantified by HPLC measurements. Calibration lines are prepared for each internal standard to calculate the total quantity of the measured sample. The obtained values are then extrapolated to one liter.

We further analyze different modified non-natural occurring *Zymomonas mobilis* bacteria in order to screen for the organism with the highest production rate of CBGA.

### SEQUENCE LISTING

SEQ ID_NO1 (DNA plasmid pSUZM2 (P1), 4377 bp):

cggaaaggtcatcctgaaaattctattcgtattctgatcaaccagatcgtgaatgatcgggcgatgagccggaaacttgtcccgtgatcagaaccggcg
cagcctcttccctttatctatttcaaattcaggcctgccacagcggagaatcttcgctgtggctttttttgcgtccaagaccggagcccgcacaatgaccgc
ctcttcctcttcagcgcccctctcggatgcctctgtcttttctttagagcctgtctctgcgtcttttgaacaaacgacgcccgtaaccagtccttttatcgaca
ggctaggccgtttcaagcgtggcacttatcgaaccatgttggttgaaacgggtat

gccatttgacagcgcaaagattatggcagatcaggccgcagaacaacataaaagagagcg

cacattctctaaattaatgcccttggccaatggcgctcatcccgaaatttcggcctcaaa

taccaatattgaggctacagcctctgcatcagatttcgacgaaatcgaatattttgacca

gatggtatcaaacggcgttgatcctagagatgccgcagatattgctcgcaaaacagctat

tgcgagacgtcgagaccctctcgaacgagccattcgctataaaacccgccgtcaaactcc

tgatcctgtgcatcgggattcttatgaggtgggcgaacgcgagaagtcggtctggaagccggtcaatccgcaagagattggtgcctatctggaagcggt
tgatcaatattcgatcaaaac

caagggcttaagcgataaggccgttcgtcttctaaagatgctgtttcggatggtcgactt

taaaactggccgtctggagccgaccctcgacacaatatgcgatcgtgtcggttatgcccg

tgcgacggtcgtcaaattactgcggcaattacaggatttaggcttcatccgctggatacg

ccggtcaatcaagatcaaggcggacggcaaaggcccccgccgcaagcagaccagtaacgc

ctacggcttcttgtctcccaaggcttggccagatttggccagacaggttttcgagcgtgt

gatgcgccggaggcatgcgcctattcctgacgatatcgaccacgcccaagaggcggacaa

agccgaaaccaaggctatcgttgaaaacttgccgcccgaagatttcgtcaaagcaatatc

gggcgataaagagccaacagagttaacagattcgttaatacagctcgccaaatctatcga

gcgggcggaacaacgggaacaagaagaacaggctaagaaggcagcacaggccggtcagga

agaggttaaaacatcttcccaaacacctaaggaggatacctcaaaaaagcccccaatctc

gggcaatcaacgcgagttcaattcttatactctatcctgttatacttatttatctaatag

cgaccatacaaaactaaaaaccaaagaagaagataaagactgcactgctcctgctaccgc

aggcagtgcagaccctatttatgatcgaaaacctgacagcaatcctttcctctcaactct

caaagctcgcggttatcgtcctgatcaaatcaacgcttgcctcgaaaacagagataaacg

gaaaaagaaaggatggatgcctctgtcacccgatgaaaccgcccgcctccttattcaaac

aaaacagatgtgcccataacaacgaccactatccttctcaaagcatatcgtccgttaatc
tctcggcgctgctcgcttcgctcacaggctcggcgagaaagaaatttgcctttcgggga
ctatctaattcctaacaattcgcattatcgataatgtaataaaggctaatctttaaaaa
ccaaattacagcgattacatcatttctatgaatgtgtattatacataatatgagaagtcg
tgatttgatcaaagaagttaaaaagccaatattaccagcttgacagatataaaataggc
cttggttgatttcgtgaaagaactggaagattgcacaattcttctttctggtgatatctt
tgacccgacggccctcagtcaatgagtgtgcgccgcatttctgcccattcaatcagggcc
tgcattgaagggccgagagcgtatcccataccgctgagctcgtattctacctttggcggg
acctgcggatagaccgttcggagcacgatgcgatccttttccagttgcttgagctgctgg
atcagcatcttctggttgatgccgtcgatctgcttttccagtgaggagaagcggattggg
ccttttgcggcaaacaactggcagatgataacaatcttccattttccttcgagtaccctg
agcgcttctgtcgtcgcctcaggaattcgagctcggtacccggggatcctctagagtcga
cctgcaggcatgcaagcttggatccataaaacgaaaggctcagtcgaaagactgggcctt
tcgttttatctgttgtttgtcggtgaacgctctcctgagtaggacaaatccgccgggagc
ggatttgaacgttgcgaagcaacggcccggagggtggcgggcaggacgcccgccataaac
tgccaggcatcaaattaagcagaaggccatcctgacggatggcctttgtagaaaagatca
aaggatcttcttgagatcctttttttctgcgcgtaatctgctgcttgcaaacaaaaaaac
caccgctaccagcggtggtttgtttgccggatcaagagctaccaactctttttccgaagg
taactggcttcagcagagcgcagataccaaatactgtccttctagtgtagccgtagttag
gccaccacttcaagaactctgtagcaccgcctacatacctcgctctgctaatcctgttac
cagtggctgctgccagtggcgataagtcgtgtcttaccgggttggactcaagacgatagt
taccggataaggcgcagcggtcgggctgaacggggggttcgtgcacacagcccagcttgg
agcgaacgacctacaccgaactgagatacctacagcgtgagctatgagaaagcgccacgc
ttcccgaagggagaaaggcggacaggtatccggtaagcggcagggtcggaacaggagagc
gcacgagggagcttccagggggaaacgcctggtatctttatagtcctgtcgggtttcgcc
acctctgacttgagcgtcgatttttgtgatgctcgtcaggggggcggagcctatggaaaa
acgccagcaacgcggcctttttacggttcctggccttttgctggccttttgctcacatat
gttcaaatatgtatccgctcatgagacaataaccctgataaatgcttcaataatattgaa
aaaggaagagtatgagtattcaacatttccgtgtcgcccttattcccttttttgcggcat

tttgccttcctgtttttgctcacccagaaacgctggtgaaagtaaaagatgctgaagatc

agttgggtgcacgagtgggttacatcgaactggatctcaacagcggtaagatccttgaga

gttttcgccccgaagaacgttttccaatgatgagcacttttaaagttctgctatgtggcg

cggtattatcccgtattgacgccgggcaagagcaactcggtcgccgcatacactattctc

agaatgacttggttgagtactcaccagtcacagaaaagcatcttacggatggcatgacag

taagagaattatgcagtgctgccataaccatgagtgataacactgcggccaacttacttc

tgacaacgatcggaggaccgaaggagctaaccgcttttttgcacaacatgggggatcatgtaactcgccttgatcgttgggaaccggagctgaatgaa
gccataccaaacgacgagcgtgacaccacgatgcctgtagcaatggcaacaacgttgcgcaaactattaactggcgaactac

ttactctagcttcccggcaacaattaatagactggatggaggcggataaagttgcaggac

cacttctgcgctcggcccttccggctggctggtttattgctgataaatctggagccggtg

agcgtggttctcgcggtatcattgcagcactggggccagatggtaagccctcccgtatcg

tagttatctacacgacggggagtcaggcaactatggatgaacgaaatagacagatcgctg

agataggtgcctcactgattaagcattggtaactgtcagaccaagtttactcatatatac

tttagattgatttaaaacttcatttttaatttaaaaggatctaggtgaagatcctttttg

ataatctcatgaccaaaatcccttaacgtgagttttcgttccactgagcgcggccgc

SEQ ID_NO2 (DNA plasmid pSUZM1 (P2), 4753 bp):

gcaccgcaaaggacatgctgatcgcgagatcggcgcgctactcgacactgtcataacaga

tcacattcggagatagctgatgaatactgcgcagaatcagactcttaaaagagcgtttttgtcatcagaagccgcagcagctgactcttcagctgactgc
ggcttttctatgtctgaaaa

agatatgccagattttgaagaaatgacgccagccagcccttttatcgacaggctaggccg

tttcaagcgcggcacttatcgaagcatgttgatgcagacggggctttcttatgaacgcgc

gaaaggcatggcagacaatgcggcggaagaatttaaaaaatcaaaagcaaagtcgaaatc

tttgccattggccaagagcgaaatcgctgaacttggggcttcagaagcaacgattgaagtaacagctcatcaggtaacattttctgaatcacaggcaca
accagaacctttcgatgctta

ctgctgcttgcaacagttaaaagatcagggcgtggacggtgccacagctgcgaagattgc

aaatcaggcctacaagcatcacaaagaagccgaaaattttgacgaagcggtatatttcaa

ggaattagtcgatactggcatagatgcggtgatggctgctaatatggctcgtaaaacagc

cgaagctaagcgccgcgacccgctggaaagatacggtatccgtttaaagccgcgccgcac

gcctgatcctgttcaccgtgactcttatgatgttggcgagcgcgaaaatacggtctggaa

gccggtcaatccgaatgagatcggggcttatctggaagcagtcgatcaatattcaatcaa

gaccggcaatatcagtgatagagccgtccggctgctcaagatgctgtttcggatggtgga

cttcaagacaggtcgattagagccgacgcttgatacaatctgcgaatgggtcggctatgc

acgggcaacggttgtccgcttgctaaaacagcttcaagagcaaggttttatccgctggat

acgccgttcaatcaagatcaagacggatggggctgggccccgccgcaaacagaccagtaa

cgcttacggtttcttgtcgcctaaatcttggccggagatggcaagaaaggttttcgagcg

cgtgatgcgccggaagaatgcgccggtctctgctgatcttgctcatgctcaagaagccga

caaagtcgaaaccggtgccatgattgaaagccagccgaatgacgactgggtgaaagaaat

cttcgcacagaagcgcgcgcaacatccgacgagtgacgagcagaaaaacaaaaaagaaca

ggccgatgatctggcagagactcttgccagcttcgccaaatctatcgagcgggcagaaca

acgggaacaagaagaacaggccaagaaagccatttcggccgagcaaaatgaggttaaaac

accctacaaaacactcaaggaggacacctcaaaaacgccccaaatttcggcagttgagcg

cgagttcaattttgatactctatcccgttatagttattttaataataaccactccaatca

atttaaaaaccaaaaagagattaaggactgcactgctcctgctgccgcaggcagtgcaga

cgttgttttgaaaacaaaacctcaaaccgacactttcctaaaaacccttcaaactcgcgg

ttaccgtcaagaccagaccaacgcttgcctcgaaaacagagataaacggaaaaagaatggatggatgcctctgtcagcggacgaaaccgcccgcctc
cttatacaaacaaggcagatgcg

accgtgataccgacagctatccttctcaaagcacaccgcccattaatctctcggcgctgc

tcgcttcgctcacaggctcggcgaaaaagaaatttgccttttgggggctattaacttct

aacaactcgcattatcgaaatgtctttaaataggtttattctctaaaagcgtttcgacat

ggcttttactgttatccatcatccaatcttcagtcggtcttgagcgatatccaatcaaacgaacaattctatcccagtcagcttgtagatctctttcaaatat
aggctcatgatgggcta

tccgattacgcagcatccgaatttcacccaaatctctatagagactctgtctctggattt

taatctcttgctgtggatcaagattcgggaacacgttcaacaaatagagatcccaaagcc

gtttatcatggcgctttgtgaacatagcctgccagaatacaaaattcagttctggtattacctttccggtcgtttccgccttcaaacaggctttgtttacatc
cttcgatgcgctgtatc

ctgatttgacaactggaaggcttcgtcggaaaccttctgaccacggccactgatctccat
aggtataggatagcgcttccgccacagcatttcgggtcgctatttctgttatatgaaggg
gggctagcaatgcgccggatagctgggcattccacacatataaatccagagcgacgagat
catcttttcccaatgccctgactgtgtcttcatatcttccaagtcggggagccgataagg
tctttctgatagaggtaatcttgtcttgaattgacagcatggctacccatttatatgatc
aaccacatattcgcctcgggtctttgttggcttatgcctcccctgaggacaaaagatac
aaaaaagacctcgcttacggcggggtctttttttatccattttcctgtattgtataaagt
ctttgctagcctgacggaaaacgtaatcctgcggttaatcggaattcgagctcggtaccc
ggggatcctctagagtcgacctgcaggcatgcaagcttggatccataaaacgaaaggctc
agtcgaaagactgggcctttcgttttatctgttgtttgtcggtgaacgctctcctgagta
ggacaaatccgccgggagcggatttgaacgttgcgaagcaacggcccggagggtggcggg
caggacgcccgccataaactgccaggcatcaaattaagcagaaggccatcctgacggatg
gcctttgtagaaaagatcaaaggatcttcttgagatcctttttttctgcgcgtaatctgc
tgcttgcaaacaaaaaaaccaccgctaccagcggtggtttgtttgccggatcaagagcta
ccaactctttttccgaaggtaactggcttcagcagagcgcagataccaaatactgtcctt
ctagtgtagccgtagttaggccaccacttcaagaactctgtagcaccgcctacatacctc
gctctgctaatcctgttaccagtggctgctgccagtggcgataagtcgtgtcttaccggg
ttggactcaagacgatagttaccggataaggcgcagcggtcgggctgaacggggggttcg
tgcacacagcccagcttggagcgaacgacctacaccgaactgagatacctacagcgtgag
ctatgagaaagcgccacgcttcccgaagggagaaaggcggacaggtatccggtaagcggc
agggtcggaacaggagagcgcacgagggagcttccagggggaaacgcctggtatctttat
agtcctgtcgggtttcgccacctctgacttgagcgtcgatttttgtgatgctcgtcaggg
gggcggagcctatggaaaaacgccagcaacgcggcctttttacggttcctggccttttgc
tggccttttgctcacatatgttcaaatatgtatccgctcatgagacaataaccctgataa
atgcttcaataatattgaaaaaggaagagtatgattgaacaggatggcctgcatgcgggt
agcccggcagcgtgggtggaacgtctgtttggctatgattgggcgcagcagaccattggc
tgctctgatgcggcggtgtttcgtctgagcgcgcagggtcgtccggtgctgtttgtgaaa
accgatctgagcggtgcgctgaacgagctgcaggatgaagcggcgcgtctgagctggctg
gccaccaccggtgttccgtgtgcggcggtgctggatgtggtgaccgaagcgggccgtgat

tggctgctgctgggcgaagtgccgggtcaggatctgctgtctagccatctggcgccggca
gaaaaagtgagcattatggcggatgccatgcgtcgtctgcataccctggacccggcgacc
tgtccgtttgatcatcaggcgaaacatcgtattgaacgtgcgcgtacccgtatggaagcg
ggcctggtggatcaggatgatctggatgaagaacatcagggcctggcaccggcagagctg
tttgcgcgtctgaaagcgagcatgccggatggcgaagatctggtggtgacccatggtgat
gcgtgcctgccgaacattatggtggaaaatggccgttttagcggctttattgattgcggc
cgtctgggcgtggcggatcgttatcaggatattgcgctggccacccgtgatattgcggaa
gaactgggcggcgaatgggcggatcgttttctggtgctgtatggcattgcggcaccggat
agccagcgtattgcgttttatcgtctgctggatgaatttttctaataactgtcagaccaa
gtttactcatatatactttagattgatttaaaacttcatttttaatttaaaaggatctag
gtgaagatcctttttgataatctcatgaccaaaatcccttaacgtgagttttcgttccac
tgagcgcggccgc

<220>
   <223> pUCZM-7 (P3) 
SEQ ID_NO 3 (DNA plasmid pUCZM-7 (P3), 4515 bp):

gtcgacggta ccatgattat cggcttctca aaacatggac agggcaaagc tgctccagcc

ataaaatacc tgctcgatca gcacagagag ggcagagaaa atcatattcc tgaagtggtg

agaggtaatc cagacttaac cgcatccatt atcgacagcc tgactttcaa acatcactat

actagcggag ttctttcttt cgctgccgaa gatcatgttt ctgccgagca ggaaaaagat

attatcaacg atttcgaaaa gctggctttt gctggcctgc aagcagacca gtttaatatc

ctttgggtgc gccattctca tgcgggtcat catgaacttc atttcatcac tccaaggata

gaactttcat ccaagaagtc cttcaacatc aagccccccg gtaagaatac ccaaaaggaa

tttgatgact tgcggagtgc catcaatgcc aaatatggct ttgctgaccc agaagacccg

atgagagcga gaaacgtcaa aacacctagc tatgaactaa aaatcgctag gaatgtcctt
 
agagagcaaa aagcgccctc tgaggacata agacagctta ttgatagcct tttaactgaa
agagccgtcc aaggcctgat acatgacaga aaaactcttc tcactaatgt tgaagagtta
ggcttcgaag ttactcgcgc aggcaagaat tatattaccg tcaaagaccc tgaaagctca
aaacgatttc gtctcaaagg ccaactctat gaacaagact ttacctctag ccgaacgatt
gaaacagcag cagctaaaag aacaagagat tattctcgac ctgactcaac agcagctaga
caatttgagg aacaagttaa actccacatt cgaaaacgag ctgaattcaa tagaaaacga
tatcgagcga ctgaaccgga gcttaggatg gaaattcctc ctatctcgct ggctgatacc
agtcgtgacc agcatcatga tattgtttgg cctgctcttg ggcgcagaag ggataatcaa
gctctatacc caccagatca gagctttgca ggaggaaaaa gcagagatgg aagtgaccgt
cgaacagctc aacaaagcag gtgggaagat gcaattcagc cattgcggag aaaaagaaag
gctttgcatc agagtaaaca cacgagagat cttcgggata accagatatt atctgatcgt
gaaagaacgg aaatagacaa tgacggaatt ggaacaggtt ttactgaacg ccttggaaaa
tttacagcag agattcgatc aacaacagaa aagtttatta cacgcgcaaa gcgaattacg
gcagatgttc gaaactacct caaccgagaa tacttattta cgacagcaag tagaaatctt
ggtcgagcga ttgaacagtt ttcccagatg aaaaccttaa gtcgaaagct acaaaaacgg
agaggtcgtt ctcgctctcg ataaacttga aaaggacgct aattccgaaa agttttttat
ctttctgata gtcgatttta gttggcgatt tccgataaaa aagccctgaa cttattgaac
tttcctatat ttcaataagt tcaataagtt cagggctttt ttattcaaaa aaatcaatgc
cttacgactt taaaaaccac ttttggggag tgaaaaacca cttttgggga gtgaaaaacc
acttttgggg agtgaaaaac cacttcaaaa aatatattaa gtggttaata aagttgtaat
gagaaaaaca ctacaaataa ttaaagatag agccggcaat aaagatcaaa atttacatgc
aggtgaaatt atagatggac atttttcaga ccttaaaaat ccgcctagcc tagctgcact
aaaactctat gagttgctta ttaaatacgc agcatataat atagataaaa atatatggca
taatatttct gtatcagaaa tgcaaaaaat caaaggaata aagaactata actcaacaga
gctaactcaa ttattaaaag aacttagagc tgttgttatg gaatatagag caaaagatag
aactattatt actggtcttt tagatattgc tgcggtcata tctaacccag acacaggata
tccagagata caatggaaat ttggagaaag ttttagagat ttaatagcta agtcagaata
ttgggcaatt gttgattata gaacaacgtt agagatgacg agccgatatt cccttcgttt
atatgaaata ttagctctta gaataaattt aaaacacaaa aattcagata catttacttt
 
agccgagctt agaggaaaac tgggagttcc tgaaggaaaa ttaaaaagat ggtctgattt
aagagataaa gcgatcaatg ttgcggtaaa agaaattaac caacggacta ctttcgccgt
aacagcacaa ccaaaaaaaa tgggaagatc cgttcaagcc gtagaattta aatgggaacg
aaaagagatt aaacttcctc aaagtatacc attaaaaaca tctaaaagca ttgtttttcc
attagatgga agtattagat atactcaatg ggaagatatt gcacgaaaaa atcttcctaa
gcctttacct gatttagata tggtagcaca acaatttaga aaatggttaa aaaataaaaa
catttcatca aacagtatta atgttttgtc catttttaag aaattttgtg aaaaatacag
aatagaattt aataattaag aattcagtac tcccggggga tcctctagac tgcagaagct
tataaaacga aaggctcagt cgaaagactg ggcctttcgt tttatctgtt gtttgtcggt
gaacgctctc ctgagtagga caaatccgcc gggagcggat ttgaacgttg cgaagcaacg
gcccggaggg tggcgggcag gacgcccgcc ataaactgcc aggcatcaaa ttaagcagaa
ggccatcctg acggatggcc tttgtagaaa agatcaaagg atcttcttga gatccttttt
ttctgcgcgt aatctgctgc ttgcaaacaa aaaaaccacc gctaccagcg gtggtttgtt
tgccggatca agagctacca actctttttc cgaaggtaac tggcttcagc agagcgcaga
taccaaatac tgtccttcta gtgtagccgt agttaggcca ccacttcaag aactctgtag
caccgcctac atacctcgct ctgctaatcc tgttaccagt ggctgctgcc agtggcgata
agtcgtgtct taccgggttg gactcaagac gatagttacc ggataaggcg cagcggtcgg
gctgaacggg gggttcgtgc acacagccca gcttggagcg aacgacctac accgaactga
gatacctaca gcgtgagcta tgagaaagcg ccacgcttcc cgaagggaga aaggcggaca
ggtatccggt aagcggcagg gtcggaacag gagagcgcac gagggagctt ccagggggaa
acgcctggta tctttatagt cctgtcgggt ttcgccacct ctgacttgag cgtcgatttt
tgtgatgctc gtcagggggg cggagcctat ggaaaaacgc cagcaacgcg gcctttttac
ggttcctggc cttttgctgg ccttttgctc acatatgttc aaatatgtat ccgctcatga
gacaataacc ctgataaatg cttcaataat attgaaaaag gaagagtatg gaaaaaaaaa
tcaccggcta taccaccgtg gatattagcc agtggcatcg taaagaacat tttgaagcgt
ttcagagcgt ggcgcagtgc acctataacc agaccgtgca gctggatatc accgcgtttc
tgaaaaccgt gaaaaaaaac aaacacaaat tctacccggc gtttattcat attctggccc
gtctgatgaa cgcgcatccg gaatttcgta tggccatgaa agatggcgaa ctggtgattt
gggatagcgt gcatccgtgc tataccgtgt ttcatgaaca gaccgaaacc tttagcagcc
 
tgtggagcga atatcatgat gattttcgcc agttcctgca tatttatagc caggatgtgg
cgtgctatgg cgaaaacctg gcctattttc cgaaaggctt catcgaaaac atgttctttg
tgagcgcgaa tccgtgggtg agctttacca gcttcgatct gaacgtggcg aacatggata
acttttttgc gccggtgttt accatgggca aatattatac ccagggcgat aaagtgctga
tgccgctggc cattcaggtg catcatgcgg tgtgtgatgg ctttcatgtg ggccgtatgc
tgaacgaact gcagcagtat tgtgatgaat ggcagggcgg tgcgtaataa ctgtcagacc
aagtttactc atatatactt tagattgatt taaaacttca tttttaattt aaaaggatct
aggtgaagat cctttttgat aatctcatga ccaaaatccc ttaacgtgag ttttcgttcc
actgagcgcg gccgc
 
SEQ ID_NO 4 (DNA Gene cassette (G1), 8603 bp):

tctagattca aggtgtcccg ttcctttttc ccctttttgg aggttggtta tgtcctataa

tcacttaatc cagaaacggg cgtttagctt tgtccatcat ggttgtttat cgctcatgat

cgcggcatgt tctgatattt ttcctctaaa aaagataaaa agtcttttcg cttcggcaga

agaggttcat catgaacaaa aattcggcat ttttaaaaat gcctatagct aaatccggaa

cgacacttta gaggtttctg ggtcatcctg attcagacat agtgttttga atatatggag

taagcaatgg cgctggtttc tatgcgtcag ctgctggacc acgcggcgga aaactcttac

ggtctgcctg cgttcaacgt taacaacctg gaacagatgc gtgcgatcat ggaagcggcg

gaccaggtta acgcgccggt tatcgttcag gcgtctgcgg gtgcgcgtaa atacgcgggt

gcgccgttcc tgcgtcacct gatcctggcg gcggttgaag aatttccgca catcccggtt

gttatgcacc aggaccacgg tgcgtctccg gacgtttgcc agcgttctat ccagctgggt

ttctcttctg ttatgatgga cggttctctg ctggaagacg gtaaaacccc gtctacctac

gaatacaacg ttaacgcgac ccgtaccgtt gttaacttct ctcacgcgtg cggtgtttct

gttgaaggtg aaatcggtgt tctgggcaac ctggaaaccg gtgaagcggg tgaagaagac

ggtgttggtg cggcgggtaa actgtctcac gaccagatgc tgacctctgt tgaagacgcg

gttcgtttcg ttaaagacac cggtgttgac gcgctggcga ttgcggttgg cacctctcac

ggtgcgtaca aattcacccg tccgccgacc ggtgacgttc tgcgtatcga ccgtatcaaa

gaaatccacc aggcgctgcc gaacacccac atcgttatgc acggttcttc ttctgttccg

caggaatggc tgaaagttat caacgaatac ggtggtaaaa tcggtgaaac ctacggtgtt
 
ccggttgaag aaatcgttga aggtatcaaa cacggtgttc gtaaagttaa catcgacacc
gacctgcgtc tggcgtctac cggtgcgatc cgtcgtttca tggcggaaaa cccgtctgaa
tttgacccgc gtaaatacct gtctaaaacc gttgaagcga tgaaacagat ttgcctggac
cgttacctgg cgttcggttg cgaaggtcag gcgggtaaaa tcaaaccggt ttctctggaa
aaaatggcga accgttacgc gaaaggtgaa ctgaaccaga tcatcaaata atttaccaac
actactacgt ataaggagag cgttgttatg cgtcgtaaac tggttgcggg taactggaaa
atgaacggtt ctcgtgcgat gctggttgaa ctggacgcga tcgcggcggc ggcgtctgcg
gctccggaaa tcgacgtttc tgttgcggtt ccgttcaccc tgatcgcggc ggcgtctgaa
cgtgttccga ccctggcgat cggtgcggaa gacgttcacg aagcggaatc tggtgcgcac
accggttgcg tttctgcgtc tatggttaaa gaagttggtg cgtctttcac catcgttggt
cactctgaac gtcgttctga ccagcacgaa acctctcacg acgcgtgggc gaaagcggcg
gcggcgcaca aacagggtct gaacgttatc ctgtgctgcg gtgaaaccga agcggaacgt
gacgcggacc gtgcggaacg tgttgttcag gcgcagatcg aaaaatctgt tccggacaac
gcggacggtt cttggttcac cctggcgtac gaaccgcgtt gggcgatcgg caccggtcgt
accccgaccc tggaagaaat cgcggcgatc cacgcgatcg cgcgtgcgaa actgcgtatg
ctgatcggtg acaaagcggc gggtgttcgt atcctgtacg gtggttctgt taccggtgcg
aacgcggcgt ctatcctgga caccccgaac gttgacggtg cgctggttgg tggtgcgtct
ctgaccgcgg cgaaattcgt tccgatcatc gaagcggcgg cgtctctgta atttaccaac
actactacgt ataaggagag cgttgttatg gaaatcgttg cgatcgacat cggtggcacc
cacgcgcgtt tctctatcgc ggaagtttct aacggtcgtg ttctgtctct gggtgaagaa
accaccttca aaaccgcgga acacgcgtct cttcagctgg cgtgggaacg tttcggtgaa
aaactgggtc gtccgctgcc gcgtgcggcg gcgatcgcgt gggcgggtcc ggttcacggt
gaagttctga aactgaccaa caacccgtgg gttctgcgtc cggcgaccct gaacgaaaaa
ctggacatcg acacccacgt tctgatcaac gacttcggtg cggttgcgca cgcggttgcg
cacatggact cttcttacct ggaccacatc tgcggtccgg acgaagcgct gccgtctgac
ggtgttatca ccatcctggg tccgggcacc ggtctgggtg ttgcgcacct gctgcgtacc
gaaggtcgtt acttcgttat cgaaaccgaa ggtggtcaca tcgacttcgc gccgctggac
cgtctggaag acaaaatcct ggcgcgtctg cgtgaacgtt tccgtcgtgt ttctatcgaa
cgtatcatct ctggtccggg tctgggtaac atctacgaag cgctggcggc gatcgaaggt
 
gttccgttct ctctgctgga cgacatcaaa ctgtggcaga tggcgctgga aggtaaagac
aacctggcgg aagcggcgct ggaccgtttc tgcctgtctc tgggtgcgat cgcgggtgac
ctggcgctgg cgcagggtgc gacctctgtt gttatcggtg gtggtgttgg tctgcgtatc
gcgtctcacc tgccggaatc tggtttccgt cagcgtttcg tttctaaagg tcgtttcgaa
cgtgttatgt ctaaaatccc ggttaaactg atcacctacc cgcagccggg tctgctgggt
gcggcggcgg cgtacgcgaa caaatactct gaagttgaat aatttaccaa cactactacg
tataaggaga gcgttgttat ggcggacctg ccggcgaccc cgctgctgga caccgtttct
accccggacg acctgcgtaa actgcgtccg gaccagctgc gtcagctggc ggacgaactg
cgtaccgaaa ccatctctgc ggttggcacc accggtggtc acctgggttc tggtctgggt
gttgttgaac tgaccaccgc gatccactac gttttcgaca ccccgcgtga ccgtctggtt
tgggacgttg gtcaccagtg ctacccgcac aaaatcctga ccggtcgtcg tgaccgtatc
cgtaccctgc gtcagggtgg tggtctgtct ggtttcacca aacgttctga atctgaatac
gacccgttcg gtgcggcgca ctcttctacc tctatctctg cggcgctggg tttcgcggtt
gcgaacaaaa tcgcgggcac ccctggtaaa ggtatcgcgg ttatcggtga cggtgcgatg
tctgcgggta tggcgtacga agcgatgaac aacgcggcgc aggcgggtaa ccgtctggtt
gttatcctga acgacaacga catgtctatc gcgccgccgg ttggtggtct gtctgcgtac
ctgtctcgta tcgtttcttc tcgtgaattt ctgggtttcc gtgacctggc gcgtaaaatc
gcgaaacgtc tgccgcgtcc gatcgcgtct ggtatgcgta aaaccgacga atttgcgcgt
ggtatgacca tgggtggcac cctgttcgaa gaactgggtt tctactacgt tggtccgatc
gacggtcaca acctggacca cctgatcccg gttctggaaa acgttcgtga cgcggaagaa
ggtccgatcc tggttcacgt tgttaccaaa aaaggtaaag gttacgcgcc ggcggaagcg
gcggcggaca aataccacgg tgttcagaaa ttcgacgtta tctctggtgt tcagaccaaa
gcgccgccgg gtccgccgca gtaccagaac gttttcggtg cgcagctggt tgcggaagcg
gcgaaagacg cgcgtatctg cgcgatcacc gcggcgatgc cgtctggcac cggtctggac
gcgttcgcga aagcgtaccc ggaccgtttc ttcgacgttg gtatcgcgga acagcacggt
gttaccttcg cggcgggtct ggcggcgcag ggtctgcgtc cgttctgcgc gatctactct
accttccttc agcgtgcgta cgaccaggtt gttcacgacg ttgcgatcca gaacctgccg
gttcgtttcg cgatcgaccg tgcgggtctg gttggtgcgg acggtgcgac ccacgcgggt
tctttcgacg ttacctacct ggcgaccctg ccgaacttcg ttgttatggc ggcggcggac
 
gaagcggaac tggttcacat gacccacacc gcggttcagt acgacgacgg tccgatcgcg
ctgcgttacc cgcgtggtaa cggcaccggt gttgcgctgc cggaaacccc gcagctgctg
gaaatcggta aaggtcgtgt tgttcgtgaa ggtaaaaccg ttgcgatcct gtctctgggc
acccgtctgg aagaagcgct gaaagcggcg gcgatcctgg aagcgaaagg tctgtctacc
accgttgcgg acctgcgttt cgcgaaaccg ctggacgttg acctgatccg taaactgctg
accacccacg aagttgcggt taccatcgaa gaaaactctg ttggtggttt cggtgcgcac
gttctgacca tggcgtctga cgaaggtctg atcgacggtg gtctgaaact gcgtaccctg
cgtctgccgg acatgtacca ggaccaggac aaaccggaag ttcagtacgc ggaagcgggt
ctggacgcga ccgcgctggt tgacaccgtt ctgaaagcgc tgcgtcacaa ctctggtggt
gttgcggaag cgcgtgcgta atttaccaac actactacgt ataaggagag cgttgttatg
gtttcttacc tggacttcga aaaaccggtt gcggaacttc agacccgtat cgcggaactg
cgtaaagcga cctctgacga aatggacctg gaccgtgaaa tctctgacct ggaagttaaa
gttcagaaac tgctgcgtga cacctacgcg cacctgaccc cgtggcagaa aacccaggtt
gcgcgtcacc cggaacgtcc gcacttccac gactacatct ctgcgctgtg cgaagaattt
atcccgctgg cgggtgaccg tctgttcggt gaagacgaag cgatcatcgg tggtctggcg
cgtatcgacg gtcagcgtgt tatgatcatc ggtcaggaaa aaggtaacga caccgcgtct
cgtctgaaac acaacttcgg tatggcgcgt ccggaaggtt accgtaaagc gatccgtctg
atgaaactgg cggaccgttt cggtctgccg gttatcaccc tggttgacac ctctggtgcg
ttccctggta tcgacgcgga agaacgtggt caggcggaag cgatcgcgcg ttctaccgaa
acctgcctgt ctctgggtgt tccgctgatc tctgttatcg ttggtgaagg tggttctggt
ggtgcgatcg cgatcgcggc ggcgaaccgt ctgctgatgt tcgaacacgc ggtttactct
gttatctctc cggaaggttg cgcgtctatc ctgtggcgtg acgcgggtaa agcgtctgac
gcggcgaccg cgatgaaact gaccgcgacc gacctgtacg gtctgggtat cgttgaccgt
atcgttctgg aaccggttgg tggtgcgcac cgtgacccga aaaccgcgat cgacgcgctg
aaaatggcgc tgcttcagga actggcgttc ctgaaaccga tggaccgtga cgcgctgcgt
tctatgcgtc gtaaaaaatt cctggacatc ggtggttaat ttaccaacac tactacgtat
aaggagagcg ttgttatgtc ttggctggaa aaagttcgta acaaaatccc gttcatcgtt
aaacgtgaaa ccccggacaa cctgtggcac cgttgcccgc actgccagac catgatcttc
aacaaagact acgaagaaaa cctgtctgtt tgcccgaaat gcggtttcca cggtcgtatc
 
ggttctcgtg cgcgtttcga ctctctgttc gacgaaggtg actactctgt tctgccgccg
ccggttgttc cggaagaccc gctgaaattc cgtgacacca aacgttacgt tgaccgtctg
aaagcggcgc gtctgaaatc tggtgaaaac gacgcgctgc tgaacgcgaa aggtcgtctg
gcgggtcgtc agaccgttat cggtgttcag gacttctctt tcatgggtgg ttctatgggt
ctggcggttg gttctgcgtt cgttgcgggt gttaaagcgg cgatcgaatg ccactctccg
tacatcatct tcaccgcggc gggtggtgcg cgtatgcagg aaggtatcct gtctctgatg
cagatgccgc gttctaccgc ggcgatcgcg gaactgcgtg aagcgggtct gccgtacatc
gttgttctga ccgacccgac caccggtggt gttaccgcgt cttacgcgat gctgggtgac
gttcagatcg cggaaccggg tgcgctgatc ggtttcgcgg gtcagcgtgt tatcgaatct
accatccgtg aaaaactgcc ggaaggtttc cagcgtgcgg aatacctgct ggaacacggt
atgatcgaca tggttgttcc gcgtgcggaa atgcgtgacc gtctgatctc tctggttgac
tacctgctgc cggcgaacga agcggcgtaa tttaccaaca ctactacgta taaggagagc
gttgttatga accacctgcg tgcggaaggt ccggcgtctg ttctggcgat cggcaccgcg
aacccggaaa acatcctgct tcaggacgaa tttccggact actacttccg tgttaccaaa
tctgaacaca tgacccagct gaaagaaaaa ttccgtaaaa tctgcgacaa atctatgatc
cgtaaacgta actgcttcct gaacgaagaa cacctgaaac agaacccgcg tctggttgaa
cacgaaatgc agaccctgga cgcgcgtcag gacatgctgg ttgttgaagt tccgaaactg
ggtaaagacg cgtgcgcgaa agcgatcaaa gaatggggtc agccgaaatc taaaatcacc
cacctgatct tcacctctgc gtctaccacc gacatgccgg gtgcggacta ccactgcgcg
aaactgctgg gtctgtctcc gtctgttaaa cgtgttatga tgtaccagct gggttgctac
ggtggtggca ccgttctgcg tatcgcgaaa gacatcgcgg aaaacaacaa aggtgcgcgt
gttctggcgg tttgctgcga catcatggcg tgcctgttcc gtggtccgtc tgaatctgac
ctggaactgc tggttggtca ggcgatcttc ggtgacggtg cggcggcggt tatcgttggt
gcggaaccgg acgaatctgt tggtgaacgt ccgatcttcg aactggtttc taccggtcag
accatcctgc cgaactctga aggcaccatc ggtggtcaca tccgtgaagc gggtctgatc
ttcgacctgc acaaagacgt tccgatgctg atctctaaca acatcgaaaa atgcctgatc
gaagcgttca ccccgatcgg tatctctgac tggaactcta tcttctggat cacccaccct
ggtggtaaag cgatcctgga caaagttgaa gaaaaactgc acctgaaatc tgacaaattc
gttgactctc gtcacgttct gtctgaacac ggtaacatgt cttcttctac cgttctgttc
 
gttatggacg aactgcgtaa acgttctctg gaagaaggta aatctaccac cggtgacggt
ttcgaatggg gtgttctgtt cggtttcggt ccgggtctga ccgttgaacg tgttgttgtt
cgttctgttc cgatcaaata ctaatttacc aacactacta cgtataagga gagcgttgtt
atggcggtta aacacctgat cgttctgaaa ttcaaagacg aaatcaccga agcgcagaaa
gaagaatttt tcaaaaccta cgttaacctg gttaacatca tcccggcgat gaaagacgtt
tactggggta aagacgttac ccagaaaaac aaagaagaag gttacaccca catcgttgaa
gttaccttcg aatctgttga aaccatccag gactacatca tccacccggc gcacgttggt
ttcggtgacg tttaccgttc tttctgggaa aaactgctga tcttcgacta caccccgcgt
aaataatatt ttttaatatt atgaactgaa tttaagaggc tgcctcccga ttttatcgga
aggcagcctt ttttatactg cag
 
SEQ ID_NO 5 (DNA Gene cassette (G2), 8033 bp):

tctagaatca acaacccgaa tcctatcgta atgatgtttt gcccgatcag cctcaatcga

caattttacg cgtttcgatc gaagcaggga cgacaattgg ctgggaacgg tatactggaa

taaatggtct tcgttatggt attgatgttt ttggtgcatc ggccccggcg aatgatctat

atgctcattt cggcttgacc gcagtcggca tcacgaacaa ggtgttggcc gcgatcgccg

gtaagtcggc acgttaaaaa atagctatgg aatatagtag ctacttaata agttaggaga

ataaacatga aatctgttac catcctgggt gcgaccggtt ctgttggttc ttctaccctg

gacctgatcg aacgtgaacc ggaccgtttc cgtgttgttg cgctgaccgc gaactgcgac

gttgcgaaac tggcggcggc ggcgatccgt accaaagcgg aatttgcggt tgttgcggac

gcgggttgcc tgccggacct gcgtgacgcg ctggcgggca ccggtatccg tgcgggtggt

ggtgcggaag cgatcgcgga agcggcggcg tctggtgcgg actggaccat gggtgcgatc

gttggttgcg cgggtctgaa accggttatg gcggcgatcg aacagggtgg caccgttgtt

atcgcgaaca aagaaccgct ggtttctgcg ggtgacgtta tcctggcggc ggcgaaacgt

accggtgcga ccctgctgcc tgcggactct gaacacaacg cgatcttcca gtgcttcgac

gcgtctcgtc cggaacgtgt tcgtcgtatc atcctgacct gctctggtgg tccgttccgt

gactggacca ccgaacagat gcgtggtgtt accctggaac aggcggttaa acacccgaac
 
tggtctatgg gtgcgaaaat ctctgttgac tctgcgacct gcatgaacaa aggtctggaa
ctgatcgaag cggcgcgtct gttcccgatc ccgcacgacc gtatcgaaat cgttatccac
ccgcagtctg ttgttcactc tctggttgac tacgttgacg gttctgttct ggcgcagctg
ggtccgccgg acatgcgtac cccgatcgcg cacaccctgg cgtggccgga ccgtatggcg
accccgatgg cgccgctgga cctggttgcg atcggtcgtc tggacttcga agcgccggac
ccggttcgtt tcccggcgct gcgtctggcg cgtgaagcgc tggacgcggg tggtgcgcgt
ccggcgatcc tgaacgcggc gaacgaagtt gcggttgaag cgttcctgaa acgtcgtatc
cgtttcctgg aaatcgcggc gatcgttgaa gacaccctgg aacgttacaa cccggcggcg
ccggactctg ttgacgcggt tctggcgatc gacgcggaag cgcgtgttct ggcgggtgaa
cgtgttaaag actgcgcggt ttaatttacc aacactacta cgtataagga gagcgttgtt
atggcgcacg gtcagcgtat cgttgcgatc atcgttgcgg cgggcaccgg tgttcgtgcg
ggtggtgcgg ttccgaaaca gtacgcgcgt atcggtggtc gtccgatgat cgcgtactct
tacgacgcgt tcgcggcgca cccggcgatt gttgaaacca tcgttgttgt tgcggacggt
cagcaggaca tggcggttga cgcgctgggt ggtgacgcga ccatcgttgt tggtggtgcg
acccgtcgtg aatctgttgc gaacggtgtt gcggcggttg gtgcggcgac ccacgttctg
atccacgacg cggcgcgtcc gttcctgtct gcggcggtta tcgactctct ggttgttgcg
ctgaaaaccc gtgacgcggc gatcccggtt ctgccggtta ccgacaccct ggcgcgtggt
tctgttctgc tgggtgacat cgttccgcgt gcggacctga accgtgttca gaccccgcag
gcgttcgcgg ttgaaggtct gcgtgcggcg cacgcggttt ggccggcgga cgaagaagcg
accgacgacg cgcagatggt tcgtcgtctg ggtgttggtg ttgcgctggt tcagggttct
gcgatgctgg aaaaagttac ccacccggaa gacttcgcgg cggcggaagc gcgtgttgcg
gcgaaaacct cttacgaaac ccgtaccgcg atgggtttcg acgttcaccg tctggaaacc
ggtgaagaac tgtggctggg tggtgttctg atcccgcacg acaaaggtct gtctggtcac
tctgacgcgg acgttgcgct gcacgcgctg accgacgcgc tgctgggcac catcgcggcg
ggtgacatcg gcacccactt cccgccgtct gacccgcagt ggaaaggtgc ggactctgcg
cagttccttc agcacgcggc gaaactgatc gcggacaaac acggtcgtat cgacttcgtt
gacctgacca tcatgtgcga agcgccgaaa atcggtccgc accgtgcggc gatgaccgcg
cgtatcgcgg acctgctggg tctgaccctg gaccgtgttt ctgttaaagc gaccaccacc
gaacgtctgg gtttcaccgg tcgtggtgaa ggtatcgcgg cgcaggcggt tgcgaccgtt
 
cgtgttccgg gtgcgccggc gtaatttacc aacactacta cgtataagga gagcgttgtt
atgccggcga ccaccgaacc ggcgccggcg aaaatcaacc tggcgctgca cgttcgtcgt
cgtcgtgacg acggttacca cgaactggaa accctgttcg cgttcgcgac cgacggtgac
atcgttaccg ttgaaccggc ggcggcgaac tctttcacca tcaccggtcc gttcgcggac
gcgctgctgc cggcgccgaa acacaacacc accccggcgt ctgcgggtgt tgcggcgtgg
ggtggtaacc tggttaccgc ggcggcggac gcgttcaccg cggcgttcgc gccggacacc
caccacgcga tcaccctgga aaaacacctg ccgatcgcgt ctggtatcgg tggtggttct
gcggacgcgg cggcgaccct gcgtgcgctg gcgcgtctgc actgcgttgg tccgaccgac
ccgcgtctgc acgcgatcgc ggcgaccctg ggtgcggacg ttccggcgtg cctggcgaac
cgtaccaccc tgggcaccgg taaaggtgac accctggcga acctggaagg tctgtctggc
accccgctgc tgctggttaa ccctggtgtt gcggtttcta ccgcggcggt tttcaaaacc
tgggacggtc aggaccgtgg tccgatcccg gaaggtgacc tgctgacccg tgcgaccgcg
ggtcgtaacg acctggaacc gccggcgctg gcgctggcgc cggttatcgc ggacgttcgt
accctgctgg acgcggcgca cgaagttctg ctgtctcgta tgtctggttc tggtgcgacc
tgcttcgcgc tgttcgaaac cgcggaagcg cgtgacgcgg cggcgaccgc ggcgctggcg
cgtggttggt ggaccctggc gaccaccctg gcgtaattta ccaacactac tacgtataag
gagagcgttg ttatgtctct gcgtccgtgg cgtgacatca cccgtcgtac ctctcgtcag
atcatggttg gcaccgttcc ggttggtggt gacgcgccgg ttaccgttca gaccatgacc
aacaccccga ccgacgacgt taaagcgacc gttgaccaga tccgtcgttg cgaagaagcg
ggtgttgaca tcatccgtgt ttcttgcccg gacgttgaat ctaccaccgc gctgaaacag
atcgttcgtg cgtctcgtgt tccgatcgtt gcggacatcc acttccacta caaacgtgcg
ctggaagcgg cggacgcggg tgcggcgtgc ctgcgtatca accctggtaa catcggttct
gcggcgcgtg ttaaagaagt tgttgacgcg gcgaaatcta acggttgcgc gatccgtatc
ggtgttaacg gtggttctct ggaacgtcac ctgctggaaa aatacggtga accgtgcccg
gacgcgctgg ttgaatctgc gatggaccac atcaaactgc ttcaggacca cgacttccac
gaatttaaag ttgcggttaa agcgtctgac ctgttcctgg cggttgcggc gtaccagcag
ctggcggaac aggttgactg cccgctgcac ctgggtatca ccgaagcggg tggtttcgtt
ggtggcaccg ttaaatctgc gatcggtatg ggttctctgc tgtggtacgg tatcggtgac
accatccgtg tttctctgtc tgcggaaccg gaagaagaag ttcgtgttgg tttcgaaatc
 
ctgaaagcgc tgggtatccg taaccgtggt gttcgtgttg tttcttgccc gtcttgcgcg
cgtcagggtt tcgacgttat ccgtaccgtt caggcgctgg aagaacgtct tcagcacatc
cgtaccccga tgtctctgtc tgttctgggt tgcgttgtta acggtccggg tgaagcgcgt
gaaaccgaca tcggtatcac cggtggtggt aacggtaaac acatggttta cctgtctggt
gttaccgacc accacgttca ggacgcggac atggttgacc acatcgttcg tctggttgaa
gcgaaagcgg cggaaatcga agcggcggac gcggcgatcc tggcgtctgt tgcggcgtaa
tttaccaaca ctactacgta taaggagagc gttgttatgc agatcctgct ggcgaacccg
cgtggtttct gcgcgggtgt tgaccgtgcg atctctatcg ttgaaaacgc gctggcgatc
tacggtgcgc cgatctacgt tcgtcacgaa gttgttcaca accgttacgt tgttgactct
ctgcgtgaac gtggtgcgat cttcatcgaa cagatctctg aagttccgga cggtgcgatc
ctgatcttct ctgcgcacgg tgtttctcag gcggttcgta acgaagcgaa atctcgtgac
ctgaccgttt tcgacgcgac ctgcccgctg gttaccaaag ttcacatgga agttgcgcgt
gcgtctcgtc gtggtgaaga atctatcctg atcggtcacg cgggtcaccc ggaagttgaa
ggcaccatgg gtcagtactc taacccggaa ggtggtatgt acctggttga atctccggac
gacgtttgga aactgaccgt taaaaacgaa gaaaaactgt ctttcatgac ccagaccacc
ctgtctgttg acgacacctc tgacgttatc gacgcgctgc gtaaacgttt cccgaaaatc
gttggtccgc gtaaagacga catctgctac gcgaccacca accgtcagga agcggttcgt
gcgctggcgg aacaggcgga agttgttctg gttgttggtt ctaaaaactc ttctaactct
aaccgtctgg cggaactggc gcagcgtatg ggtaaacgtg cgttcctgat cgacgacgcg
aaagacatcc aggaagaatg ggttaaaggt gttaactgcg ttggtgttac cgcgggtgcg
tctgcgccgg acatcctggt tcagaacgtt gttgcgcgtc tgcaccagct gggtggtggt
gaagcgatcc cgctggaagg tcgtgaagaa aacatcgttt tcgaagttcc gaaagaactg
cgtgttgaca tccgtgaagt tgactaattt accaacacta ctacgtataa ggagagcgtt
gttatgttct acctgggtga atttggtctg gaccgtaaag accagcacgt tggtctggcg
aaccagcagt actctgcgac cccggcgaaa gacttcaccg aaaccctgtt cgttcaccac
tctctgccgc agaccaaagt tgacgaagtt gacatctcta cctctgttgc gggtctggac
ttcgcgttcc cgttcttcat caacgcgatg accggtggtt ctaaaaaaac ccgtgaaatc
aaccgtctgc tgggtatcat gggtcacttc ggtaaaatcg cgctggcgtc tggttctgtt
tctgcggcga tcaaagaccc gtctgttgcg gaaaccttct ctgttatgcg tcgtgaaaac
 
ccgtacggta tcatcttcgc gaacctgggt gcgcaccact ctgttgaaaa cgcgaaacgt
gcggttgacc tgctggaagc gaacgcgatc cagatccacg ttaacgcgcc gcaggaaatc
gttatgccgg aaggtgaccg tgacttcacc atgtggctga aaaacatcga aaccctggtt
cgtgaaatgg aagttccggt tatcgttaaa gaagttggtt tcggtatgtc tcgtgaaacc
gttgcgcagc tggcgtctgc gggtgttcag accatcgacg tttctggcac cggtggcacc
gacttcgcga aaatcgaaaa cgcgcgtcgt accttcaacg actacgcgta cctggaaggt
tggggtcagt ctaccgttac ctctctggtt gaagcgatgt ctgtttctga agaagtttgc
ccgtctctga tcgcgtctgg tggtatcaaa accccgctgg acatcgttaa atctctggcg
ctgggtgcgg acctggttgg tatgtctaac cacttccttc agtacgttaa agacggtaaa
ggtcaccgtt tcgacgacgg tcttcaggcg atcaaagttt accagtggca gatcgcggaa
atcatgacca tgctgggtgc gaaaaacatc gcggaactgc gtcagaaaga cctggttctg
gcgccgaacg ttcagaactg gtgcgaagcg cgtggtatcg actggaaagc gtacgcgcgt
cgttctgcga acgtttctta atttaccaac actactacgt ataaggagag cgttgttatg
gcgggtgcga tcaccgacca gctgcgtcgt tacctgcacg gtcgtcgtcg tgcggcggcg
cacatgggtt ctgactacga cggtctgatc gcggacctgg aagacttcgt tctgggtggt
ggtaaacgtc tgcgtccgct gttcgcgtac tggggttggc acgcggttgc gtctcgtgaa
ccggacccgg acgttctgct gctgttctct gcgctggaac tgctgcacgc gtgggcgctg
gttcacgacg acctgatcga ccgttctgcg acccgtcgtg gtcgtccgac cgcgcagctg
cgttacgcgg cgctgcaccg tgaccgtgac tggcgtggtt ctccggacca gttcggtatg
tctgcggcga tcctgctggg tgacctggcg caggtttggg cggacgacat cgtttctaaa
gtttgccagt ctgcgctggc gccggacgcg cagcgtcgtg ttcaccgtgt ttgggcggac
atccgtaacg aagttctggg tggtcagtac ctggacatcg ttgcggaagc gtctgcggcg
gaatctatcg aatctgcgat gaacgttgcg accctgaaaa ccgcgtgcta caccgtttct
cgtccgcttc agctgggcac cgcggcggcg gcggaccgtt ctgacgttgc ggcgatcttc
gaacacttcg gtgcggacct gggtgttgcg ttccagctgc gtgacgacgt tctgggtgtt
ttcggtgacc cggcggttac cggtaaaccg tctggtgacg acctgaaatc tggtaaacgt
accgttctgg ttgcggaagc ggttgaactg gcggaccgtt ctgacccgct ggcggcgaaa
ctgctgcgta cctctatcgg cacccgtctg accgacgcgc aggttcgtga actgcgtacc
gttatcgaag cggttggtgc gcgtgcggcg gcggaatctc gtatcgcggc gctgacccag
 
cgtgcgctgg cgaccctggc gtctgcgccg atcaacgcga ccgcgaaagc gggtctgtct

gaactggcga tgatggcggc gaaccgttct gcgtaatatt ttttaatatt atgaactgaa

tttaagaggc tgcctcccga ttttatcgga aggcagcctt ttttataaag ctt
 
SEQ ID_NO 6 (DNA Gene cassette (G3), 8531 bp):

6

cccgggtgtc tatactccag ttactcaata cgtaacaata atcagtttat cctaactata

gaatcgcatg agaagcgata acgtttcacc ataagcaata tattcattgc aacagtggaa

ttgccttatg cgtcaaggaa ggatagatca ttgacggact gagttcaaaa agagactcgt

ctaaaagatt ttaagaaagg tttcgatatg acccgtgaag ttgttgttgt ttctggtgtt

cgtaccgcga tcggcacctt cggtggttct ctgaaagacg ttgcgccggc ggaactgggt

gcgctggttg ttcgtgaagc gctggcgcgt gcgcaggttt ctggtgacga cgttggtcac

gttgttttcg gtaacgttat ccagaccgaa ccgcgtgaca tgtacctggg tcgtgttgcg

gcggttaacg gtggtgttac cgttaacgcg ccggcgctga ccgttaaccg tctgtgcggt

tctggtcttc aggcgatcgt ttctgcggcg cagaccatcc tgctgggtga caccgacgtt

gcgatcggtg gtggtgcgga atctatgtct cgtgcgccgt acctggcgcc ggcggcgcgt

tggggtgcgc gtatgggtga cgcgggtctg gttgacatga tgctgggtgc gctgcacgac

ccgttccacc gtatccacat gggtgttacc gcggaaaacg ttgcgaaaga atacgacatc

tctcgtaccc agcaggacga agcggcgctg gaatctcacc gtcgtgcgtc tgcggcgatc

aaagcgggtt acttcaaaga ccagatcgtt ccggttgttt ctaaaggtcg taaaggtgac

gttaccttcg acaccgacga acacgttcgt cacgacgcga ccatcgacga catgaccaaa

ctgcgtccgg ttttcgttaa agaaaacggc accgttaccg cgggtaactc ttctggtctg

aacgacgcgg cggcggcggt tgttatgatg gaacgtgcgg aagcggaacg tcgtggtctg

aaaccgctgg cgcgtctggt ttcttacggt cacgcgggtg ttgacccgaa aaccatgggt

atcggtccgg ttccggcgac caaaatcgcg ctggaacgtg cgggtcttca ggtttctgac

ctggacgtta tcgaagcgaa cgaagcgttc gcggcgcagg cgtgcgcggt taccaaagcg

ctgggtctgg acccggcgaa agttaacccg aacggttctg gtatctctct gggtcacccg

atcggtgcga ccggtgcgct gatcaccgtt aaagcgctgc acgaactgaa ccgtgttcag
 
ggtcgttacg cgctggttac catgtgcatc ggtggtggtc agggtatcgc ggcgatcttc
gaacgtatct aatttaccaa cactactacg tataaggaga gcgttgttat gctgtacaaa

ggtgacaccc tgtacctgga ctggctggaa gacggtatcg cggaactggt tttcgacgcg
ccgggttctg ttaacaaact ggacaccgcg accgttgcgt ctctgggtga agcgatcggt
gttctggaac agcagtctga cctgaaaggt ctgctgctgc gttctaacaa agcggcgttc
atcgttggtg cggacatcac cgaatttctg tctctgttcc tggttccgga agaacagctg
tctcagtggc tgcacttcgc gaactctgtt ttcaaccgtc tggaagacct gccggttccg
accatcgcgg cggttaacgg ttacgcgctg ggtggtggtt gcgaatgcgt tctggcgacc
gactaccgtc tggcgacccc ggacctgcgt atcggtctgc cggaaaccaa actgggtatc
atgccgggtt tcggtggttc tgttcgtatg ccgcgtatgc tgggtgcgga ctctgcgctg
gaaatcatcg cggcgggtaa agacgttggt gcggaccagg cgctgaaaat cggtctggtt
gacggtgttg ttaaagcgga aaaactggtt gaaggtgcga aagcggttct gcgtcaggcg
atcaacggtg acctggactg gaaagcgaaa cgtcagccga aactggaacc gctgaaactg
tctaaaatcg aagcgaccat gtctttcacc atcgcgaaag gtatggttgc gcagaccgcg
ggtaaacact acccggcgcc gatcaccgcg gttaaaacca tcgaagcggc ggcgcgtttc
ggtcgtgaag aagcgctgaa cctggaaaac aaatctttcg ttccgctggc gcacaccaac
gaagcgcgtg cgctggttgg tatcttcctg aacgaccagt acgttaaagg taaagcgaaa
aaactgacca aagacgttga aaccccgaaa caggcggcgg ttctgggtgc gggtatcatg
ggtggtggta tcgcgtacca gtctgcgtgg aaaggtgttc cggttgttat gaaagacatc
aacgacaaat ctctgaccct gggtatgacc gaagcggcga aactgctgaa caaacagctg
gaacgtggta aaatcgacgg tctgaaactg gcgggtgtta tctctaccat ccacccgacc
ctggactacg cgggtttcga ccgtgttgac atcgttgttg aagcggttgt tgaaaacccg
aaagttaaaa aagcggttct ggcggaaacc gaacagaaag ttcgtcagga caccgttctg
gcgtctaaca cctctaccat cccgatctct gaactggcga acgcgctgga acgtccggaa
aacttctgcg gtatgcactt cttcaacccg gttcaccgta tgccgctggt tgaaatcatc
cgtggtgaaa aatcttctga cgaaaccatc gcgaaagttg ttgcgtgggc gtctaaaatg
ggtaaaaccc cgatcgttgt taacgactgc cctggtttct tcgttaaccg tgttctgttc
ccgtacttcg cgggtttctc tcagctgctg cgtgacggtg cggacttccg taaaatcgac
 
aaagttatgg aaaaacagtt cggttggccg atgggtccgg cgtacctgct ggacgttgtt
ggtatcgaca ccgcgcacca cgcgcaggcg gttatggcgg cgggtttccc gcagcgtatg
cagaaagact accgtgacgc gatcgacgcg ctgttcgacg cgaaccgttt cggtcagaaa
aacggtctgg gtttctggcg ttacaaagaa gactctaaag gtaaaccgaa aaaagaagaa
gacgcggcgg ttgaagacct gctggcggaa gtttctcagc cgaaacgtga cttctctgaa
gaagaaatca tcgcgcgtat gatgatcccg atggttaacg aagttgttcg ttgcctggaa
gaaggtatca tcgcgacccc ggcggaagcg gacatggcgc tggtttacgg tctgggtttc
ccgccgttcc acggtggtgc gttccgttgg ctggacaccc tgggttctgc gaaatacctg
gacatggcgc agcagtacca gcacctgggt ccgctgtacg aagttccgga aggtctgcgt
aacaaagcgc gtcacaacga accgtactac ccgccggttg aaccggcgcg tccggttggt
gacctgaaaa ccgcgtaatt taccaacact actacgtata aggagagcgt tgttatgctg
taccagtctg aaacccttca gctgcactgg ctggaaaacg gtatcgcgga actggttttc
gacgcgccgg gttctgttaa caaactggac acccagaccg ttgcgaacct gggtgaagcg
ctggttgttc tggaaaaaca gtctgaactg aaaggtctgc tgctgcgttc tgcgaaagcg
gcgttcatcg ttggtgcgga catcaccgaa tttctgtctc tgttcaacga accgccggaa
aaactgcacc agtggctggt tttcgcgaac gacatcttca accgtctgga agacctgccg
gttccgacca tcgcggcgat caacggttac gcgctgggtg gtggttgcga atgcatcctg
gcgaccgact tccgtgttgc gtctccggaa gcgcgtatcg gtctgccgga aaccaaactg
ggtatcatgc cgggtttcgg tggttctgtt cgtctgccgc gtctgctggg tgcggactct
gcgctggaaa tcatcgcggc gggtaaagac atcatcgcga aagacgcgct gaaagttggt
ctggttgacg cggttgttgc gccggaaaaa ctggttgacg cggcgctgtc tatcctgaac
caggcgatcg acggtaaact ggactggcag gcggcgcgtc gtccgaaact ggaaccgctg
aaactgaacc cgaccgaagc ggcgatgtgc ttcaccatcg cgaaaggtat ggttatgcag
gttgcgggta aacactaccc ggcgccgctg accgcggtta aaaccatcga agcggcggcg
aaattcggtc gtgcggaagc gctgatcctg gaaaccaact ctttcgttcc gctggcgggt
tctaacgaag cgcgtgcgct ggttggtatc ttcctgaacg accagtacgt taaaggtcag
gcgaaaaaac tgtctaaagg tgtttctgcg ccgaaacagg cggcggttct gggtgcgggt
atcatgggtg gtggtatcgc gtaccagtct gcgctgaaag gtgttccggt tatcatgaaa
gacatcaacg acaaatctct gaccctgggt atgaacgaag cggcgaaact gctgaacaaa
 
cagctggaac gtggtaaaat cgacggtctg aaaatggcga acatcctggc gaccatccag
ccgaccctgg actacgcggg tatcgaacgt gcgcaggtta tcgttgaagc ggttgttgaa
aacccgaaag ttaaagcggc ggttctggcg gaagttgaaa ccctgatcgg tgaagaaacc
gttctggcgt ctaacacctc taccatcccg atcgaccagc tggcgaaatc tctgaaacgt
ccggaaaact tctgcggtat gcacttcttc aacccggttc accgtatgcc gctggttgaa
atcatccgtg gcaccaaaac ctctgacaaa accatcgcgg cggttgttgc gtacgcgacc
cagatgggta aaaccccgat cgttgttaac gactgccctg gtttcttcgt taaccgtgtt
ctgttcccgt acctggcggg tttcggtatg ctggttcgtg acggtgcgga cttccgtcag
atcgacaaag ttatggaaaa acagttcggt tggccgatgg gtccggcgta cctgctggac
gttgttggta tcgacaccgc gcaccacgcg caggcggtta tggcggttgg tttcccggaa
cgtatgaaca aagactaccg tgacgcggtt gacgttatgt tcgacaacca gcgtttcggt
cagaaaaacg gtcagggttt ctaccgttac acccaggaca ccaaaggtaa accgcgtaaa
gaaaacgacg aacaggttga cgcgctgctg gcgcaggttt ctcagccgct tcagaaattc
tctgacgacg acatcatcgc gcgtaccatg atcccgatga tcaacgaagt tgttcgttgc
ctggaagaag gtatcatcgc gtctccggcg gaaggtgaca tggcgctggt ttacggtctg
ggtttcccgc cgttccacgg tggtgttttc cgttacctgg acaccatcgg ttctgcgaac
tacgttgaaa tggcgcagcg ttacacccac ctgggtgcgc tgtaccaggt tccgccgggt
ctgcgtgcga aagcggaaca caacgaatct tactacccgg ttgcggcggc gctgctggac
gtttctatct ctcagccggc gtaatttacc aacactacta cgtataagga gagcgttgtt
atgtcttgcc cggcgtctcc gtctgcggcg gttgtttctg cgggtgcgct gtgcctgtgc
gttgcgaccg ttctgctggc gaccggttct aacccgaccg cgctgtctac cgcgtctacc
cgttctccga cctctctggt tcgtggtgtt gaccgtggtc tgatgcgtcc gaccaccgcg
gcggcgctga ccaccatgcg tgaagttccg cagatggcgg aaggtttctc tggtgaagcg
acctctgcgt gggcggcggc gggtccgcag tgggcggcgc cgctggttgc ggcggcgtct
tctgcgctgg cgctgtggtg gtgggcggcg cgtcgttctg ttcgtcgtcc gctggcggcg
ctggcggaac tgccgaccgc ggttacccac ctggcgccgc cgatggcgat gttcaccacc
accgcgaaag ttatccagcc gaaaatccgt ggtttcatct gcaccaccac ccacccgatc
ggttgcgaaa aacgtgttca ggaagaaatc gcgtacgcgc gtgcgcaccc gccgacctct
ccgggtccga aacgtgttct ggttatcggt tgctctaccg gttacggtct gtctacccgt
 
atcaccgcgg cgttcggtta ccaggcggcg accctgggtg ttttcctggc gggtccgccg
accaaaggtc gtccggcggc ggcgggttgg tacaacaccg ttgcgttcga aaaagcggcg
ctggaagcgg gtctgtacgc gcgttctctg aacggtgacg cgttcgactc taccaccaaa
gcgcgtaccg ttgaagcgat caaacgtgac ctgggcaccg ttgacctggt tgtttactct
atcgcggcgc cgaaacgtac cgacccggcg accggtgttc tgcacaaagc gtgcctgaaa
ccgatcggtg cgacctacac caaccgtacc gttaacaccg acaaagcgga agttaccgac
gtttctatcg aaccggcgtc tccggaagaa atcgcggaca ccgttaaagt tatgggtggt
gaagactggg aactgtggat tcaggcgctg tctgaagcgg gtgttctggc ggaaggtgcg
aaaaccgttg cgtactctta catcggtccg gaaatgacct ggccggttta ctggtctggc
accatcggtg aagcgaaaaa agacgttgaa aaagcggcga aacgtatcac ccagcagtac
ggttgcccgg cgtacccggt tgttgcgaaa gcgctggtta cccaggcgtc ttctgcgatc
ccggttgttc cgctgtacat ctgcctgctg taccgtgtta tgaaagaaaa aggcacccac
gaaggttgca tcgaacagat ggttcgtctg ctgaccacca aactgtaccc ggaaaacggt
gcgccgatcg ttgacgaagc gggtcgtgtt cgtgttgacg actgggaaat ggcggaagac
gttcagcagg cggttaaaga cctgtggtct caggtttcta ccgcgaacct gaaagacatc
tctgacttcg cgggttacca gaccgaattt ctgcgtctgt tcggtttcgg tatcgacggt
gttgactacg accagccggt tgacgttgaa gcggacctgc cgtctgcggc gcagcagtaa
tttaccaaca ctactacgta taaggagagc gttgttatgt ctggtgcggc ggacgttgaa
cgtgtttacg cggcgatgga agaagcggcg ggtctgctgg acgtttcttg cgcgcgtgaa
aaaatctacc cgctgctgac cgttttccag gacaccctga ccgacggtgt tgttgttttc
tctatggcgt ctggtcgtcg ttctaccgaa ctggacttct ctatctctgt tccggtttct
cagggtgacc cgtacgcgac cgttgttaaa gaaggtctgt tccaggcgac cggttctccg
gttgacgaac tgctggcgga caccgttgcg cacctgccgg tttctatgtt cgcgatcgac
ggtgaagtta ccggtggttt caaaaaaacc tacgcgttct tcccgaccga cgacatgccg
ggtgttgcgc agctggcggc gatcccgtct atgccggcgt ctgttgcgga aaacgcggaa
ctgttcgcgc gttacggtct ggacaaagtt cagatgacct ctatggacta caaaaaacgt
caggttaacc tgtacttctc tgacctgaaa caggaatacc ttcagccgga atctgttgtt
gcgctggcgc gtgaactggg tctgcgtgtt ccgggtgaac tgggtctgga attttgcaaa
cgttctttcg cggtttaccc gaccctgaac tgggacaccg gtaaaatcga ccgtctgtgc
 
ttcgcggcga tctctaccga cccgaccctg gttccgtctg aagacgaacg tgacatcgaa
atgttccgta actacgcgac caaagcgccg tacgcgtacg ttggtgaaaa acgtaccctg
gtttacggtc tgaccctgtc ttctaccgaa gaatactaca aactgggtgc gtactaccac
atcaccgaca tccagcgtca gctgctgaaa gcgttcgacg cgctggaaga ctaatatttt
ttaatattat gaactgaatt taagaggctg cctcccgatt ttatcggaag gcagcctttt
ttataggatc c
 
SEQ ID_NO 7 (DNA plasmid CRISRP_Loci-pUCZM-7, 4723 bp):
ccgggaattt gacccttatt ttgaccctct tttttcgagg gtataaaaaa tcctttcgat
tcaatatgtt acatatgagc atatttttta gggttatttt gcctttttgg cgagatatcc
ctttatttta ggggcaattc tatcttttgc ctctagttca ctgccgcaca ggcagcttag
aaaggagacc gaggtctcag ttcactgccg cacaggcagc ttagaaagga tcctctagac
tgcagaagct tataaaacga aaggctcagt cgaaagactg ggcctttcgt tttatctgtt
gtttgtcggt gaacgctctc ctgagtagga caaatccgcc gggagcggat ttgaacgttg
cgaagcaacg gcccggaggg tggcgggcag gacgcccgcc ataaactgcc aggcatcaaa
ttaagcagaa ggccatcctg acggatggcc tttgtagaaa agatcaaagg atcttcttga
gatccttttt ttctgcgcgt aatctgctgc ttgcaaacaa aaaaaccacc gctaccagcg
gtggtttgtt tgccggatca agagctacca actctttttc cgaaggtaac tggcttcagc
agagcgcaga taccaaatac tgtccttcta gtgtagccgt agttaggcca ccacttcaag
aactctgtag caccgcctac atacctcgct ctgctaatcc tgttaccagt ggctgctgcc
agtggcgata agtcgtgtct taccgggttg gactcaagac gatagttacc ggataaggcg
cagcggtcgg gctgaacggg gggttcgtgc acacagccca gcttggagcg aacgacctac
accgaactga gatacctaca gcgtgagcta tgagaaagcg ccacgcttcc cgaagggaga
aaggcggaca ggtatccggt aagcggcagg gtcggaacag gagagcgcac gagggagctt
ccagggggaa acgcctggta tctttatagt cctgtcgggt ttcgccacct ctgacttgag
cgtcgatttt tgtgatgctc gtcagggggg cggagcctat ggaaaaacgc cagcaacgcg
gcctttttac ggttcctggc cttttgctgg ccttttgctc attcaaatat gtatccgctc
atgagacaat aaccctgata aatgcttcaa taatattgaa aaaggaagag tatggaaaaa
 
aaaatcaccg gctataccac cgtggatatt agccagtggc atcgtaaaga acattttgaa
gcgtttcaga gcgtggcgca gtgcacctat aaccagaccg tgcagctgga tatcaccgcg
tttctgaaaa ccgtgaaaaa aaacaaacac aaattctacc cggcgtttat tcatattctg
gcccgtctga tgaacgcgca tccggaattt cgtatggcca tgaaagatgg cgaactggtg
atttgggata gcgtgcatcc gtgctatacc gtgtttcatg aacagaccga aacctttagc
agcctgtgga gcgaatatca tgatgatttt cgccagttcc tgcatattta tagccaggat
gtggcgtgct atggcgaaaa cctggcctat tttccgaaag gcttcatcga aaacatgttc
tttgtgagcg cgaatccgtg ggtgagcttt accagcttcg atctgaacgt ggcgaacatg
gataactttt ttgcgccggt gtttaccatg ggcaaatatt atacccaggg cgataaagtg
ctgatgccgc tggccattca ggtgcatcat gcggtgtgtg atggctttca tgtgggccgt
atgctgaacg aactgcagca gtattgtgat gaatggcagg gcggtgcgta ataactgtca
gaccaagttt actcatatat actttagatt gatttaaaac ttcattttta atttaaaagg
atctaggtga agatcctttt tgataatctc atgaccaaaa tcccttaacg tgagttttcg
ttccactgag cgtcgacggt accatgatta tcggcttctc aaaacatgga cagggcaaag
ctgctccagc cataaaatac ctgctcgatc agcacagaga gggcagagaa aatcatattc
ctgaagtggt gagaggtaat ccagacttaa ccgcatccat tatcgacagc ctgactttca
aacatcacta tactagcgga gttctttctt tcgctgccga agatcatgtt tctgccgagc
aggaaaaaga tattatcaac gatttcgaaa agctggcttt tgctggcctg caagcagacc
agtttaatat cctttgggtg cgccattctc atgcgggtca tcatgaactt catttcatca
ctccaaggat agaactttca tccaagaagt ccttcaacat caagcccccc ggtaagaata
cccaaaagga atttgatgac ttgcggagtg ccatcaatgc caaatatggc tttgctgacc
cagaagaccc gatgagagcg agaaacgtca aaacacctag ctatgaacta aaaatcgcta
ggaatgtcct tagagagcaa aaagcgccct ctgaggacat aagacagctt attgatagcc
ttttaactga aagagccgtc caaggcctga tacatgacag aaaaactctt ctcactaatg
ttgaagagtt aggcttcgaa gttactcgcg caggcaagaa ttatattacc gtcaaagacc
ctgaaagctc aaaacgattt cgtctcaaag gccaactcta tgaacaagac tttacctcta
gccgaacgat tgaaacagca gcagctaaaa gaacaagaga ttattctcga cctgactcaa
cagcagctag acaatttgag gaacaagtta aactccacat tcgaaaacga gctgaattca
atagaaaacg atatcgagcg actgaaccgg agcttaggat ggaaattcct cctatctcgc
 
tggctgatac cagtcgtgac cagcatcatg atattgtttg gcctgctctt gggcgcagaa
gggataatca agctctatac ccaccagatc aaagctttgc aggaggaaaa agcagagatg
gaagtgaccg tcgaacagct caacaaagca ggtgggaaga tgcaattcag ccattgcgga
gaaaaagaaa ggctttgcat cagagtaaac acacgagaga tcttcgggat aaccagatat
tatctgatcg tgaaagaacg gaaatagaca atgacggaat tggaacaggt tttactgaac
gccttggaaa atttacagca gagattcgat caacaacaga aaagtttatt acacgcgcaa
agcgaattac ggcagatgtt cgaaactacc tcaaccgaga atacttattt acgacagcaa
gtagaaatct tggtcgagcg attgaacagt tttcccagat gaaaacctta agtcgaaagc
tacaaaaacg gagaggtcgt tctcgctctc gataaacttg aaaaggacgc taattccgaa
aagtttttta tctttctgat agtcgatttt agttggcgat ttccgataaa aaagccctga
acttattgaa ctttcctata tttcaataag ttcaataagt tcagggcttt tttattcaaa
aaaatcaatg ccttacgact ttaaaaacca cttttgggga gtgaaaaacc acttttgggg
agtgaaaaac cacttttggg gagtgaaaaa ccacttcaaa aaatatatta agtggttaat
aaagttgtaa tgagaaaaac actacaaata attaaagata gagccggcaa taaagatcaa
aatttacatg caggtgaaat tatagatgga catttttcag accttaaaaa tccgcctagc
ctagctgcac taaaactcta tgagttgctt attaaatacg cagcatataa tatagataaa
aatatatggc ataatatttc tgtatcagaa atgcaaaaaa tcaaaggaat aaagaactat
aactcaacag agctaactca attattaaaa gaacttagag ctgttgttat ggaatataga
gcaaaagata gaactattat tactggtctt ttagatattg ctgcggtcat atctaaccca
gacacaggat atctagagat acaatggaaa tttggagaaa gttttagaga tttaatagct
aagtcagaat attgggcaat tgttgattat agaacaacgt tagagatgac gagccgatat
tcccttcgtt tatatgaaat attagctctt agaataaatt taaaacacaa aaattcagat
acatttactt tagccgagct tagaggaaaa ctgggagttc ctgaaggaaa attaaaaaga
tggtctgatt taagagataa agcgatcaat gttgcggtaa aagaaattaa ccaacggact
actttcgccg taacagcaca accaaaaaaa atgggaagat ccgttcaagc cgtagaattt
aaatgggaac gaaaagagat taaacttcct caaagtatac cattaaaaac atctaaaagc
attgtttttc cattagatgg aagtattaga tatactcaat gggaagatat tgcacgaaaa
aatcttccta agcctttacc tgatttagat atggtagcac aacaatttag aaaatggtta
aaaaataaaa acatttcatc aaacagtatt aatgttttgt ccatttttaa gaaattttgt
 gaaaaataca gaatagaatt taataattaa gaattcagta ctc
SEQ ID_NO 8 (DNA, CRISPR Plasmid Repeat Sequence, 28 bp): gttcactgcc gcacaggcag cttagaaa 
SEQ ID_NO 9 (DNA, Zymomonas mobilis Origen of replication 1 (Ori1), 2362bp): cggaaaggtc atcctgaaaa ttctattcgt attctgatca accagatcgt gaatgatcgg

gcgatgagcc ggaaacttgt cccgtgatca gaaccggcgc agcctcttcc cttttatcta

tttcaaattc aggcctgcca cagcggagaa tcttcgctgt ggcttttttg cgtccaagac

cggagcccgc acaatgaccg cctcttcctc ttcagcgccc ctctcggatg cctctgtctt

ttctttagag cctgtctctg cgtcttttga acaaacgacg cccgtaacca gtccttttat

cgacaggcta ggccgtttca agcgtggcac ttatcgaacc atgttggttg aaacgggtat

gccatttgac agcgcaaaga ttatggcaga tcaggccgca gaacaacata aaagagagcg

cacattctct aaattaatgc ccttggccaa tggcgctcat cccgaaattt cggcctcaaa

taccaatatt gaggctacag cctctgcatc agatttcgac gaaatcgaat attttgacca

gatggtatca aacggcgttg atcctagaga tgccgcagat attgctcgca aaacagctat

tgcgagacgt cgagaccctc tcgaacgagc cattcgctat aaaacccgcc gtcaaactcc

tgatcctgtg catcgggatt cttatgaggt gggcgaacgc gagaagtcgg tctggaagcc

ggtcaatccg caagagattg gtgcctatct ggaagcggtt gatcaatatt cgatcaaaac

caagggctta agcgataagg ccgttcgtct tctaaagatg ctgtttcgga tggtcgactt

taaaactggc cgtctggagc cgaccctcga cacaatatgc gatcgtgtcg gttatgcccg

tgcgacggtc gtcaaattac tgcggcaatt acaggattta ggcttcatcc gctggatacg

ccggtcaatc aagatcaagg cggacggcaa aggcccccgc cgcaagcaga ccagtaacgc

ctacggcttc ttgtctccca aggcttggcc agatttggcc agacaggttt tcgagcgtgt

gatgcgccgg aggcatgcgc ctattcctga cgatatcgac cacgcccaag aggcggacaa

agccgaaacc aaggctatcg ttgaaaactt gccgcccgaa gatttcgtca aagcaatatc

gggcgataaa gagccaacag agttaacaga ttcgttaata cagctcgcca aatctatcga
 
gcgggcggaa caacgggaac aagaagaaca ggctaagaag gcagcacagg ccggtcagga
agaggttaaa acatcttccc aaacacctaa ggaggatacc tcaaaaaagc ccccaatctc
gggcaatcaa cgcgagttca attcttatac tctatcctgt tatacttatt tatctaatag
cgaccataca aaactaaaaa ccaaagaaga agataaagac tgcactgctc ctgctaccgc
aggcagtgca gaccctattt atgatcgaaa acctgacagc aatcctttcc tctcaactct
caaagctcgc ggttatcgtc ctgatcaaat caacgcttgc ctcgaaaaca gagataaacg
gaaaaagaaa ggatggatgc ctctgtcacc cgatgaaacc gcccgcctcc ttattcaaac
aaaacagatg tgcccataac aacgaccact atccttctca aagcatatcg tccgttaatc
tctcggcgct gctcgcttcg ctcacaggct cggcgagaaa gaaatttgcc tttcggggga
ctatctaatt cctaacaatt cgcattatcg ataatgtaat aaaaggctaa tctttaaaaa
ccaaattaca gcgattacat catttctatg aatgtgtatt atacataata tgagaagtcg
tgatttgatc aaagaagtta aaaaagccaa tattaccagc ttgacagata taaaataggc
cttggttgat ttcgtgaaag aactggaaga ttgcacaatt cttctttctg gtgatatctt
tgacccgacg gccctcagtc aatgagtgtg cgccgcattt ctgcccattc aatcagggcc
tgcattgaag ggccgagagc gtatcccata ccgctgagct cgtattctac ctttggcggg
acctgcggat agaccgttcg gagcacgatg cgatcctttt ccagttgctt gagctgctgg
atcagcatct tctggttgat gccgtcgatc tgcttttcca gtgaggagaa gcggattggg
ccttttgcgg caaacaactg gcagatgata acaatcttcc attttccttc gagtaccctg
agcgcttctg tcgtcgcctc ag
 
SEQ ID_NO 10 (DNA, Zymomonas mobilis Origen of replication 2 (Ori2), 2801 bp): gcaccgcaaa ggacatgctg
atcgcgagat cggcgcgcta ctcgacactg tcataacaga

tcacattcgg agatagctga tgaatactgc gcagaatcag actcttaaaa gagcgttttt

gtcatcagaa gccgcagcag ctgactcttc agctgactgc ggcttttcta tgtctgaaaa

agatatgcca gattttgaag aaatgacgcc agccagccct tttatcgaca ggctaggccg

tttcaagcgc ggcacttatc gaagcatgtt gatgcagacg gggctttctt atgaacgcgc

gaaaggcatg gcagacaatg cggcggaaga atttaaaaaa tcaaaagcaa agtcgaaatc

tttgccattg gccaagagcg aaatcgctga acttggggct tcagaagcaa cgattgaagt

aacagctcat caggtaacat tttctgaatc acaggcacaa ccagaacctt tcgatgctta
 
ctgctgcttg caacagttaa aagatcaggg cgtggacggt gccacagctg cgaagattgc
aaatcaggcc tacaagcatc acaaagaagc cgaaaatttt gacgaagcgg tatatttcaa
ggaattagtc gatactggca tagatgcggt gatggctgct aatatggctc gtaaaacagc
cgaagctaag cgccgcgacc cgctggaaag atacggtatc cgtttaaagc cgcgccgcac
gcctgatcct gttcaccgtg actcttatga tgttggcgag cgcgaaaata cggtctggaa
gccggtcaat ccgaatgaga tcggggctta tctggaagca gtcgatcaat attcaatcaa
gaccggcaat atcagtgata gagccgtccg gctgctcaag atgctgtttc ggatggtgga
cttcaagaca ggtcgattag agccgacgct tgatacaatc tgcgaatggg tcggctatgc
acgggcaacg gttgtccgct tgctaaaaca gcttcaagag caaggtttta tccgctggat
acgccgttca atcaagatca agacggatgg ggctgggccc cgccgcaaac agaccagtaa
cgcttacggt ttcttgtcgc ctaaatcttg gccggagatg gcaagaaagg ttttcgagcg
cgtgatgcgc cggaagaatg cgccggtctc tgctgatctt gctcatgctc aagaagccga
caaagtcgaa accggtgcca tgattgaaag ccagccgaat gacgactggg tgaaagaaat
cttcgcacag aagcgcgcgc aacatccgac gagtgacgag cagaaaaaca aaaaagaaca
ggccgatgat ctggcagaga ctcttgccag cttcgccaaa tctatcgagc gggcagaaca
acgggaacaa gaagaacagg ccaagaaagc catttcggcc gagcaaaatg aggttaaaac
accctacaaa acactcaagg aggacacctc aaaaacgccc caaatttcgg cagttgagcg
cgagttcaat tttgatactc tatcccgtta tagttatttt aataataacc actccaatca
atttaaaaac caaaaagaga ttaaggactg cactgctcct gctgccgcag gcagtgcaga
cgttgttttg aaaacaaaac ctcaaaccga cactttccta aaaacccttc aaactcgcgg
ttaccgtcaa gaccagacca acgcttgcct cgaaaacaga gataaacgga aaaagaatgg
atggatgcct ctgtcagcgg acgaaaccgc ccgcctcctt atacaaacaa ggcagatgcg
accgtgatac cgacagctat ccttctcaaa gcacaccgcc cattaatctc tcggcgctgc
tcgcttcgct cacaggctcg gcgaaaaaga aatttgcctt ttggggggct attaacttct
aacaactcgc attatcgaaa tgtctttaaa taggtttatt ctctaaaagc gtttcgacat
ggcttttact gttatccatc atccaatctt cagtcggtct tgagcgatat ccaatcaaac
gaacaattct atcccagtca gcttgtagat ctctttcaaa tataggctca tgatgggcta
tccgattacg cagcatccga atttcaccca aatctctata gagactctgt ctctggattt
taatctcttg ctgtggatca agattcggga acacgttcaa caaatagaga tcccaaagcc
 
gtttatcatg gcgctttgtg aacatagcct gccagaatac aaaattcagt tctggtatta
cctttccggt cgtttccgcc ttcaaacagg ctttgtttac atccttcgat gcgctgtatc
ctgatttgac aactggaagg cttcgtcgga aaccttctga ccacggccac tgatctccat
aggtatagga tagcgcttcc gccacagcat ttcgggtcgc tatttctgtt atatgaaggg
gggctagcaa tgcgccggat agctgggcat tccacacata taaatccaga gcgacgagat
catcttttcc caatgccctg actgtgtctt catatcttcc aagtcgggga gccgataagg
tctttctgat agaggtaatc ttgtcttgaa ttgacagcat ggctacccat ttatatgatc
aaccacatat tcgcctcggg tctttgttgg cttatgcctc cccctgagga caaaagatac
aaaaaagacc tcgcttacgg cggggtcttt ttttatccat tttcctgtat tgtataaagt
ctttgctagc ctgacggaaa acgtaatcct gcggttaatc g
 
SEQ ID_NO 11 (DNA, Zymomonas mobilis Origen of replication 3 (Ori3), 2707 bp):atgattatcg gcttctcaaa acatggacag ggcaaagctg ctccagccat aaaatacctg

ctcgatcagc acagagaggg cagagaaaat catattcctg aagtggtgag aggtaatcca

gacttaaccg catccattat cgacagcctg actttcaaac atcactatac tagcggagtt

ctttctttcg ctgccgaaga tcatgtttct gccgagcagg aaaaagatat tatcaacgat

ttcgaaaagc tggcttttgc tggcctgcaa gcagaccagt ttaatatcct ttgggtgcgc

cattctcatg cgggtcatca tgaacttcat ttcatcactc caaggataga actttcatcc

aagaagtcct tcaacatcaa gccccccggt aagaataccc aaaaggaatt tgatgacttg

cggagtgcca tcaatgccaa atatggcttt gctgacccag aagacccgat gagagcgaga

aacgtcaaaa cacctagcta tgaactaaaa atcgctagga atgtccttag agagcaaaaa

gcgccctctg aggacataag acagcttatt gatagccttt taactgaaag agccgtccaa

ggcctgatac atgacagaaa aactcttctc actaatgttg aagagttagg cttcgaagtt

actcgcgcag gcaagaatta tattaccgtc aaagaccctg aaagctcaaa acgatttcgt

ctcaaaggcc aactctatga acaagacttt acctctagcc gaacgattga aacagcagca

gctaaaagaa caagagatta ttctcgacct gactcaacag cagctagaca atttgaggaa

caagttaaac tccacattcg aaaacgagct gaattcaata gaaaacgata tcgagcgact

gaaccggagc ttaggatgga aattcctcct atctcgctgg ctgataccag tcgtgaccag

catcatgata ttgtttggcc tgctcttggg cgcagaaggg ataatcaagc tctataccca
 
ccagatcaga gctttgcagg aggaaaaagc agagatggaa gtgaccgtcg aacagctcaa
caaagcaggt gggaagatgc aattcagcca ttgcggagaa aaagaaaggc tttgcatcag
agtaaacaca cgagagatct tcgggataac cagatattat ctgatcgtga aagaacggaa
atagacaatg acggaattgg aacaggtttt actgaacgcc ttggaaaatt tacagcagag
attcgatcaa caacagaaaa gtttattaca cgcgcaaagc gaattacggc agatgttcga
aactacctca accgagaata cttatttacg acagcaagta gaaatcttgg tcgagcgatt
gaacagtttt cccagatgaa aaccttaagt cgaaagctac aaaaacggag aggtcgttct
cgctctcgat aaacttgaaa aggacgctaa ttccgaaaag ttttttatct ttctgatagt
cgattttagt tggcgatttc cgataaaaaa gccctgaact tattgaactt tcctatattt
caataagttc aataagttca gggctttttt attcaaaaaa atcaatgcct tacgacttta
aaaaccactt ttggggagtg aaaaaccact tttggggagt gaaaaaccac ttttggggag
tgaaaaacca cttcaaaaaa tatattaagt ggttaataaa gttgtaatga gaaaaacact
acaaataatt aaagatagag ccggcaataa agatcaaaat ttacatgcag gtgaaattat
agatggacat ttttcagacc ttaaaaatcc gcctagccta gctgcactaa aactctatga
gttgcttatt aaatacgcag catataatat agataaaaat atatggcata atatttctgt
atcagaaatg caaaaaatca aaggaataaa gaactataac tcaacagagc taactcaatt
attaaaagaa cttagagctg ttgttatgga atatagagca aaagatagaa ctattattac
tggtctttta gatattgctg cggtcatatc taacccagac acaggatatc cagagataca
atggaaattt ggagaaagtt ttagagattt aatagctaag tcagaatatt gggcaattgt
tgattataga acaacgttag agatgacgag ccgatattcc cttcgtttat atgaaatatt
agctcttaga ataaatttaa aacacaaaaa ttcagataca tttactttag ccgagcttag
aggaaaactg ggagttcctg aaggaaaatt aaaaagatgg tctgatttaa gagataaagc
gatcaatgtt gcggtaaaag aaattaacca acggactact ttcgccgtaa cagcacaacc
aaaaaaaatg ggaagatccg ttcaagccgt agaatttaaa tgggaacgaa aagagattaa
acttcctcaa agtataccat taaaaacatc taaaagcatt gtttttccat tagatggaag
tattagatat actcaatggg aagatattgc acgaaaaaat cttcctaagc ctttacctga
tttagatatg gtagcacaac aatttagaaa atggttaaaa aataaaaaca tttcatcaaa
cagtattaat gttttgtcca tttttaagaa attttgtgaa aaatacagaa tagaatttaa
taattaa
 
SEQ ID_NO 12 (DNA, Origen of replication E. coli (pBR322), 668 bp):
gtagaaaaga tcaaaggatc ttcttgagat cctttttttc tgcgcgtaat ctgctgcttg
caaacaaaaa aaccaccgct accagcggtg gtttgtttgc cggatcaaga gctaccaact
ctttttccga aggtaactgg cttcagcaga gcgcagatac caaatactgt ccttctagtg
tagccgtagt taggccacca cttcaagaac tctgtagcac cgcctacata cctcgctctg
ctaatcctgt taccagtggc tgctgccagt ggcgataagt cgtgtcttac cgggttggac
tcaagacgat agttaccgga taaggcgcag cggtcgggct gaacgggggg ttcgtgcaca
cagcccagct tggagcgaac gacctacacc gaactgagat acctacagcg tgagctatga
gaaagcgcca cgcttcccga agggagaaag gcggacaggt atccggtaag cggcagggtc
ggaacaggag agcgcacgag ggagcttcca gggggaaacg cctggtatct ttatagtcct
gtcgggtttc gccacctctg acttgagcgt cgatttttgt gatgctcgtc aggggggcgg
agcctatgga aaaacgccag caacgcggcc tttttacggt tcctggcctt ttgctggcct
tttgctca
 
SEQ ID_NO 13 (DNA, Escherichia coli origin of replication, colE1, 589 bp): ttgagatcct ttttttctgc gcgtaatctg
ctgcttgcaa acaaaaaaac caccgctacc

agcggtggtt tgtttgccgg atcaagagct accaactctt tttccgaagg taactggctt

cagcagagcg cagataccaa atactgtcct tctagtgtag ccgtagttag gccaccactt

caagaactct gtagcaccgc ctacatacct cgctctgcta atcctgttac cagtggctgc

tgccagtggc gataagtcgt gtcttaccgg gttggactca agacgatagt taccggataa

ggcgcagcgg tcgggctgaa cggggggttc gtgcacacag cccagcttgg agcgaacgac

ctacaccgaa ctgagatacc tacagcgtga gctatgagaa agcgccacgc ttcccgaagg

gagaaaggcg gacaggtatc cggtaagcgg cagggtcgga acaggagagc gcacgaggga

gcttccaggg ggaaacgcct ggtatcttta tagtcctgtc gggtttcgcc acctctgact

tgagcgtcga tttttgtgat gctcgtcagg ggggcggagc ctatggaaa
 
SEQ ID_NO 14 (DNA, chloramphenicol-resistance-cassette (CamR), Escherichia coli, 870 bp):
ttcaaatatg tatccgctca tgagacaata accctgataa atgcttcaat aatattgaaa

aaggaagagt atggaaaaaa aaatcaccgg ctataccacc gtggatatta gccagtggca

tcgtaaagaa cattttgaag cgtttcagag cgtggcgcag tgcacctata accagaccgt

gcagctggat atcaccgcgt ttctgaaaac cgtgaaaaaa aacaaacaca aattctaccc

ggcgtttatt catattctgg cccgtctgat gaacgcgcat ccggaatttc gtatggccat

gaaagatggc gaactggtga tttgggatag cgtgcatccg tgctataccg tgtttcatga

acagaccgaa acctttagca gcctgtggag cgaatatcat gatgattttc gccagttcct

gcatatttat agccaggatg tggcgtgcta tggcgaaaac ctggcctatt ttccgaaagg

cttcatcgaa aacatgttct ttgtgagcgc gaatccgtgg gtgagcttta ccagcttcga

tctgaacgtg gcgaacatgg ataacttttt tgcgccggtg tttaccatgg gcaaatatta

tacccagggc gataaagtgc tgatgccgct ggccattcag gtgcatcatg cggtgtgtga

tggctttcat gtgggccgta tgctgaacga actgcagcag tattgtgatg aatggcaggg

cggtgcgtaa taactgtcag accaagttta ctcatatata ctttagattg atttaaaact

tcatttttaa tttaaaagga tctaggtgaa gatccttttt gataatctca tgaccaaaat

cccttaacgt gagttttcgt tccactgagc
 
SEQ ID_NO 15 (DNA, ampicillin-resistance-cassette (AmpR), Escherichia coli, 1068 bp): ttcaaatatg
tatccgctca tgagacaata accctgataa atgcttcaat aatattgaaa

aaggaagagt atgagtattc aacatttccg tgtcgccctt attccctttt ttgcggcatt

ttgccttcct gtttttgctc acccagaaac gctggtgaaa gtaaaagatg ctgaagatca

gttgggtgca cgagtgggtt acatcgaact ggatctcaac agcggtaaga tccttgagag

ttttcgcccc gaagaacgtt ttccaatgat gagcactttt aaagttctgc tatgtggcgc

ggtattatcc cgtattgacg ccgggcaaga gcaactcggt cgccgcatac actattctca

gaatgacttg gttgagtact caccagtcac agaaaagcat cttacggatg gcatgacagt

aagagaatta tgcagtgctg ccataaccat gagtgataac actgcggcca acttacttct

gacaacgatc ggaggaccga aggagctaac cgcttttttg cacaacatgg gggatcatgt
 
aactcgcctt gatcgttggg aaccggagct gaatgaagcc ataccaaacg acgagcgtga
caccacgatg cctgtagcaa tggcaacaac gttgcgcaaa ctattaactg gcgaactact
tactctagct tcccggcaac aattaataga ctggatggag gcggataaag ttgcaggacc
acttctgcgc tcggcccttc cggctggctg gtttattgct gataaatctg gagccggtga
gcgtggttct cgcggtatca ttgcagcact ggggccagat ggtaagccct cccgtatcgt
agttatctac acgacgggga gtcaggcaac tatggatgaa cgaaatagac agatcgctga
gataggtgcc tcactgatta agcattggta actgtcagac caagtttact catatatact
ttagattgat ttaaaacttc atttttaatt taaaaggatc taggtgaaga tcctttttga
taatctcatg accaaaatcc cttaacgtga gttttcgttc cactgagc
 
SEQ ID_NO 16 (DNA, kanamycin-resistance-cassette (KanR), Escherichia coli, 1005 bp): ttcaaatatg
tatccgctca tgagacaata accctgataa atgcttcaat aatattgaaa

aaggaagagt atgattgaac aggatggcct gcatgcgggt agcccggcag cgtgggtgga

acgtctgttt ggctatgatt gggcgcagca gaccattggc tgctctgatg cggcggtgtt

tcgtctgagc gcgcagggtc gtccggtgct gtttgtgaaa accgatctga gcggtgcgct

gaacgagctg caggatgaag cggcgcgtct gagctggctg gccaccaccg gtgttccgtg

tgcggcggtg ctggatgtgg tgaccgaagc gggccgtgat tggctgctgc tgggcgaagt

gccgggtcag gatctgctgt ctagccatct ggcgccggca gaaaaagtga gcattatggc

ggatgccatg cgtcgtctgc ataccctgga cccggcgacc tgtccgtttg atcatcaggc

gaaacatcgt attgaacgtg cgcgtacccg tatggaagcg ggcctggtgg atcaggatga

tctggatgaa gaacatcagg gcctggcacc ggcagagctg tttgcgcgtc tgaaagcgag

catgccggat ggcgaagatc tggtggtgac ccatggtgat gcgtgcctgc cgaacattat

ggtggaaaat ggccgtttta gcggctttat tgattgcggc cgtctgggcg tggcggatcg

ttatcaggat attgcgctgg ccacccgtga tattgcggaa gaactgggcg gcgaatgggc

ggatcgtttt ctggtgctgt atggcattgc ggcaccggat agccagcgta ttgcgtttta

tcgtctgctg gatgaatttt tctaataact gtcagaccaa gtttactcat atatacttta

gattgattta aaacttcatt tttaatttaa aaggatctag gtgaagatcc tttttgataa

tctcatgacc aaaatccctt aacgtgagtt ttcgttccac tgagc
 
SEQ ID_NO 17 (DNA, tetracyclin-resistance-cassette (TetR), Escherichia coli, 792 bp): atgagggaag
cggtgatcgc cgaagtatcg actcaactat cagaggtagt tggcgtcatc

gagcgccatc tcgaaccgac gttgctggcc gtacatttgt acggctccgc agtggatggc

ggcctgaagc cacacagtga tattgatttg ctggttacgg tgaccgtaag gcttgatgaa

acaacgcggc gagctttgat caacgacctt ttggaaactt cggcttcccc tggagagagc

gagattctcc gcgctgtaga agtcaccatt gttgtgcacg acgacatcat tccgtggcgt

tatccagcta agcgcgaact gcaatttgga gaatggcagc gcaatgacat tcttgcaggt

atcttcgagc cagccacgat cgacattgat ctggctatct tgctgacaaa agcaagagaa

catagcgttg ccttggtagg tccagcggcg gaggaactct ttgatccggt tcctgaacag

gatctatttg aggcgctaaa tgaaacctta acgctatgga actcgccgcc cgactgggct

ggcgatgagc gaaatgtagt gcttacgttg tcccgcattt ggtacagcgc agtaaccggc

aaaatcgcgc cgaaggatgt cgctgccgac tgggcaatgg agcgcctgcc ggcccagtat

cagcccgtca tacttgaagc tagacaggct tatcttggac aagaagaaga tcgcttggcc

tcgcgcgcag atcagttgga agaatttgtc cactacgtga aaggcgagat caccaaggta

gtcggcaaat aa
 
SEQ ID_NO 18 (DNA, Spectinomycin resistance cassette (SpectinomycinR), Escherichia coli, 792 bp):

atgagggaag cggtgatcgc cgaagtatcg actcaactat cagaggtagt tggcgtcatc

gagcgccatc tcgaaccgac gttgctggcc gtacatttgt acggctccgc agtggatggc

ggcctgaagc cacacagtga tattgatttg ctggttacgg tgaccgtaag gcttgatgaa

acaacgcggc gagctttgat caacgacctt ttggaaactt cggcttcccc tggagagagc

gagattctcc gcgctgtaga agtcaccatt gttgtgcacg acgacatcat tccgtggcgt

tatccagcta agcgcgaact gcaatttgga gaatggcagc gcaatgacat tcttgcaggt

atcttcgagc cagccacgat cgacattgat ctggctatct tgctgacaaa agcaagagaa

catagcgttg ccttggtagg tccagcggcg gaggaactct ttgatccggt tcctgaacag

gatctatttg aggcgctaaa tgaaacctta acgctatgga actcgccgcc cgactgggct

ggcgatgagc gaaatgtagt gcttacgttg tcccgcattt ggtacagcgc agtaaccggc
 
aaaatcgcgc cgaaggatgt cgctgccgac tgggcaatgg agcgcctgcc ggcccagtat

cagcccgtca tacttgaagc tagacaggct tatcttggac aagaagaaga tcgcttggcc

tcgcgcgcag atcagttgga agaatttgtc cactacgtga aaggcgagat caccaaggta

gtcggcaaat aa
 
SEQ ID_NO 19 (DNA, leader sequence in the crispr plasmid, 147 bp):

aatttgaccc ttattttgac cctctttttt cgagggtata aaaaatcctt tcgattcaat

atgttacata tgagcatatt ttttagggtt attttgcctt tttggcgaga tatcccttta

ttttaggggc aattctatct tttgcct
 
SEQ ID_NO 20 (DNA, rrnB T1 terminator sequence, Escherichia coli, 202 bp):ataaaacgaa aggctcagtc
gaaagactgg gcctttcgtt ttatctgttg tttgtcggtg

aacgctctcc tgagtaggac aaatccgccg ggagcggatt tgaacgttgc gaagcaacgg

cccggagggt ggcgggcagg acgcccgcca taaactgcca ggcatcaaat taagcagaag

gccatcctga cggatggcct tt
 
SEQ ID_NO 21 (DNA, Zymomonas mobilis Lambda red gen 1, 417 bp):atggatatta atactgaaac tgagatcaag
caaaagcatt cactaacccc ctttcctgtt

ttcctaatca gcccggcatt tcgcgggcga tattttcaca gctatttcag gagttcagcc

atgaacgctt attacattca ggatcgtctt gaggctcaga gctgggcgcg tcactaccag

cagctcgccc gtgaagagaa agaggcagaa ctggcagacg acatggaaaa aggcctgccc

cagcacctgt ttgaatcgct atgcatcgat catttgcaac gccacggggc cagcaaaaaa

tccattaccc gtgcgtttga tgacgatgtt gagtttcagg agcgcatggc agaacacatc

cggtacatgg ttgaaaccat tgctcaccac caggttgata ttgattcaga ggtataa
 
SEQ ID_NO 22 (DNA, Zymomonas mobilis Lambda red gen 2, 786 bp):atgagtactg cactcgcaac gctggctggg aagctggctg aacgtgtcgg catggattct

gtcgacccac aggaactgat caccactctt cgccagacgg catttaaagg tgatgccagc

gatgcgcagt tcatcgcatt actgatcgtt gccaaccagt acggccttaa tccgtggacg

aaagaaattt acgcctttcc tgataagcag aatggcatcg ttccggtggt gggcgttgat

ggctggtccc gcatcatcaa tgaaaaccag cagtttgatg gcatggactt tgagcaggac

aatgaatcct gtacatgccg gatttaccgc aaggaccgta atcatccgat ctgcgttacc

gaatggatgg atgaatgccg ccgcgaacca ttcaaaactc gcgaaggcag agaaatcacg

gggccgtggc agtcgcatcc caaacggatg ttacgtcata aagccatgat tcagtgtgcc

cgtctggcct tcggatttgc tggtatctat gacaaggatg aagccgagcg cattgtcgaa

aatactgcat acactgcaga acgtcagccg gaacgcgaca tcactccggt taacgatgaa

accatgcagg agattaacac tctgctgatc gccctggata aaacatggga tgacgactta

ttgccgctct gttcccagat atttcgccgc gacattcgtg catcgtcaga actgacacag

gccgaagcag taaaagctct tggattcctg aaacagaaag ccgcagagca gaaggtggca

gcatga
 
SEQ ID_NO 23 (DNA, Zymomonas mobilis Lambda red gen 3, 681 bp): atgacaccgg acattatcct gcagcgtacc gggatcgatg tgagagctgt cgaacagggg

gaagatgcgt ggcacaaatt acggctcggc gtcatcaccg cttcagaagt tcacaacgtg

atagcaaaac cccgctccgg aaagaagtgg cctgacatga aaatgtccta cttccacacc

ctgcttgctg aggtttgcac cggtgtggct ccggaagtta acgctaaagc actggcctgg

ggaaaacagt acgagaacga cgccagaacc ctgtttgaat tcacttccgg cgtgaatgtt

actgaatccc cgatcatcta tcgcgacgaa agtatgcgta ccgcctgctc tcccgatggt

ttatgcagtg acggcaacgg ccttgaactg aaatgcccgt ttacctcccg ggatttcatg

aagttccggc tcggtggttt cgaggccata aagtcagctt acatggccca ggtgcagtac

agcatgtggg tgacgcgaaa aaatgcctgg tactttgcca actatgaccc gcgtatgaag

cgtgaaggcc tgcattatgt cgtgattgag cgggatgaaa agtacatggc gagttttgac
 
gagatcgtgc cggagttcat cgaaaaaatg gacgaggcac tggctgaaat tggttttgta

tttggggagc aatggcgatg a
 
SEQ ID_NO 24 (DNA, Streptococcus pyogenes Cas9, 4107 bp) atggataaga aatactcaat aggcttagat
atcggcacaa atagcgtcgg atgggcggtg

atcactgatg aatataaggt tccgtctaaa aagttcaagg ttctgggaaa tacagaccgc

cacagtatca aaaaaaatct tataggggct cttttatttg acagtggaga gacagcggaa

gcgactcgtc tcaaacggac agctcgtaga aggtatacac gtcggaagaa tcgtatttgt

tatctacagg agattttttc aaatgagatg gcgaaagtag atgatagttt ctttcatcga

cttgaagagt cttttttggt ggaagaagac aagaagcatg aacgtcatcc tatttttgga

aatatagtag atgaagttgc ttatcatgag aaatatccaa ctatctatca tctgcgaaaa

aaattggtag attctactga taaagcggat ttgcgcttaa tctatttggc cttagcgcat

atgattaagt ttcgtggtca ttttttgatt gagggagatt taaatcctga taatagtgat

gtggacaaac tatttatcca gttggtacaa acctacaatc aattatttga agaaaaccct

attaacgcaa gtggagtaga tgctaaagcg attctttctg cacgattgag taaatcaaga

cgattagaaa atctcattgc tcagctcccc ggtgagaaga aaaatggctt atttgggaat

ctcattgctt tgtcattggg tttgacccct aattttaaat caaattttga tttggcagaa

gatgctaaat tacagctttc aaaagatact tacgatgatg atttagataa tttattggcg

caaattggag atcaatatgc tgatttgttt ttggcagcta agaatttatc agatgctatt

ttactttcag atatcctaag agtaaatact gaaataacta aggctcccct atcagcttca

atgattaaac gctacgatga acatcatcaa gacttgactc ttttaaaagc tttagttcga

caacaacttc cagaaaagta taaagaaatc ttttttgatc aatcaaaaaa cggatatgca

ggttatattg atgggggagc tagccaagaa gaattttata aatttatcaa accaatttta

gaaaaaatgg atggtactga ggaattattg gtgaaactaa atcgtgaaga tttgctgcgc

aagcaacgga cctttgacaa cggctctatt ccccatcaaa ttcacttggg tgagctgcat

gctattttga gaagacaaga agacttttat ccatttttaa aagacaatcg tgagaagatt

gaaaaaatct tgacttttcg aattccttat tatgttggtc cattggcgcg tggcaatagt

cgttttgcat ggatgactcg gaagtctgaa gaaacaatta ccccatggaa ttttgaagaa

gttgtcgata aaggtgcttc agctcaatca tttattgaac gcatgacaaa ctttgataaa
 
aatcttccaa atgaaaaagt actaccaaaa catagtttgc tttatgagta ttttacggtt
tataacgaat tgacaaaggt caaatatgtt actgaaggaa tgcgaaaacc agcatttctt
tcaggtgaac agaagaaagc cattgttgat ttactcttca aaacaaatcg aaaagtaacc
gttaagcaat taaaagaaga ttatttcaaa aaaatagaat gttttgatag tgttgaaatt
tcaggagttg aagatagatt taatgcttca ttaggtacct accatgattt gctaaaaatt
attaaagata aagatttttt ggataatgaa gaaaatgaag atatcttaga ggatattgtt
ttaacattga ccttatttga agatagggag atgattgagg aaagacttaa aacatatgct
cacctctttg atgataaggt gatgaaacag cttaaacgtc gccgttatac tggttgggga
cgtttgtctc gaaaattgat taatggtatt agggataagc aatctggcaa aacaatatta
gattttttga aatcagatgg ttttgccaat cgcaatttta tgcagctgat ccatgatgat
agtttgacat ttaaagaaga cattcaaaaa gcacaagtgt ctggacaagg cgatagttta
catgaacata ttgcaaattt agctggtagc cctgctatta aaaaaggtat tttacagact
gtaaaagttg ttgatgaatt ggtcaaagta atggggcggc ataagccaga aaatatcgtt
attgaaatgg cacgtgaaaa tcagacaact caaaagggcc agaaaaattc gcgagagcgt
atgaaacgaa tcgaagaagg tatcaaagaa ttaggaagtc agattcttaa agagcatcct
gttgaaaata ctcaattgca aaatgaaaag ctctatctct attatctcca aaatggaaga
gacatgtatg tggaccaaga attagatatt aatcgtttaa gtgattatga tgtcgatcac
attgttccac aaagtttcct taaagacgat tcaatagaca ataaggtctt aacgcgttct
gataaaaatc gtggtaaatc ggataacgtt ccaagtgaag aagtagtcaa aaagatgaaa
aactattgga gacaacttct aaacgccaag ttaatcactc aacgtaagtt tgataattta
acgaaagctg aacgtggagg tttgagtgaa cttgataaag ctggttttat caaacgccaa
ttggttgaaa ctcgccaaat cactaagcat gtggcacaaa ttttggatag tcgcatgaat
actaaatacg atgaaaatga taaacttatt cgagaggtta aagtgattac cttaaaatct
aaattagttt ctgacttccg aaaagatttc caattctata aagtacgtga gattaacaat
taccatcatg cccatgatgc gtatctaaat gccgtcgttg gaactgcttt gattaagaaa
tatccaaaac ttgaatcgga gtttgtctat ggtgattata aagtttatga tgttcgtaaa
atgattgcta agtctgagca agaaataggc aaagcaaccg caaaatattt cttttactct
aatatcatga acttcttcaa aacagaaatt acacttgcaa atggagagat tcgcaaacgc
cctctaatcg aaactaatgg ggaaactgga gaaattgtct gggataaagg gcgagatttt
 
gccacagtgc gcaaagtatt gtccatgccc caagtcaata ttgtcaagaa aacagaagta

cagacaggcg gattctccaa ggagtcaatt ttaccaaaaa gaaattcgga caagcttatt

gctcgtaaaa aagactggga tccaaaaaaa tatggtggtt ttgatagtcc aacggtagct

tattcagtcc tagtggttgc taaggtggaa aaagggaaat cgaagaagtt aaaatccgtt

aaagagttac tagggatcac aattatggaa agaagttcct ttgaaaaaaa tccgattgac

tttttagaag ctaaaggata taaggaagtt aaaaaagact taatcattaa actacctaaa

tatagtcttt ttgagttaga aaacggtcgt aaacggatgc tggctagtgc cggagaatta

caaaaaggaa atgagctggc tctgccaagc aaatatgtga attttttata tttagctagt

cattatgaaa agttgaaggg tagtccagaa gataacgaac aaaaacaatt gtttgtggag

cagcataagc attatttaga tgagattatt gagcaaatca gtgaattttc taagcgtgtt

attttagcag atgccaattt agataaagtt cttagtgcat ataacaaaca tagagacaaa

ccaatacgtg aacaagcaga aaatattatt catttattta cgttgacgaa tcttggagct

cccgctgctt ttaaatattt tgatacaaca attgatcgta aacgatatac gtctacaaaa

gaagttttag atgccactct tatccatcaa tccatcactg gtctttatga aacacgcatt

gatttgagtc agctaggagg tgactaa
 
SEQ ID_NO 25 (DNA, Pyruvate Decarboxylase_sgRNA_FW – BsaI primer, 36 bp)  gaaatcaatg caggtgaact
gaagaaagcc gctccg
 
SEQ ID_NO 26 (DNA, Pyruvate Decarboxylase_sgRNA_RV - BsaI  primer, 36 bp):gaaccggagc ggctttcttc
agttcacctg cattga
 
SEQ ID_NO 27 (DNA, Pyruvate Decarboxylase_HDR_FW_1 – SalI primer, 27 bp):cgtcgacgtt ggttatgtcc
tataatc
 
SEQ ID_NO 28 (DNA, Pyruvate Decarboxylase_HDR_RV_1 - XhoI/SacI  primer, 30 bp):ctcgaggagc
tctgcttact ccatatattc
 
SEQ ID_NO 29 (DNA, Pyruvate Decarboxylase_HDR_FW_2 -SacI/XhoI primer, 31 bp):gagctcctcg agtttttaaa
taaacttaga g
SEQ ID_NO 30 (DNA, Pyruvate Decarboxylase_HDR_RV_2 - KpNI, 23 bp):tggtacctta aggcactgac ttc 
SEQ ID_NO 31 (DNA, Lactatedehydrogenase_sgRNA_FW – BsaI primer, 36 bp): gaaagaaaca attttaagtc
gtgttgaaaa aggcga
 
SEQ ID_NO 32 (DNA, Lactatedehydrogenase_sgRNA_RV – BsaI primer, 36 bp): gaactcgcct ttttcaacac
gacttaaaat tgtttc
 
SEQ ID_NO 33 (DNA, Lactatedehydrogenase_HDR_FW_1 – SalI primer, 27 bp):cgtcgacgaa agatagctat
tattatc
 
SEQ ID_NO 34 (DNA, Lactatedehydrogenase_HDR_RV_1 - XhoI/SacI primer, 32 bp) ctcgaggagc tctatatcga
ccttttattt tc
 
SEQ ID_NO 35 (DNA, Lactatedehydrogenase_HDR_FW_2 - SacI/XhoI primer, 29 bp) gagctcctcg agttaagctg
ctataaata
SEQ ID_NO 36 (DNA, Lactatedehydrogenase_HDR_RV_2 - KpNI primer, 24 bp) tggtacctct gtcgccagat cgac 
SEQ ID_NO 37 (DNA, Phosphoenolpyruvatecarboxylase_sgRNA_FW – BsaI primer, 36 bp): gaaaatgaaa
tttttggccc tgaatccatt acgact
 
SEQ ID_NO 38 (DNA, Phosphoenolpyruvatecarboxylase_sgRNA_RV - BsaI primer, 36 bp):gaacagtcgt
aatggattca gggccaaaaa tttcat
 
SEQ ID_NO 39 (DNA, Phosphoenolpyruvatecarboxylase_HDR_FW_1 – SalI primer, 22 bp):cgtcgacttt
atcgggaagc tg
 
SEQ ID_NO 40 (DNA, Phosphoenolpyruvatecarboxylase_HDR_RV_1 - XhoI/SacI primer, 28 bp)ctcgaggagc
tcaaaccatc cctttgcc
 
SEQ ID_NO 41 (DNA, Phosphoenolpyruvatecarboxylase_HDR_FW_2 - SacI/XhoI primer, 33 bp):gagctcctcg
agtacatcag ccctatttta aag
 
SEQ ID_NO 42 (DNA, Phosphoenolpyruvatecarboxylase_HDR_RV_2 – KpNI primer, 24 bp)tggtaccccg
ccttgggctg tctg
SEQ ID_NO 43 (DNA, KnockIN_G1_FW-Sacl primer, 33 bp):gtcatgagct catggcgctg gtttctatgc gtc
SEQ ID_NO 44 (DNA, KnockIN_G1_RV- Xhol primer, 32 bp): cagtactcga gttatttacg cggggtgtag tc
SEQ ID_NO 45 (DNA, KnockIN_G2_FW-Sac primer, 33 bp):gtcatgagct catgaaatct gttaccatcc tgg
SEQ ID_NO 46 (DNA, KnockIN_G2_RV-Xhol primer, 31 bp):cagtactcga gttacgcaga acggttcgcc g
SEQ ID_NO 47 (DNA, KnockIN_G3_FW- Sacl primer, 33 bp):gtcatgagct catgacccgt gaagttgttg ttg
SEQ ID_NO 48 (DNA, KnockIN_G3_RV-Xhol primer, 34 bp): cagtactcga gttagtcttc cagcgcgtcg aacg
SEQ ID_NO 49 (DNA, SequencingPrimer_ ORF_FW, 25 bp):cttcctaagc ctttacctga tttag
SEQ ID_NO 50 (DNA, SequencingPrimer_ ORF_RV, 20 bp):cgactgagcc tttcgtttta
SEQ ID_NO 51 (DNA, SequencingPrimer_ HDR_FW, 24 bp): tcatgaccaa aatcccttaa cgtg
SEQ ID_NO 52 (DNA, SequencingPrimer_HDR _RV, 24 bp):ccatgttttg agaagccgat aatc

## Claims

1. Modified non-naturally occurring strain of the bacterial species *Zymomonas mobilis*, **characterized in that** the strain
a) Is modified to contain the genes coding for the enzymes *aldolase* and *triose-phosphate isomerase,*
b) has an inactivation of the gene coding for the enzyme *pyruvate decarboxylase* (preferably *pdc),*
c) has an inactivation of the gene coding for the enzyme *lactate dehydrogenase* (preferably *ddh*)*,*
d) has an inactivation of the gene coding for the enzyme *phosphoenolpyruvate carboxylase* (preferably *ppc),* **and**
e) contains the gene coding for the enzyme *glucokinase* and has an increased activity of the enzyme *glucokinase* compared to a non-modified strain.

2. Modified non-naturally occurring strain according to **claim 1, characterized in that** the inactivation of the genes is induced by genetic engineering or generated by selection, the inactivation preferably being a permanent inactivation.

3. Modified non-naturally occurring strain according to **claim 1 or 2, characterized in that** the strain is transformed with the genes coding for the enzymes *aldolase, triose-phosphate isomerase* and/or *hexokinase* (preferably *glucokinase).*

4. Modified non-naturally occurring strain according to one of the **claims 1 to 3, characterized in that** the modified strain, in comparison with a non-modified strain, has an overexpression of the gene coding for the enzyme *hexokinase* (preferably *glucokinase),* preferably mediated by transformation with the gene and/or modification of an expression regulation sequence of the gene.

5. Modified non-naturally occurring strain according to one of the **claims 1 to 4, characterized in that** the strain contains the genes coding for the enzymes
a) *Deoxy-xylulose-phosphate synthase*
*b) 1-deoxy-D-xylulose-5-phosphate reductoisomerase*
*c) Cytidine diphosphate methylerythritolsynthase*
*d) Cytidyl methyl kinase*
*e) Methyl-erythritol cyclo-diphosphate synthase*
*f) Hydroxy-methyl-butenyl diphosphate synthase*
*g) IPP*/*DMAPP Synthase*
*h) Isopentenyl-diphosphate isomerase* **and**
*i) Geranyl pyrophosphate synthase.*

6. Modified non-naturally occurring strain according to one of the **claims 1 to 5, characterized in that** the strain is transformed with the genes coding for the enzymes
a) *Deoxy-xylulose-phosphate synthase*
*b) 1-deoxy-D-xylulose-5-phosphate reductoisomerase*
*c) Cytidine diphosphate methylerythritolsynthase*
*d) Cytidyl methyl kinase*
*e) Methyl-erythritol cyclo-diphosphate synthase*
*f) Hydroxy-methyl-butenyl diphosphate synthase*
*g) IPP*/*DMAPP Synthase*
*h) Isopentenyl-diphosphate isomerase* **and/or**
i) *Geranyl pyrophosphate synthase.*

7. Modified non-naturally occurring strain according to one of the **claims 1 to 6, characterized in that** the strain contains the genes coding for the enzymes
*a) Acetyl-CoA carboxylase*
*b) Acetyl-CoA acetyltransferase*
*c) FadB Hydroxyacyl-CoA Dehydrogenase*
*d) FadB Enoyl-CoA Hydratase*
*e) Trans-2-enoyl-CoA reductase*
*f) Olivetolsynthase* **and**
*g) Olivetolic acid cyclase.*

8. Modified non-naturally occurring strain according to one of the **claims 1 to 7, characterized in that** the strain is transformed with the genes coding for the enzymes
*a) Acetyl-CoA carboxylase*
*b) Acetyl-CoA acetyltransferase*
*c) FadB Hydroxyacyl-CoA Dehydrogenase*
*d) FadB Enoyl-CoA Hydratase*
*e) Trans-2-enoyl-CoA reductase*
*f) Olivetolsynthase* **and/or**
**g)** *Olivetolic acid cyclase.*

9. Modified non-naturally occurring strain according to one of the **claims 1 to 8, characterized in that** the strain contains the gene coding for the enzyme *geranyl-pyrophosphate-olivetolic acid geranyltransferase,* the strain preferably being transformed with this gene.

10. Modified non-naturally occurring strain according to one of the **claims 1 to 9, characterized in that** the strain contains the gene coding for the enzyme *cannabidiolic acid synthase,* the strain preferably being transformed with this gene.

11. Modified non-naturally occurring strain according to one of the **claims 1 to 10, characterized in that** the strain contains the gene coding for the enzyme *tetrahydrocannabinolic acid synthase,* the strain preferably being transformed with this gene.

12. Modified non-naturally occurring strain according to one of the **claims 1 to 11, characterized in that** the strain is transformed with the genes coding for one or more of the enzymes by means of a genetic vector system or several genetic vector systems.

13. Modified non-naturally occurring strain according to one of the **claims 1 to 12, characterized in that** the strain is a genetically engineered strain.

14. Modified non-naturally occurring strain according to one of the **claims 1 to 13, characterized in that** the strain is a strain for the biosynthesis of cannabinoids, cannabinoid acids or precursors thereof from D-glucose, the biosynthesis preferably being *de-novo* biosynthesis.

15. Method for the production of cannabinoids, cannabinoid acids or precursors thereof from D-glucose, which comprises the cultivation of a modified strain of the bacterial species *Zymomonas mobilis* according to one of the claims 1 to 14 as well as obtaining the cannabinoids, cannabinoid acids or precursors thereof from the bacterial culture.

16. Method according to claim 15 for the production of the cannabinoid cannabidiol, which comprises the cultivation of a modified strain according to claim 10 as well as obtaining the cannabinoid from the bacterial culture.

17. Method according to claim 15 for the production of the cannabinoid tetrahydrocannabinol, which comprises the cultivation of a modified strain according to claim 11 as well as obtaining the cannabinoid from the bacterial culture.

18. Method according to claim 16 or 17, wherein obtaining the cannabinoid comprises the disruption of the cultivated bacterium, the purification of an acid of the cannabinoid as well as the decarboxylation of the acid.


SEQUENCE LISTING

<110> Synbionik Gmbh

<120> Production of plant-based active substances (e.g. cannabinoids) by
recombinant microorganisms

<130> PCT

<160> 52

<170> BiSSAP 1.3.6

<210> 1
<211> 4377
<212> DNA
<213> plasmids


<220>
<223> pSUZM2 (P1)

<400> 1
cggaaaggtc atcctgaaaa ttctattcgt attctgatca accagatcgt gaatgatcgg 60

gcgatgagcc ggaaacttgt cccgtgatca gaaccggcgc agcctcttcc cttttatcta 120

tttcaaattc aggcctgcca cagcggagaa tcttcgctgt ggcttttttg cgtccaagac 180

cggagcccgc acaatgaccg cctcttcctc ttcagcgccc ctctcggatg cctctgtctt 240

ttctttagag cctgtctctg cgtcttttga acaaacgacg cccgtaacca gtccttttat 300

cgacaggcta ggccgtttca agcgtggcac ttatcgaacc atgttggttg aaacgggtat 360

gccatttgac agcgcaaaga ttatggcaga tcaggccgca gaacaacata aaagagagcg 420

cacattctct aaattaatgc ccttggccaa tggcgctcat cccgaaattt cggcctcaaa 480

taccaatatt gaggctacag cctctgcatc agatttcgac gaaatcgaat attttgacca 540

gatggtatca aacggcgttg atcctagaga tgccgcagat attgctcgca aaacagctat 600

tgcgagacgt cgagaccctc tcgaacgagc cattcgctat aaaacccgcc gtcaaactcc 660

tgatcctgtg catcgggatt cttatgaggt gggcgaacgc gagaagtcgg tctggaagcc 720

ggtcaatccg caagagattg gtgcctatct ggaagcggtt gatcaatatt cgatcaaaac 780

caagggctta agcgataagg ccgttcgtct tctaaagatg ctgtttcgga tggtcgactt 840

taaaactggc cgtctggagc cgaccctcga cacaatatgc gatcgtgtcg gttatgcccg 900

tgcgacggtc gtcaaattac tgcggcaatt acaggattta ggcttcatcc gctggatacg 960

ccggtcaatc aagatcaagg cggacggcaa aggcccccgc cgcaagcaga ccagtaacgc 1020

ctacggcttc ttgtctccca aggcttggcc agatttggcc agacaggttt tcgagcgtgt 1080

gatgcgccgg aggcatgcgc ctattcctga cgatatcgac cacgcccaag aggcggacaa 1140

agccgaaacc aaggctatcg ttgaaaactt gccgcccgaa gatttcgtca aagcaatatc 1200

gggcgataaa gagccaacag agttaacaga ttcgttaata cagctcgcca aatctatcga 1260

gcgggcggaa caacgggaac aagaagaaca ggctaagaag gcagcacagg ccggtcagga 1320

agaggttaaa acatcttccc aaacacctaa ggaggatacc tcaaaaaagc ccccaatctc 1380

gggcaatcaa cgcgagttca attcttatac tctatcctgt tatacttatt tatctaatag 1440

cgaccataca aaactaaaaa ccaaagaaga agataaagac tgcactgctc ctgctaccgc 1500

aggcagtgca gaccctattt atgatcgaaa acctgacagc aatcctttcc tctcaactct 1560

caaagctcgc ggttatcgtc ctgatcaaat caacgcttgc ctcgaaaaca gagataaacg 1620

gaaaaagaaa ggatggatgc ctctgtcacc cgatgaaacc gcccgcctcc ttattcaaac 1680

aaaacagatg tgcccataac aacgaccact atccttctca aagcatatcg tccgttaatc 1740

tctcggcgct gctcgcttcg ctcacaggct cggcgagaaa gaaatttgcc tttcggggga 1800

ctatctaatt cctaacaatt cgcattatcg ataatgtaat aaaaggctaa tctttaaaaa 1860

ccaaattaca gcgattacat catttctatg aatgtgtatt atacataata tgagaagtcg 1920

tgatttgatc aaagaagtta aaaaagccaa tattaccagc ttgacagata taaaataggc 1980

cttggttgat ttcgtgaaag aactggaaga ttgcacaatt cttctttctg gtgatatctt 2040

tgacccgacg gccctcagtc aatgagtgtg cgccgcattt ctgcccattc aatcagggcc 2100

tgcattgaag ggccgagagc gtatcccata ccgctgagct cgtattctac ctttggcggg 2160

acctgcggat agaccgttcg gagcacgatg cgatcctttt ccagttgctt gagctgctgg 2220

atcagcatct tctggttgat gccgtcgatc tgcttttcca gtgaggagaa gcggattggg 2280

ccttttgcgg caaacaactg gcagatgata acaatcttcc attttccttc gagtaccctg 2340

agcgcttctg tcgtcgcctc aggaattcga gctcggtacc cggggatcct ctagagtcga 2400

cctgcaggca tgcaagcttg gatccataaa acgaaaggct cagtcgaaag actgggcctt 2460

tcgttttatc tgttgtttgt cggtgaacgc tctcctgagt aggacaaatc cgccgggagc 2520

ggatttgaac gttgcgaagc aacggcccgg agggtggcgg gcaggacgcc cgccataaac 2580

tgccaggcat caaattaagc agaaggccat cctgacggat ggcctttgta gaaaagatca 2640

aaggatcttc ttgagatcct ttttttctgc gcgtaatctg ctgcttgcaa acaaaaaaac 2700

caccgctacc agcggtggtt tgtttgccgg atcaagagct accaactctt tttccgaagg 2760

taactggctt cagcagagcg cagataccaa atactgtcct tctagtgtag ccgtagttag 2820

gccaccactt caagaactct gtagcaccgc ctacatacct cgctctgcta atcctgttac 2880

cagtggctgc tgccagtggc gataagtcgt gtcttaccgg gttggactca agacgatagt 2940

taccggataa ggcgcagcgg tcgggctgaa cggggggttc gtgcacacag cccagcttgg 3000

agcgaacgac ctacaccgaa ctgagatacc tacagcgtga gctatgagaa agcgccacgc 3060

ttcccgaagg gagaaaggcg gacaggtatc cggtaagcgg cagggtcgga acaggagagc 3120

gcacgaggga gcttccaggg ggaaacgcct ggtatcttta tagtcctgtc gggtttcgcc 3180

acctctgact tgagcgtcga tttttgtgat gctcgtcagg ggggcggagc ctatggaaaa 3240

acgccagcaa cgcggccttt ttacggttcc tggccttttg ctggcctttt gctcacatat 3300

gttcaaatat gtatccgctc atgagacaat aaccctgata aatgcttcaa taatattgaa 3360

aaaggaagag tatgagtatt caacatttcc gtgtcgccct tattcccttt tttgcggcat 3420

tttgccttcc tgtttttgct cacccagaaa cgctggtgaa agtaaaagat gctgaagatc 3480

agttgggtgc acgagtgggt tacatcgaac tggatctcaa cagcggtaag atccttgaga 3540

gttttcgccc cgaagaacgt tttccaatga tgagcacttt taaagttctg ctatgtggcg 3600

cggtattatc ccgtattgac gccgggcaag agcaactcgg tcgccgcata cactattctc 3660

agaatgactt ggttgagtac tcaccagtca cagaaaagca tcttacggat ggcatgacag 3720

taagagaatt atgcagtgct gccataacca tgagtgataa cactgcggcc aacttacttc 3780

tgacaacgat cggaggaccg aaggagctaa ccgctttttt gcacaacatg ggggatcatg 3840

taactcgcct tgatcgttgg gaaccggagc tgaatgaagc cataccaaac gacgagcgtg 3900

acaccacgat gcctgtagca atggcaacaa cgttgcgcaa actattaact ggcgaactac 3960

ttactctagc ttcccggcaa caattaatag actggatgga ggcggataaa gttgcaggac 4020

cacttctgcg ctcggccctt ccggctggct ggtttattgc tgataaatct ggagccggtg 4080

agcgtggttc tcgcggtatc attgcagcac tggggccaga tggtaagccc tcccgtatcg 4140

tagttatcta cacgacgggg agtcaggcaa ctatggatga acgaaataga cagatcgctg 4200

agataggtgc ctcactgatt aagcattggt aactgtcaga ccaagtttac tcatatatac 4260

tttagattga tttaaaactt catttttaat ttaaaaggat ctaggtgaag atcctttttg 4320

ataatctcat gaccaaaatc ccttaacgtg agttttcgtt ccactgagcg cggccgc 4377

<210> 2
<211> 4753
<212> DNA
<213> plasmids

<220>
<223> pSUZM1 (P2)

<400> 2
gcaccgcaaa ggacatgctg atcgcgagat cggcgcgcta ctcgacactg tcataacaga 60

tcacattcgg agatagctga tgaatactgc gcagaatcag actcttaaaa gagcgttttt 120

gtcatcagaa gccgcagcag ctgactcttc agctgactgc ggcttttcta tgtctgaaaa 180

agatatgcca gattttgaag aaatgacgcc agccagccct tttatcgaca ggctaggccg 240

tttcaagcgc ggcacttatc gaagcatgtt gatgcagacg gggctttctt atgaacgcgc 300

gaaaggcatg gcagacaatg cggcggaaga atttaaaaaa tcaaaagcaa agtcgaaatc 360

tttgccattg gccaagagcg aaatcgctga acttggggct tcagaagcaa cgattgaagt 420

aacagctcat caggtaacat tttctgaatc acaggcacaa ccagaacctt tcgatgctta 480

ctgctgcttg caacagttaa aagatcaggg cgtggacggt gccacagctg cgaagattgc 540

aaatcaggcc tacaagcatc acaaagaagc cgaaaatttt gacgaagcgg tatatttcaa 600

ggaattagtc gatactggca tagatgcggt gatggctgct aatatggctc gtaaaacagc 660

cgaagctaag cgccgcgacc cgctggaaag atacggtatc cgtttaaagc cgcgccgcac 720

gcctgatcct gttcaccgtg actcttatga tgttggcgag cgcgaaaata cggtctggaa 780

gccggtcaat ccgaatgaga tcggggctta tctggaagca gtcgatcaat attcaatcaa 840

gaccggcaat atcagtgata gagccgtccg gctgctcaag atgctgtttc ggatggtgga 900

cttcaagaca ggtcgattag agccgacgct tgatacaatc tgcgaatggg tcggctatgc 960

acgggcaacg gttgtccgct tgctaaaaca gcttcaagag caaggtttta tccgctggat 1020

acgccgttca atcaagatca agacggatgg ggctgggccc cgccgcaaac agaccagtaa 1080

cgcttacggt ttcttgtcgc ctaaatcttg gccggagatg gcaagaaagg ttttcgagcg 1140

cgtgatgcgc cggaagaatg cgccggtctc tgctgatctt gctcatgctc aagaagccga 1200

caaagtcgaa accggtgcca tgattgaaag ccagccgaat gacgactggg tgaaagaaat 1260

cttcgcacag aagcgcgcgc aacatccgac gagtgacgag cagaaaaaca aaaaagaaca 1320

ggccgatgat ctggcagaga ctcttgccag cttcgccaaa tctatcgagc gggcagaaca 1380

acgggaacaa gaagaacagg ccaagaaagc catttcggcc gagcaaaatg aggttaaaac 1440

accctacaaa acactcaagg aggacacctc aaaaacgccc caaatttcgg cagttgagcg 1500

cgagttcaat tttgatactc tatcccgtta tagttatttt aataataacc actccaatca 1560

atttaaaaac caaaaagaga ttaaggactg cactgctcct gctgccgcag gcagtgcaga 1620

cgttgttttg aaaacaaaac ctcaaaccga cactttccta aaaacccttc aaactcgcgg 1680

ttaccgtcaa gaccagacca acgcttgcct cgaaaacaga gataaacgga aaaagaatgg 1740

atggatgcct ctgtcagcgg acgaaaccgc ccgcctcctt atacaaacaa ggcagatgcg 1800

accgtgatac cgacagctat ccttctcaaa gcacaccgcc cattaatctc tcggcgctgc 1860

tcgcttcgct cacaggctcg gcgaaaaaga aatttgcctt ttggggggct attaacttct 1920

aacaactcgc attatcgaaa tgtctttaaa taggtttatt ctctaaaagc gtttcgacat 1980

ggcttttact gttatccatc atccaatctt cagtcggtct tgagcgatat ccaatcaaac 2040

gaacaattct atcccagtca gcttgtagat ctctttcaaa tataggctca tgatgggcta 2100

tccgattacg cagcatccga atttcaccca aatctctata gagactctgt ctctggattt 2160

taatctcttg ctgtggatca agattcggga acacgttcaa caaatagaga tcccaaagcc 2220

gtttatcatg gcgctttgtg aacatagcct gccagaatac aaaattcagt tctggtatta 2280

cctttccggt cgtttccgcc ttcaaacagg ctttgtttac atccttcgat gcgctgtatc 2340

ctgatttgac aactggaagg cttcgtcgga aaccttctga ccacggccac tgatctccat 2400

aggtatagga tagcgcttcc gccacagcat ttcgggtcgc tatttctgtt atatgaaggg 2460

gggctagcaa tgcgccggat agctgggcat tccacacata taaatccaga gcgacgagat 2520

catcttttcc caatgccctg actgtgtctt catatcttcc aagtcgggga gccgataagg 2580

tctttctgat agaggtaatc ttgtcttgaa ttgacagcat ggctacccat ttatatgatc 2640

aaccacatat tcgcctcggg tctttgttgg cttatgcctc cccctgagga caaaagatac 2700

aaaaaagacc tcgcttacgg cggggtcttt ttttatccat tttcctgtat tgtataaagt 2760

ctttgctagc ctgacggaaa acgtaatcct gcggttaatc ggaattcgag ctcggtaccc 2820

ggggatcctc tagagtcgac ctgcaggcat gcaagcttgg atccataaaa cgaaaggctc 2880

agtcgaaaga ctgggccttt cgttttatct gttgtttgtc ggtgaacgct ctcctgagta 2940

ggacaaatcc gccgggagcg gatttgaacg ttgcgaagca acggcccgga gggtggcggg 3000

caggacgccc gccataaact gccaggcatc aaattaagca gaaggccatc ctgacggatg 3060

gcctttgtag aaaagatcaa aggatcttct tgagatcctt tttttctgcg cgtaatctgc 3120

tgcttgcaaa caaaaaaacc accgctacca gcggtggttt gtttgccgga tcaagagcta 3180

ccaactcttt ttccgaaggt aactggcttc agcagagcgc agataccaaa tactgtcctt 3240

ctagtgtagc cgtagttagg ccaccacttc aagaactctg tagcaccgcc tacatacctc 3300

gctctgctaa tcctgttacc agtggctgct gccagtggcg ataagtcgtg tcttaccggg 3360

ttggactcaa gacgatagtt accggataag gcgcagcggt cgggctgaac ggggggttcg 3420

tgcacacagc ccagcttgga gcgaacgacc tacaccgaac tgagatacct acagcgtgag 3480

ctatgagaaa gcgccacgct tcccgaaggg agaaaggcgg acaggtatcc ggtaagcggc 3540

agggtcggaa caggagagcg cacgagggag cttccagggg gaaacgcctg gtatctttat 3600

agtcctgtcg ggtttcgcca cctctgactt gagcgtcgat ttttgtgatg ctcgtcaggg 3660

gggcggagcc tatggaaaaa cgccagcaac gcggcctttt tacggttcct ggccttttgc 3720

tggccttttg ctcacatatg ttcaaatatg tatccgctca tgagacaata accctgataa 3780

atgcttcaat aatattgaaa aaggaagagt atgattgaac aggatggcct gcatgcgggt 3840

agcccggcag cgtgggtgga acgtctgttt ggctatgatt gggcgcagca gaccattggc 3900

tgctctgatg cggcggtgtt tcgtctgagc gcgcagggtc gtccggtgct gtttgtgaaa 3960

accgatctga gcggtgcgct gaacgagctg caggatgaag cggcgcgtct gagctggctg 4020

gccaccaccg gtgttccgtg tgcggcggtg ctggatgtgg tgaccgaagc gggccgtgat 4080

tggctgctgc tgggcgaagt gccgggtcag gatctgctgt ctagccatct ggcgccggca 4140

gaaaaagtga gcattatggc ggatgccatg cgtcgtctgc ataccctgga cccggcgacc 4200

tgtccgtttg atcatcaggc gaaacatcgt attgaacgtg cgcgtacccg tatggaagcg 4260

ggcctggtgg atcaggatga tctggatgaa gaacatcagg gcctggcacc ggcagagctg 4320

tttgcgcgtc tgaaagcgag catgccggat ggcgaagatc tggtggtgac ccatggtgat 4380

gcgtgcctgc cgaacattat ggtggaaaat ggccgtttta gcggctttat tgattgcggc 4440

cgtctgggcg tggcggatcg ttatcaggat attgcgctgg ccacccgtga tattgcggaa 4500

gaactgggcg gcgaatgggc ggatcgtttt ctggtgctgt atggcattgc ggcaccggat 4560

agccagcgta ttgcgtttta tcgtctgctg gatgaatttt tctaataact gtcagaccaa 4620

gtttactcat atatacttta gattgattta aaacttcatt tttaatttaa aaggatctag 4680

gtgaagatcc tttttgataa tctcatgacc aaaatccctt aacgtgagtt ttcgttccac 4740

tgagcgcggc cgc 4753

<210> 3
<211> 4515
<212> DNA
<213> plasmids

<220>
<223> pUCZM-7 (P3)

<400> 3
gtcgacggta ccatgattat cggcttctca aaacatggac agggcaaagc tgctccagcc 60

ataaaatacc tgctcgatca gcacagagag ggcagagaaa atcatattcc tgaagtggtg 120

agaggtaatc cagacttaac cgcatccatt atcgacagcc tgactttcaa acatcactat 180

actagcggag ttctttcttt cgctgccgaa gatcatgttt ctgccgagca ggaaaaagat 240

attatcaacg atttcgaaaa gctggctttt gctggcctgc aagcagacca gtttaatatc 300

ctttgggtgc gccattctca tgcgggtcat catgaacttc atttcatcac tccaaggata 360

gaactttcat ccaagaagtc cttcaacatc aagccccccg gtaagaatac ccaaaaggaa 420

tttgatgact tgcggagtgc catcaatgcc aaatatggct ttgctgaccc agaagacccg 480

atgagagcga gaaacgtcaa aacacctagc tatgaactaa aaatcgctag gaatgtcctt 540

agagagcaaa aagcgccctc tgaggacata agacagctta ttgatagcct tttaactgaa 600

agagccgtcc aaggcctgat acatgacaga aaaactcttc tcactaatgt tgaagagtta 660

ggcttcgaag ttactcgcgc aggcaagaat tatattaccg tcaaagaccc tgaaagctca 720

aaacgatttc gtctcaaagg ccaactctat gaacaagact ttacctctag ccgaacgatt 780

gaaacagcag cagctaaaag aacaagagat tattctcgac ctgactcaac agcagctaga 840

caatttgagg aacaagttaa actccacatt cgaaaacgag ctgaattcaa tagaaaacga 900

tatcgagcga ctgaaccgga gcttaggatg gaaattcctc ctatctcgct ggctgatacc 960

agtcgtgacc agcatcatga tattgtttgg cctgctcttg ggcgcagaag ggataatcaa 1020

gctctatacc caccagatca gagctttgca ggaggaaaaa gcagagatgg aagtgaccgt 1080

cgaacagctc aacaaagcag gtgggaagat gcaattcagc cattgcggag aaaaagaaag 1140

gctttgcatc agagtaaaca cacgagagat cttcgggata accagatatt atctgatcgt 1200

gaaagaacgg aaatagacaa tgacggaatt ggaacaggtt ttactgaacg ccttggaaaa 1260

tttacagcag agattcgatc aacaacagaa aagtttatta cacgcgcaaa gcgaattacg 1320

gcagatgttc gaaactacct caaccgagaa tacttattta cgacagcaag tagaaatctt 1380

ggtcgagcga ttgaacagtt ttcccagatg aaaaccttaa gtcgaaagct acaaaaacgg 1440

agaggtcgtt ctcgctctcg ataaacttga aaaggacgct aattccgaaa agttttttat 1500

ctttctgata gtcgatttta gttggcgatt tccgataaaa aagccctgaa cttattgaac 1560

tttcctatat ttcaataagt tcaataagtt caggcttttt ttattcaaaa aaatcaatgc 1620

cttacgactt taaaaaccac ttttggggag tgaaaaacca cttttgggga gtgaaaaacc 1680

acttttgggg agtgaaaaac cacttcaaaa aatatattaa gtggttaata aagttgtaat 1740

gagaaaaaca ctacaaataa ttaaagatag agccggcaat aaagatcaaa atttacatgc 1800

aggtgaaatt atagatggac atttttcaga ccttaaaaat ccgcctagcc tagctgcact 1860

aaaactctat gagttgctta ttaaatacgc agcatataat atagataaaa atatatggca 1920

taatatttct gtatcagaaa tgcaaaaaat caaaggaata aagaactata actcaacaga 1980

gctaactcaa ttattaaaag aacttagagc tgttgttatg gaatatagag caaaagatag 2040

aactattatt actggtcttt tagatattgc tgcggtcata tctaacccag acacaggata 2100

tccagagata caatggaaat ttggagaaag ttttagagat ttaatagcta agtcagaata 2160

ttgggcaatt gttgattata gaacaacgtt agagatgacg agccgatatt cccttcgttt 2220

atatgaaata ttagctctta gaataaattt aaaacacaaa aattcagata catttacttt 2280

agccgagctt agaggaaaac tgggagttcc tgaaggaaaa ttaaaaagat ggtctgattt 2340

aagagataaa gcgatcaatg ttgcggtaaa agaaattaac caacggacta ctttcgccgt 2400

aacagcacaa ccaaaaaaaa tgggaagatc cgttcaagcc gtagaattta aatgggaacg 2460

aaaagagatt aaacttcctc aaagtatacc attaaaaaca tctaaaagca ttgtttttcc 2520

attagatgga agtattagat atactcaatg ggaagatatt gcacgaaaaa atcttcctaa 2580

gcctttacct gatttagata tggtagcaca acaatttaga aaatggttaa aaaataaaaa 2640

catttcatca aacagtatta atgttttgtc catttttaag aaattttgtg aaaaatacag 2700

aatagaattt aataattaag aattcagtac tcccggggga tcctctagac tgcagaagct 2760

tataaaacga aaggctcagt cgaaagactg ggcctttcgt tttatctgtt gtttgtcggt 2820

gaacgctctc ctgagtagga caaatccgcc gggagcggat ttgaacgttg cgaagcaacg 2880

gcccggaggg tggcgggcag gacgcccgcc ataaactgcc aggcatcaaa ttaagcagaa 2940

ggccatcctg acggatggcc tttgtagaaa agatcaaagg atcttcttga gatccttttt 3000

ttctgcgcgt aatctgctgc ttgcaaacaa aaaaaccacc gctaccagcg gtggtttgtt 3060

tgccggatca agagctacca actctttttc cgaaggtaac tggcttcagc agagcgcaga 3120

taccaaatac tgtccttcta gtgtagccgt agttaggcca ccacttcaag aactctgtag 3180

caccgcctac atacctcgct ctgctaatcc tgttaccagt ggctgctgcc agtggcgata 3240

agtcgtgtct taccgggttg gactcaagac gatagttacc ggataaggcg cagcggtcgg 3300

gctgaacggg gggttcgtgc acacagccca gcttggagcg aacgacctac accgaactga 3360

gatacctaca gcgtgagcta tgagaaagcg ccacgcttcc cgaagggaga aaggcggaca 3420

ggtatccggt aagcggcagg gtcggaacag gagagcgcac gagggagctt ccagggggaa 3480

acgcctggta tctttatagt cctgtcgggt ttcgccacct ctgacttgag cgtcgatttt 3540

tgtgatgctc gtcagggggg cggagcctat ggaaaaacgc cagcaacgcg gcctttttac 3600

ggttcctggc cttttgctgg ccttttgctc acatatgttc aaatatgtat ccgctcatga 3660

gacaataacc ctgataaatg cttcaataat attgaaaaag gaagagtatg gaaaaaaaaa 3720

tcaccggcta taccaccgtg gatattagcc agtggcatcg taaagaacat tttgaagcgt 3780

ttcagagcgt ggcgcagtgc acctataacc agaccgtgca gctggatatc accgcgtttc 3840

tgaaaaccgt gaaaaaaaac aaacacaaat tctacccggc gtttattcat attctggccc 3900

gtctgatgaa cgcgcatccg gaatttcgta tggccatgaa agatggcgaa ctggtgattt 3960

gggatagcgt gcatccgtgc tataccgtgt ttcatgaaca gaccgaaacc tttagcagcc 4020

tgtggagcga atatcatgat gattttcgcc agttcctgca tatttatagc caggatgtgg 4080

cgtgctatgg cgaaaacctg gcctattttc cgaaaggctt catcgaaaac atgttctttg 4140

tgagcgcgaa tccgtgggtg agctttacca gcttcgatct gaacgtggcg aacatggata 4200

acttttttgc gccggtgttt accatgggca aatattatac ccagggcgat aaagtgctga 4260

tgccgctggc cattcaggtg catcatgcgg tgtgtgatgg ctttcatgtg ggccgtatgc 4320

tgaacgaact gcagcagtat tgtgatgaat ggcagggcgg tgcgtaataa ctgtcagacc 4380

aagtttactc atatatactt tagattgatt taaaacttca tttttaattt aaaaggatct 4440

aggtgaagat cctttttgat aatctcatga ccaaaatccc ttaacgtgag ttttcgttcc 4500

actgagcgcg gccgc 4515

<210> 4
<211> 8603
<212> DNA
<213> cassette

<220>
<223> Gene cassette (G1)

<400> 4
tctagattca aggtgtcccg ttcctttttc ccctttttgg aggttggtta tgtcctataa 60

tcacttaatc cagaaacggg cgtttagctt tgtccatcat ggttgtttat cgctcatgat 120

cgcggcatgt tctgatattt ttcctctaaa aaagataaaa agtcttttcg cttcggcaga 180

agaggttcat catgaacaaa aattcggcat ttttaaaaat gcctatagct aaatccggaa 240

cgacacttta gaggtttctg ggtcatcctg attcagacat agtgttttga atatatggag 300

taagcaatgg cgctggtttc tatgcgtcag ctgctggacc acgcggcgga aaactcttac 360

ggtctgcctg cgttcaacgt taacaacctg gaacagatgc gtgcgatcat ggaagcggcg 420

gaccaggtta acgcgccggt tatcgttcag gcgtctgcgg gtgcgcgtaa atacgcgggt 480

gcgccgttcc tgcgtcacct gatcctggcg gcggttgaag aatttccgca catcccggtt 540

gttatgcacc aggaccacgg tgcgtctccg gacgtttgcc agcgttctat ccagctgggt 600

ttctcttctg ttatgatgga cggttctctg ctggaagacg gtaaaacccc gtctacctac 660

gaatacaacg ttaacgcgac ccgtaccgtt gttaacttct ctcacgcgtg cggtgtttct 720

gttgaaggtg aaatcggtgt tctgggcaac ctggaaaccg gtgaagcggg tgaagaagac 780

ggtgttggtg cggcgggtaa actgtctcac gaccagatgc tgacctctgt tgaagacgcg 840

gttcgtttcg ttaaagacac cggtgttgac gcgctggcga ttgcggttgg cacctctcac 900

ggtgcgtaca aattcacccg tccgccgacc ggtgacgttc tgcgtatcga ccgtatcaaa 960

gaaatccacc aggcgctgcc gaacacccac atcgttatgc acggttcttc ttctgttccg 1020

caggaatggc tgaaagttat caacgaatac ggtggtaaaa tcggtgaaac ctacggtgtt 1080

ccggttgaag aaatcgttga aggtatcaaa cacggtgttc gtaaagttaa catcgacacc 1140

gacctgcgtc tggcgtctac cggtgcgatc cgtcgtttca tggcggaaaa cccgtctgaa 1200

tttgacccgc gtaaatacct gtctaaaacc gttgaagcga tgaaacagat ttgcctggac 1260

cgttacctgg cgttcggttg cgaaggtcag gcgggtaaaa tcaaaccggt ttctctggaa 1320

aaaatggcga accgttacgc gaaaggtgaa ctgaaccaga tcatcaaata atttaccaac 1380

actactacgt ataaggagag cgttgttatg cgtcgtaaac tggttgcggg taactggaaa 1440

atgaacggtt ctcgtgcgat gctggttgaa ctggacgcga tcgcggcggc ggcgtctgcg 1500

gctccggaaa tcgacgtttc tgttgcggtt ccgttcaccc tgatcgcggc ggcgtctgaa 1560

cgtgttccga ccctggcgat cggtgcggaa gacgttcacg aagcggaatc tggtgcgcac 1620

accggttgcg tttctgcgtc tatggttaaa gaagttggtg cgtctttcac catcgttggt 1680

cactctgaac gtcgttctga ccagcacgaa acctctcacg acgcgtgggc gaaagcggcg 1740

gcggcgcaca aacagggtct gaacgttatc ctgtgctgcg gtgaaaccga agcggaacgt 1800

gacgcggacc gtgcggaacg tgttgttcag gcgcagatcg aaaaatctgt tccggacaac 1860

gcggacggtt cttggttcac cctggcgtac gaaccgcgtt gggcgatcgg caccggtcgt 1920

accccgaccc tggaagaaat cgcggcgatc cacgcgatcg cgcgtgcgaa actgcgtatg 1980

ctgatcggtg acaaagcggc gggtgttcgt atcctgtacg gtggttctgt taccggtgcg 2040

aacgcggcgt ctatcctgga caccccgaac gttgacggtg cgctggttgg tggtgcgtct 2100

ctgaccgcgg cgaaattcgt tccgatcatc gaagcggcgg cgtctctgta atttaccaac 2160

actactacgt ataaggagag cgttgttatg gaaatcgttg cgatcgacat cggtggcacc 2220

cacgcgcgtt tctctatcgc ggaagtttct aacggtcgtg ttctgtctct gggtgaagaa 2280

accaccttca aaaccgcgga acacgcgtct cttcagctgg cgtgggaacg tttcggtgaa 2340

aaactgggtc gtccgctgcc gcgtgcggcg gcgatcgcgt gggcgggtcc ggttcacggt 2400

gaagttctga aactgaccaa caacccgtgg gttctgcgtc cggcgaccct gaacgaaaaa 2460

ctggacatcg acacccacgt tctgatcaac gacttcggtg cggttgcgca cgcggttgcg 2520

cacatggact cttcttacct ggaccacatc tgcggtccgg acgaagcgct gccgtctgac 2580

ggtgttatca ccatcctggg tccgggcacc ggtctgggtg ttgcgcacct gctgcgtacc 2640

gaaggtcgtt acttcgttat cgaaaccgaa ggtggtcaca tcgacttcgc gccgctggac 2700

cgtctggaag acaaaatcct ggcgcgtctg cgtgaacgtt tccgtcgtgt ttctatcgaa 2760

cgtatcatct ctggtccggg tctgggtaac atctacgaag cgctggcggc gatcgaaggt 2820

gttccgttct ctctgctgga cgacatcaaa ctgtggcaga tggcgctgga aggtaaagac 2880

aacctggcgg aagcggcgct ggaccgtttc tgcctgtctc tgggtgcgat cgcgggtgac 2940

ctggcgctgg cgcagggtgc gacctctgtt gttatcggtg gtggtgttgg tctgcgtatc 3000

gcgtctcacc tgccggaatc tggtttccgt cagcgtttcg tttctaaagg tcgtttcgaa 3060

cgtgttatgt ctaaaatccc ggttaaactg atcacctacc cgcagccggg tctgctgggt 3120

gcggcggcgg cgtacgcgaa caaatactct gaagttgaat aatttaccaa cactactacg 3180

tataaggaga gcgttgttat ggcggacctg ccggcgaccc cgctgctgga caccgtttct 3240

accccggacg acctgcgtaa actgcgtccg gaccagctgc gtcagctggc ggacgaactg 3300

cgtaccgaaa ccatctctgc ggttggcacc accggtggtc acctgggttc tggtctgggt 3360

gttgttgaac tgaccaccgc gatccactac gttttcgaca ccccgcgtga ccgtctggtt 3420

tgggacgttg gtcaccagtg ctacccgcac aaaatcctga ccggtcgtcg tgaccgtatc 3480

cgtaccctgc gtcagggtgg tggtctgtct ggtttcacca aacgttctga atctgaatac 3540
gacccgttcg gtgcggcgca ctcttctacc tctatctctg cggcgctggg tttcgcggtt 3600
gcgaacaaaa tcgcgggcac ccctggtaaa ggtatcgcgg ttatcggtga cggtgcgatg 3660
tctgcgggta tggcgtacga agcgatgaac aacgcggcgc aggcgggtaa ccgtctggtt 3720
gttatcctga acgacaacga catgtctatc gcgccgccgg ttggtggtct gtctgcgtac 3780
ctgtctcgta tcgtttcttc tcgtgaattt ctgggtttcc gtgacctggc gcgtaaaatc 3840
gcgaaacgtc tgccgcgtcc gatcgcgtct ggtatgcgta aaaccgacga atttgcgcgt 3900
ggtatgacca tgggtggcac cctgttcgaa gaactgggtt tctactacgt tggtccgatc 3960
gacggtcaca acctggacca cctgatcccg gttctggaaa acgttcgtga cgcggaagaa 4020
ggtccgatcc tggttcacgt tgttaccaaa aaaggtaaag gttacgcgcc ggcggaagcg 4080
gcggcggaca aataccacgg tgttcagaaa ttcgacgtta tctctggtgt tcagaccaaa 4140
gcgccgccgg gtccgccgca gtaccagaac gttttcggtg cgcagctggt tgcggaagcg 4200
gcgaaagacg cgcgtatctg cgcgatcacc gcggcgatgc cgtctggcac cggtctggac 4260
gcgttcgcga aagcgtaccc ggaccgtttc ttcgacgttg gtatcgcgga acagcacggt 4320
gttaccttcg cggcgggtct ggcggcgcag ggtctgcgtc cgttctgcgc gatctactct 4380
accttccttc agcgtgcgta cgaccaggtt gttcacgacg ttgcgatcca gaacctgccg 4440
gttcgtttcg cgatcgaccg tgcgggtctg gttggtgcgg acggtgcgac ccacgcgggt 4500
tctttcgacg ttacctacct ggcgaccctg ccgaacttcg ttgttatggc ggcggcggac 4560
gaagcggaac tggttcacat gacccacacc gcggttcagt acgacgacgg tccgatcgcg 4620
ctgcgttacc cgcgtggtaa cggcaccggt gttgcgctgc cggaaacccc gcagctgctg 4680
gaaatcggta aaggtcgtgt tgttcgtgaa ggtaaaaccg ttgcgatcct gtctctgggc 4740
acccgtctgg aagaagcgct gaaagcggcg gcgatcctgg aagcgaaagg tctgtctacc 4800
accgttgcgg acctgcgttt cgcgaaaccg ctggacgttg acctgatccg taaactgctg 4860
accacccacg aagttgcggt taccatcgaa gaaaactctg ttggtggttt cggtgcgcac 4920
gttctgacca tggcgtctga cgaaggtctg atcgacggtg gtctgaaact gcgtaccctg 4980
cgtctgccgg acatgtacca ggaccaggac aaaccggaag ttcagtacgc ggaagcgggt 5040
ctggacgcga ccgcgctggt tgacaccgtt ctgaaagcgc tgcgtcacaa ctctggtggt 5100
gttgcggaag cgcgtgcgta atttaccaac actactacgt ataaggagag cgttgttatg 5160
gtttcttacc tggacttcga aaaaccggtt gcggaacttc agacccgtat cgcggaactg 5220
cgtaaagcga cctctgacga aatggacctg gaccgtgaaa tctctgacct ggaagttaaa 5280
gttcagaaac tgctgcgtga cacctacgcg cacctgaccc cgtggcagaa aacccaggtt 5340
gcgcgtcacc cggaacgtcc gcacttccac gactacatct ctgcgctgtg cgaagaattt 5400

atcccgctgg cgggtgaccg tctgttcggt gaagacgaag cgatcatcgg tggtctggcg 5460

cgtatcgacg gtcagcgtgt tatgatcatc ggtcaggaaa aaggtaacga caccgcgtct 5520

cgtctgaaac acaacttcgg tatggcgcgt ccggaaggtt accgtaaagc gatccgtctg 5580

atgaaactgg cggaccgttt cggtctgccg gttatcaccc tggttgacac ctctggtgcg 5640

ttccctggta tcgacgcgga agaacgtggt caggcggaag cgatcgcgcg ttctaccgaa 5700

acctgcctgt ctctgggtgt tccgctgatc tctgttatcg ttggtgaagg tggttctggt 5760

ggtgcgatcg cgatcgcggc ggcgaaccgt ctgctgatgt tcgaacacgc ggtttactct 5820

gttatctctc cggaaggttg cgcgtctatc ctgtggcgtg acgcgggtaa agcgtctgac 5880

gcggcgaccg cgatgaaact gaccgcgacc gacctgtacg gtctgggtat cgttgaccgt 5940

atcgttctgg aaccggttgg tggtgcgcac cgtgacccga aaaccgcgat cgacgcgctg 6000

aaaatggcgc tgcttcagga actggcgttc ctgaaaccga tggaccgtga cgcgctgcgt 6060

tctatgcgtc gtaaaaaatt cctggacatc ggtggttaat ttaccaacac tactacgtat 6120

aaggagagcg ttgttatgtc ttggctggaa aaagttcgta acaaaatccc gttcatcgtt 6180

aaacgtgaaa ccccggacaa cctgtggcac cgttgcccgc actgccagac catgatcttc 6240

aacaaagact acgaagaaaa cctgtctgtt tgcccgaaat gcggtttcca cggtcgtatc 6300

ggttctcgtg cgcgtttcga ctctctgttc gacgaaggtg actactctgt tctgccgccg 6360

ccggttgttc cggaagaccc gctgaaattc cgtgacacca aacgttacgt tgaccgtctg 6420

aaagcggcgc gtctgaaatc tggtgaaaac gacgcgctgc tgaacgcgaa aggtcgtctg 6480

gcgggtcgtc agaccgttat cggtgttcag gacttctctt tcatgggtgg ttctatgggt 6540

ctggcggttg gttctgcgtt cgttgcgggt gttaaagcgg cgatcgaatg ccactctccg 6600

tacatcatct tcaccgcggc gggtggtgcg cgtatgcagg aaggtatcct gtctctgatg 6660

cagatgccgc gttctaccgc ggcgatcgcg gaactgcgtg aagcgggtct gccgtacatc 6720

gttgttctga ccgacccgac caccggtggt gttaccgcgt cttacgcgat gctgggtgac 6780

gttcagatcg cggaaccggg tgcgctgatc ggtttcgcgg gtcagcgtgt tatcgaatct 6840

accatccgtg aaaaactgcc ggaaggtttc cagcgtgcgg aatacctgct ggaacacggt 6900

atgatcgaca tggttgttcc gcgtgcggaa atgcgtgacc gtctgatctc tctggttgac 6960

tacctgctgc cggcgaacga agcggcgtaa tttaccaaca ctactacgta taaggagagc 7020

gttgttatga accacctgcg tgcggaaggt ccggcgtctg ttctggcgat cggcaccgcg 7080

aacccggaaa acatcctgct tcaggacgaa tttccggact actacttccg tgttaccaaa 7140

tctgaacaca tgacccagct gaaagaaaaa ttccgtaaaa tctgcgacaa atctatgatc 7200

cgtaaacgta actgcttcct gaacgaagaa cacctgaaac agaacccgcg tctggttgaa 7260

cacgaaatgc agaccctgga cgcgcgtcag gacatgctgg ttgttgaagt tccgaaactg 7320

ggtaaagacg cgtgcgcgaa agcgatcaaa gaatggggtc agccgaaatc taaaatcacc 7380

cacctgatct tcacctctgc gtctaccacc gacatgccgg gtgcggacta ccactgcgcg 7440

aaactgctgg gtctgtctcc gtctgttaaa cgtgttatga tgtaccagct gggttgctac 7500

ggtggtggca ccgttctgcg tatcgcgaaa gacatcgcgg aaaacaacaa aggtgcgcgt 7560

gttctggcgg tttgctgcga catcatggcg tgcctgttcc gtggtccgtc tgaatctgac 7620

ctggaactgc tggttggtca ggcgatcttc ggtgacggtg cggcggcggt tatcgttggt 7680

gcggaaccgg acgaatctgt tggtgaacgt ccgatcttcg aactggtttc taccggtcag 7740

accatcctgc cgaactctga aggcaccatc ggtggtcaca tccgtgaagc gggtctgatc 7800

ttcgacctgc acaaagacgt tccgatgctg atctctaaca acatcgaaaa atgcctgatc 7860

gaagcgttca ccccgatcgg tatctctgac tggaactcta tcttctggat cacccaccct 7920

ggtggtaaag cgatcctgga caaagttgaa gaaaaactgc acctgaaatc tgacaaattc 7980

gttgactctc gtcacgttct gtctgaacac ggtaacatgt cttcttctac cgttctgttc 8040

gttatggacg aactgcgtaa acgttctctg gaagaaggta aatctaccac cggtgacggt 8100

ttcgaatggg gtgttctgtt cggtttcggt ccgggtctga ccgttgaacg tgttgttgtt 8160

cgttctgttc cgatcaaata ctaatttacc aacactacta cgtataagga gagcgttgtt 8220

atggcggtta aacacctgat cgttctgaaa ttcaaagacg aaatcaccga agcgcagaaa 8280

gaagaatttt tcaaaaccta cgttaacctg gttaacatca tcccggcgat gaaagacgtt 8340

tactggggta aagacgttac ccagaaaaac aaagaagaag gttacaccca catcgttgaa 8400

gttaccttcg aatctgttga aaccatccag gactacatca tccacccggc gcacgttggt 8460

ttcggtgacg tttaccgttc tttctgggaa aaactgctga tcttcgacta caccccgcgt 8520

aaataatatt ttttaatatt atgaactgaa tttaagaggc tgcctcccga ttttatcgga 8580

aggcagcctt ttttatactg cag 8603

<210> 5
<211> 8033
<212> DNA
<213> cassette

<220>
<223> Gene cassette (G2)

<400> 5
tctagaatca acaacccgaa tcctatcgta atgatgtttt gcccgatcag cctcaatcga 60

caattttacg cgtttcgatc gaagcaggga cgacaattgg ctgggaacgg tatactggaa 120

taaatggtct tcgttatggt attgatgttt ttggtgcatc ggccccggcg aatgatctat 180

atgctcattt cggcttgacc gcagtcggca tcacgaacaa ggtgttggcc gcgatcgccg 240

gtaagtcggc acgttaaaaa atagctatgg aatatagtag ctacttaata agttaggaga 300

ataaacatga aatctgttac catcctgggt gcgaccggtt ctgttggttc ttctaccctg 360

gacctgatcg aacgtgaacc ggaccgtttc cgtgttgttg cgctgaccgc gaactgcgac 420

gttgcgaaac tggcggcggc ggcgatccgt accaaagcgg aatttgcggt tgttgcggac 480

gcgggttgcc tgccggacct gcgtgacgcg ctggcgggca ccggtatccg tgcgggtggt 540

ggtgcggaag cgatcgcgga agcggcggcg tctggtgcgg actggaccat gggtgcgatc 600

gttggttgcg cgggtctgaa accggttatg gcggcgatcg aacagggtgg caccgttgtt 660

atcgcgaaca aagaaccgct ggtttctgcg ggtgacgtta tcctggcggc ggcgaaacgt 720

accggtgcga ccctgctgcc tgcggactct gaacacaacg cgatcttcca gtgcttcgac 780

gcgtctcgtc cggaacgtgt tcgtcgtatc atcctgacct gctctggtgg tccgttccgt 840

gactggacca ccgaacagat gcgtggtgtt accctggaac aggcggttaa acacccgaac 900

tggtctatgg gtgcgaaaat ctctgttgac tctgcgacct gcatgaacaa aggtctggaa 960

ctgatcgaag cggcgcgtct gttcccgatc ccgcacgacc gtatcgaaat cgttatccac 1020

ccgcagtctg ttgttcactc tctggttgac tacgttgacg gttctgttct ggcgcagctg 1080

ggtccgccgg acatgcgtac cccgatcgcg cacaccctgg cgtggccgga ccgtatggcg 1140

accccgatgg cgccgctgga cctggttgcg atcggtcgtc tggacttcga agcgccggac 1200

ccggttcgtt tcccggcgct gcgtctggcg cgtgaagcgc tggacgcggg tggtgcgcgt 1260

ccggcgatcc tgaacgcggc gaacgaagtt gcggttgaag cgttcctgaa acgtcgtatc 1320

cgtttcctgg aaatcgcggc gatcgttgaa gacaccctgg aacgttacaa cccggcggcg 1380

ccggactctg ttgacgcggt tctggcgatc gacgcggaag cgcgtgttct ggcgggtgaa 1440

cgtgttaaag actgcgcggt ttaatttacc aacactacta cgtataagga gagcgttgtt 1500

atggcgcacg gtcagcgtat cgttgcgatc atcgttgcgg cgggcaccgg tgttcgtgcg 1560

ggtggtgcgg ttccgaaaca gtacgcgcgt atcggtggtc gtccgatgat cgcgtactct 1620

tacgacgcgt tcgcggcgca cccggcgatt gttgaaacca tcgttgttgt tgcggacggt 1680

cagcaggaca tggcggttga cgcgctgggt ggtgacgcga ccatcgttgt tggtggtgcg 1740

acccgtcgtg aatctgttgc gaacggtgtt gcggcggttg gtgcggcgac ccacgttctg 1800

atccacgacg cggcgcgtcc gttcctgtct gcggcggtta tcgactctct ggttgttgcg 1860

ctgaaaaccc gtgacgcggc gatcccggtt ctgccggtta ccgacaccct ggcgcgtggt 1920

tctgttctgc tgggtgacat cgttccgcgt gcggacctga accgtgttca gaccccgcag 1980

gcgttcgcgg ttgaaggtct gcgtgcggcg cacgcggttt ggccggcgga cgaagaagcg 2040

accgacgacg cgcagatggt tcgtcgtctg ggtgttggtg ttgcgctggt tcagggttct 2100

gcgatgctgg aaaaagttac ccacccggaa gacttcgcgg cggcggaagc gcgtgttgcg 2160

gcgaaaacct cttacgaaac ccgtaccgcg atgggtttcg acgttcaccg tctggaaacc 2220

ggtgaagaac tgtggctggg tggtgttctg atcccgcacg acaaaggtct gtctggtcac 2280

tctgacgcgg acgttgcgct gcacgcgctg accgacgcgc tgctgggcac catcgcggcg 2340

ggtgacatcg gcacccactt cccgccgtct gacccgcagt ggaaaggtgc ggactctgcg 2400

cagttccttc agcacgcggc gaaactgatc gcggacaaac acggtcgtat cgacttcgtt 2460

gacctgacca tcatgtgcga agcgccgaaa atcggtccgc accgtgcggc gatgaccgcg 2520

cgtatcgcgg acctgctggg tctgaccctg gaccgtgttt ctgttaaagc gaccaccacc 2580

gaacgtctgg gtttcaccgg tcgtggtgaa ggtatcgcgg cgcaggcggt tgcgaccgtt 2640

cgtgttccgg gtgcgccggc gtaatttacc aacactacta cgtataagga gagcgttgtt 2700

atgccggcga ccaccgaacc ggcgccggcg aaaatcaacc tggcgctgca cgttcgtcgt 2760

cgtcgtgacg acggttacca cgaactggaa accctgttcg cgttcgcgac cgacggtgac 2820

atcgttaccg ttgaaccggc ggcggcgaac tctttcacca tcaccggtcc gttcgcggac 2880

gcgctgctgc cggcgccgaa acacaacacc accccggcgt ctgcgggtgt tgcggcgtgg 2940

ggtggtaacc tggttaccgc ggcggcggac gcgttcaccg cggcgttcgc gccggacacc 3000

caccacgcga tcaccctgga aaaacacctg ccgatcgcgt ctggtatcgg tggtggttct 3060

gcggacgcgg cggcgaccct gcgtgcgctg gcgcgtctgc actgcgttgg tccgaccgac 3120

ccgcgtctgc acgcgatcgc ggcgaccctg ggtgcggacg ttccggcgtg cctggcgaac 3180

cgtaccaccc tgggcaccgg taaaggtgac accctggcga acctggaagg tctgtctggc 3240

accccgctgc tgctggttaa ccctggtgtt gcggtttcta ccgcggcggt tttcaaaacc 3300

tgggacggtc aggaccgtgg tccgatcccg gaaggtgacc tgctgacccg tgcgaccgcg 3360

ggtcgtaacg acctggaacc gccggcgctg gcgctggcgc cggttatcgc ggacgttcgt 3420

accctgctgg acgcggcgca cgaagttctg ctgtctcgta tgtctggttc tggtgcgacc 3480

tgcttcgcgc tgttcgaaac cgcggaagcg cgtgacgcgg cggcgaccgc ggcgctggcg 3540

cgtggttggt ggaccctggc gaccaccctg gcgtaattta ccaacactac tacgtataag 3600

gagagcgttg ttatgtctct gcgtccgtgg cgtgacatca cccgtcgtac ctctcgtcag 3660

atcatggttg gcaccgttcc ggttggtggt gacgcgccgg ttaccgttca gaccatgacc 3720

aacaccccga ccgacgacgt taaagcgacc gttgaccaga tccgtcgttg cgaagaagcg 3780

ggtgttgaca tcatccgtgt ttcttgcccg gacgttgaat ctaccaccgc gctgaaacag 3840

atcgttcgtg cgtctcgtgt tccgatcgtt gcggacatcc acttccacta caaacgtgcg 3900

ctggaagcgg cggacgcggg tgcggcgtgc ctgcgtatca accctggtaa catcggttct 3960

gcggcgcgtg ttaaagaagt tgttgacgcg gcgaaatcta acggttgcgc gatccgtatc 4020

ggtgttaacg gtggttctct ggaacgtcac ctgctggaaa aatacggtga accgtgcccg 4080

gacgcgctgg ttgaatctgc gatggaccac atcaaactgc ttcaggacca cgacttccac 4140

gaatttaaag ttgcggttaa agcgtctgac ctgttcctgg cggttgcggc gtaccagcag 4200

ctggcggaac aggttgactg cccgctgcac ctgggtatca ccgaagcggg tggtttcgtt 4260

ggtggcaccg ttaaatctgc gatcggtatg ggttctctgc tgtggtacgg tatcggtgac 4320

accatccgtg tttctctgtc tgcggaaccg gaagaagaag ttcgtgttgg tttcgaaatc 4380

ctgaaagcgc tgggtatccg taaccgtggt gttcgtgttg tttcttgccc gtcttgcgcg 4440

cgtcagggtt tcgacgttat ccgtaccgtt caggcgctgg aagaacgtct tcagcacatc 4500

cgtaccccga tgtctctgtc tgttctgggt tgcgttgtta acggtccggg tgaagcgcgt 4560

gaaaccgaca tcggtatcac cggtggtggt aacggtaaac acatggttta cctgtctggt 4620

gttaccgacc accacgttca ggacgcggac atggttgacc acatcgttcg tctggttgaa 4680

gcgaaagcgg cggaaatcga agcggcggac gcggcgatcc tggcgtctgt tgcggcgtaa 4740

tttaccaaca ctactacgta taaggagagc gttgttatgc agatcctgct ggcgaacccg 4800

cgtggtttct gcgcgggtgt tgaccgtgcg atctctatcg ttgaaaacgc gctggcgatc 4860

tacggtgcgc cgatctacgt tcgtcacgaa gttgttcaca accgttacgt tgttgactct 4920

ctgcgtgaac gtggtgcgat cttcatcgaa cagatctctg aagttccgga cggtgcgatc 4980

ctgatcttct ctgcgcacgg tgtttctcag gcggttcgta acgaagcgaa atctcgtgac 5040

ctgaccgttt tcgacgcgac ctgcccgctg gttaccaaag ttcacatgga agttgcgcgt 5100

gcgtctcgtc gtggtgaaga atctatcctg atcggtcacg cgggtcaccc ggaagttgaa 5160

ggcaccatgg gtcagtactc taacccggaa ggtggtatgt acctggttga atctccggac 5220

gacgtttgga aactgaccgt taaaaacgaa gaaaaactgt ctttcatgac ccagaccacc 5280

ctgtctgttg acgacacctc tgacgttatc gacgcgctgc gtaaacgttt cccgaaaatc 5340

gttggtccgc gtaaagacga catctgctac gcgaccacca accgtcagga agcggttcgt 5400

gcgctggcgg aacaggcgga agttgttctg gttgttggtt ctaaaaactc ttctaactct 5460

aaccgtctgg cggaactggc gcagcgtatg ggtaaacgtg cgttcctgat cgacgacgcg 5520

aaagacatcc aggaagaatg ggttaaaggt gttaactgcg ttggtgttac cgcgggtgcg 5580

tctgcgccgg acatcctggt tcagaacgtt gttgcgcgtc tgcaccagct gggtggtggt 5640

gaagcgatcc cgctggaagg tcgtgaagaa aacatcgttt tcgaagttcc gaaagaactg 5700

cgtgttgaca tccgtgaagt tgactaattt accaacacta ctacgtataa ggagagcgtt 5760

gttatgttct acctgggtga atttggtctg gaccgtaaag accagcacgt tggtctggcg 5820

aaccagcagt actctgcgac cccggcgaaa gacttcaccg aaaccctgtt cgttcaccac 5880

tctctgccgc agaccaaagt tgacgaagtt gacatctcta cctctgttgc gggtctggac 5940

ttcgcgttcc cgttcttcat caacgcgatg accggtggtt ctaaaaaaac ccgtgaaatc 6000

aaccgtctgc tgggtatcat gggtcacttc ggtaaaatcg cgctggcgtc tggttctgtt 6060

tctgcggcga tcaaagaccc gtctgttgcg gaaaccttct ctgttatgcg tcgtgaaaac 6120

ccgtacggta tcatcttcgc gaacctgggt gcgcaccact ctgttgaaaa cgcgaaacgt 6180

gcggttgacc tgctggaagc gaacgcgatc cagatccacg ttaacgcgcc gcaggaaatc 6240

gttatgccgg aaggtgaccg tgacttcacc atgtggctga aaaacatcga aaccctggtt 6300

cgtgaaatgg aagttccggt tatcgttaaa gaagttggtt tcggtatgtc tcgtgaaacc 6360

gttgcgcagc tggcgtctgc gggtgttcag accatcgacg tttctggcac cggtggcacc 6420

gacttcgcga aaatcgaaaa cgcgcgtcgt accttcaacg actacgcgta cctggaaggt 6480

tggggtcagt ctaccgttac ctctctggtt gaagcgatgt ctgtttctga agaagtttgc 6540

ccgtctctga tcgcgtctgg tggtatcaaa accccgctgg acatcgttaa atctctggcg 6600

ctgggtgcgg acctggttgg tatgtctaac cacttccttc agtacgttaa agacggtaaa 6660

ggtcaccgtt tcgacgacgg tcttcaggcg atcaaagttt accagtggca gatcgcggaa 6720

atcatgacca tgctgggtgc gaaaaacatc gcggaactgc gtcagaaaga cctggttctg 6780

gcgccgaacg ttcagaactg gtgcgaagcg cgtggtatcg actggaaagc gtacgcgcgt 6840

cgttctgcga acgtttctta atttaccaac actactacgt ataaggagag cgttgttatg 6900

gcgggtgcga tcaccgacca gctgcgtcgt tacctgcacg gtcgtcgtcg tgcggcggcg 6960

cacatgggtt ctgactacga cggtctgatc gcggacctgg aagacttcgt tctgggtggt 7020

ggtaaacgtc tgcgtccgct gttcgcgtac tggggttggc acgcggttgc gtctcgtgaa 7080

ccggacccgg acgttctgct gctgttctct gcgctggaac tgctgcacgc gtgggcgctg 7140

gttcacgacg acctgatcga ccgttctgcg acccgtcgtg gtcgtccgac cgcgcagctg 7200

cgttacgcgg cgctgcaccg tgaccgtgac tggcgtggtt ctccggacca gttcggtatg 7260

tctgcggcga tcctgctggg tgacctggcg caggtttggg cggacgacat cgtttctaaa 7320

gtttgccagt ctgcgctggc gccggacgcg cagcgtcgtg ttcaccgtgt ttgggcggac 7380

atccgtaacg aagttctggg tggtcagtac ctggacatcg ttgcggaagc gtctgcggcg 7440

gaatctatcg aatctgcgat gaacgttgcg accctgaaaa ccgcgtgcta caccgtttct 7500

cgtccgcttc agctgggcac cgcggcggcg gcggaccgtt ctgacgttgc ggcgatcttc 7560

gaacacttcg gtgcggacct gggtgttgcg ttccagctgc gtgacgacgt tctgggtgtt 7620

ttcggtgacc cggcggttac cggtaaaccg tctggtgacg acctgaaatc tggtaaacgt 7680

accgttctgg ttgcggaagc ggttgaactg gcggaccgtt ctgacccgct ggcggcgaaa 7740

ctgctgcgta cctctatcgg cacccgtctg accgacgcgc aggttcgtga actgcgtacc 7800

gttatcgaag cggttggtgc gcgtgcggcg gcggaatctc gtatcgcggc gctgacccag 7860

cgtgcgctgg cgaccctggc gtctgcgccg atcaacgcga ccgcgaaagc gggtctgtct 7920

gaactggcga tgatggcggc gaaccgttct gcgtaatatt ttttaatatt atgaactgaa 7980

tttaagaggc tgcctcccga ttttatcgga aggcagcctt ttttataaag ctt 8033

<210> 6
<211> 8531
<212> DNA
<213> cassette

<220>
<223> Gene cassette (G3)

<400> 6
cccgggtgtc tatactccag ttactcaata cgtaacaata atcagtttat cctaactata 60

gaatcgcatg agaagcgata acgtttcacc ataagcaata tattcattgc aacagtggaa 120

ttgccttatg cgtcaaggaa ggatagatca ttgacggact gagttcaaaa agagactcgt 180

ctaaaagatt ttaagaaagg tttcgatatg acccgtgaag ttgttgttgt ttctggtgtt 240

cgtaccgcga tcggcacctt cggtggttct ctgaaagacg ttgcgccggc ggaactgggt 300

gcgctggttg ttcgtgaagc gctggcgcgt gcgcaggttt ctggtgacga cgttggtcac 360

gttgttttcg gtaacgttat ccagaccgaa ccgcgtgaca tgtacctggg tcgtgttgcg 420

gcggttaacg gtggtgttac cgttaacgcg ccggcgctga ccgttaaccg tctgtgcggt 480

tctggtcttc aggcgatcgt ttctgcggcg cagaccatcc tgctgggtga caccgacgtt 540

gcgatcggtg gtggtgcgga atctatgtct cgtgcgccgt acctggcgcc ggcggcgcgt 600

tggggtgcgc gtatgggtga cgcgggtctg gttgacatga tgctgggtgc gctgcacgac 660

ccgttccacc gtatccacat gggtgttacc gcggaaaacg ttgcgaaaga atacgacatc 720

tctcgtaccc agcaggacga agcggcgctg gaatctcacc gtcgtgcgtc tgcggcgatc 780

aaagcgggtt acttcaaaga ccagatcgtt ccggttgttt ctaaaggtcg taaaggtgac 840

gttaccttcg acaccgacga acacgttcgt cacgacgcga ccatcgacga catgaccaaa 900

ctgcgtccgg ttttcgttaa agaaaacggc accgttaccg cgggtaactc ttctggtctg 960

aacgacgcgg cggcggcggt tgttatgatg gaacgtgcgg aagcggaacg tcgtggtctg 1020

aaaccgctgg cgcgtctggt ttcttacggt cacgcgggtg ttgacccgaa aaccatgggt 1080

atcggtccgg ttccggcgac caaaatcgcg ctggaacgtg cgggtcttca ggtttctgac 1140

ctggacgtta tcgaagcgaa cgaagcgttc gcggcgcagg cgtgcgcggt taccaaagcg 1200

ctgggtctgg acccggcgaa agttaacccg aacggttctg gtatctctct gggtcacccg 1260

atcggtgcga ccggtgcgct gatcaccgtt aaagcgctgc acgaactgaa ccgtgttcag 1320

ggtcgttacg cgctggttac catgtgcatc ggtggtggtc agggtatcgc ggcgatcttc 1380

gaacgtatct aatttaccaa cactactacg tataaggaga gcgttgttat gctgtacaaa 1440

ggtgacaccc tgtacctgga ctggctggaa gacggtatcg cggaactggt tttcgacgcg 1500

ccgggttctg ttaacaaact ggacaccgcg accgttgcgt ctctgggtga agcgatcggt 1560

gttctggaac agcagtctga cctgaaaggt ctgctgctgc gttctaacaa agcggcgttc 1620

atcgttggtg cggacatcac cgaatttctg tctctgttcc tggttccgga agaacagctg 1680

tctcagtggc tgcacttcgc gaactctgtt ttcaaccgtc tggaagacct gccggttccg 1740

accatcgcgg cggttaacgg ttacgcgctg ggtggtggtt gcgaatgcgt tctggcgacc 1800

gactaccgtc tggcgacccc ggacctgcgt atcggtctgc cggaaaccaa actgggtatc 1860

atgccgggtt tcggtggttc tgttcgtatg ccgcgtatgc tgggtgcgga ctctgcgctg 1920

gaaatcatcg cggcgggtaa agacgttggt gcggaccagg cgctgaaaat cggtctggtt 1980

gacggtgttg ttaaagcgga aaaactggtt gaaggtgcga aagcggttct gcgtcaggcg 2040

atcaacggtg acctggactg gaaagcgaaa cgtcagccga aactggaacc gctgaaactg 2100

tctaaaatcg aagcgaccat gtctttcacc atcgcgaaag gtatggttgc gcagaccgcg 2160

ggtaaacact acccggcgcc gatcaccgcg gttaaaacca tcgaagcggc ggcgcgtttc 2220

ggtcgtgaag aagcgctgaa cctggaaaac aaatctttcg ttccgctggc gcacaccaac 2280

gaagcgcgtg cgctggttgg tatcttcctg aacgaccagt acgttaaagg taaagcgaaa 2340

aaactgacca aagacgttga aaccccgaaa caggcggcgg ttctgggtgc gggtatcatg 2400

ggtggtggta tcgcgtacca gtctgcgtgg aaaggtgttc cggttgttat gaaagacatc 2460

aacgacaaat ctctgaccct gggtatgacc gaagcggcga aactgctgaa caaacagctg 2520

gaacgtggta aaatcgacgg tctgaaactg gcgggtgtta tctctaccat ccacccgacc 2580

ctggactacg cgggtttcga ccgtgttgac atcgttgttg aagcggttgt tgaaaacccg 2640

aaagttaaaa aagcggttct ggcggaaacc gaacagaaag ttcgtcagga caccgttctg 2700

gcgtctaaca cctctaccat cccgatctct gaactggcga acgcgctgga acgtccggaa 2760

aacttctgcg gtatgcactt cttcaacccg gttcaccgta tgccgctggt tgaaatcatc 2820

cgtggtgaaa aatcttctga cgaaaccatc gcgaaagttg ttgcgtgggc gtctaaaatg 2880

ggtaaaaccc cgatcgttgt taacgactgc cctggtttct tcgttaaccg tgttctgttc 2940

ccgtacttcg cgggtttctc tcagctgctg cgtgacggtg cggacttccg taaaatcgac 3000

aaagttatgg aaaaacagtt cggttggccg atgggtccgg cgtacctgct ggacgttgtt 3060

ggtatcgaca ccgcgcacca cgcgcaggcg gttatggcgg cgggtttccc gcagcgtatg 3120

cagaaagact accgtgacgc gatcgacgcg ctgttcgacg cgaaccgttt cggtcagaaa 3180

aacggtctgg gtttctggcg ttacaaagaa gactctaaag gtaaaccgaa aaaagaagaa 3240

gacgcggcgg ttgaagacct gctggcggaa gtttctcagc cgaaacgtga cttctctgaa 3300

gaagaaatca tcgcgcgtat gatgatcccg atggttaacg aagttgttcg ttgcctggaa 3360

gaaggtatca tcgcgacccc ggcggaagcg gacatggcgc tggtttacgg tctgggtttc 3420

ccgccgttcc acggtggtgc gttccgttgg ctggacaccc tgggttctgc gaaatacctg 3480

gacatggcgc agcagtacca gcacctgggt ccgctgtacg aagttccgga aggtctgcgt 3540

aacaaagcgc gtcacaacga accgtactac ccgccggttg aaccggcgcg tccggttggt 3600

gacctgaaaa ccgcgtaatt taccaacact actacgtata aggagagcgt tgttatgctg 3660

taccagtctg aaacccttca gctgcactgg ctggaaaacg gtatcgcgga actggttttc 3720

gacgcgccgg gttctgttaa caaactggac acccagaccg ttgcgaacct gggtgaagcg 3780

ctggttgttc tggaaaaaca gtctgaactg aaaggtctgc tgctgcgttc tgcgaaagcg 3840

gcgttcatcg ttggtgcgga catcaccgaa tttctgtctc tgttcaacga accgccggaa 3900

aaactgcacc agtggctggt tttcgcgaac gacatcttca accgtctgga agacctgccg 3960

gttccgacca tcgcggcgat caacggttac gcgctgggtg gtggttgcga atgcatcctg 4020

gcgaccgact tccgtgttgc gtctccggaa gcgcgtatcg gtctgccgga aaccaaactg 4080

ggtatcatgc cgggtttcgg tggttctgtt cgtctgccgc gtctgctggg tgcggactct 4140

gcgctggaaa tcatcgcggc gggtaaagac atcatcgcga aagacgcgct gaaagttggt 4200

ctggttgacg cggttgttgc gccggaaaaa ctggttgacg cggcgctgtc tatcctgaac 4260

caggcgatcg acggtaaact ggactggcag gcggcgcgtc gtccgaaact ggaaccgctg 4320

aaactgaacc cgaccgaagc ggcgatgtgc ttcaccatcg cgaaaggtat ggttatgcag 4380

gttgcgggta aacactaccc ggcgccgctg accgcggtta aaaccatcga agcggcggcg 4440

aaattcggtc gtgcggaagc gctgatcctg gaaaccaact ctttcgttcc gctggcgggt 4500

tctaacgaag cgcgtgcgct ggttggtatc ttcctgaacg accagtacgt taaaggtcag 4560

gcgaaaaaac tgtctaaagg tgtttctgcg ccgaaacagg cggcggttct gggtgcgggt 4620

atcatgggtg gtggtatcgc gtaccagtct gcgctgaaag gtgttccggt tatcatgaaa 4680

gacatcaacg acaaatctct gaccctgggt atgaacgaag cggcgaaact gctgaacaaa 4740

cagctggaac gtggtaaaat cgacggtctg aaaatggcga acatcctggc gaccatccag 4800

ccgaccctgg actacgcggg tatcgaacgt gcgcaggtta tcgttgaagc ggttgttgaa 4860

aacccgaaag ttaaagcggc ggttctggcg gaagttgaaa ccctgatcgg tgaagaaacc 4920

gttctggcgt ctaacacctc taccatcccg atcgaccagc tggcgaaatc tctgaaacgt 4980

ccggaaaact tctgcggtat gcacttcttc aacccggttc accgtatgcc gctggttgaa 5040

atcatccgtg gcaccaaaac ctctgacaaa accatcgcgg cggttgttgc gtacgcgacc 5100

cagatgggta aaaccccgat cgttgttaac gactgccctg gtttcttcgt taaccgtgtt 5160

ctgttcccgt acctggcggg tttcggtatg ctggttcgtg acggtgcgga cttccgtcag 5220

atcgacaaag ttatggaaaa acagttcggt tggccgatgg gtccggcgta cctgctggac 5280

gttgttggta tcgacaccgc gcaccacgcg caggcggtta tggcggttgg tttcccggaa 5340

cgtatgaaca aagactaccg tgacgcggtt gacgttatgt tcgacaacca gcgtttcggt 5400

cagaaaaacg gtcagggttt ctaccgttac acccaggaca ccaaaggtaa accgcgtaaa 5460

gaaaacgacg aacaggttga cgcgctgctg gcgcaggttt ctcagccgct tcagaaattc 5520

tctgacgacg acatcatcgc gcgtaccatg atcccgatga tcaacgaagt tgttcgttgc 5580

ctggaagaag gtatcatcgc gtctccggcg gaaggtgaca tggcgctggt ttacggtctg 5640

ggtttcccgc cgttccacgg tggtgttttc cgttacctgg acaccatcgg ttctgcgaac 5700

tacgttgaaa tggcgcagcg ttacacccac ctgggtgcgc tgtaccaggt tccgccgggt 5760

ctgcgtgcga aagcggaaca caacgaatct tactacccgg ttgcggcggc gctgctggac 5820

gtttctatct ctcagccggc gtaatttacc aacactacta cgtataagga gagcgttgtt 5880

atgtcttgcc cggcgtctcc gtctgcggcg gttgtttctg cgggtgcgct gtgcctgtgc 5940

gttgcgaccg ttctgctggc gaccggttct aacccgaccg cgctgtctac cgcgtctacc 6000

cgttctccga cctctctggt tcgtggtgtt gaccgtggtc tgatgcgtcc gaccaccgcg 6060

gcggcgctga ccaccatgcg tgaagttccg cagatggcgg aaggtttctc tggtgaagcg 6120

acctctgcgt gggcggcggc gggtccgcag tgggcggcgc cgctggttgc ggcggcgtct 6180

tctgcgctgg cgctgtggtg gtgggcggcg cgtcgttctg ttcgtcgtcc gctggcggcg 6240

ctggcggaac tgccgaccgc ggttacccac ctggcgccgc cgatggcgat gttcaccacc 6300

accgcgaaag ttatccagcc gaaaatccgt ggtttcatct gcaccaccac ccacccgatc 6360

ggttgcgaaa aacgtgttca ggaagaaatc gcgtacgcgc gtgcgcaccc gccgacctct 6420

ccgggtccga aacgtgttct ggttatcggt tgctctaccg gttacggtct gtctacccgt 6480

atcaccgcgg cgttcggtta ccaggcggcg accctgggtg ttttcctggc gggtccgccg 6540

accaaaggtc gtccggcggc ggcgggttgg tacaacaccg ttgcgttcga aaaagcggcg 6600

ctggaagcgg gtctgtacgc gcgttctctg aacggtgacg cgttcgactc taccaccaaa 6660

gcgcgtaccg ttgaagcgat caaacgtgac ctgggcaccg ttgacctggt tgtttactct 6720

atcgcggcgc cgaaacgtac cgacccggcg accggtgttc tgcacaaagc gtgcctgaaa 6780

ccgatcggtg cgacctacac caaccgtacc gttaacaccg acaaagcgga agttaccgac 6840

gtttctatcg aaccggcgtc tccggaagaa atcgcggaca ccgttaaagt tatgggtggt 6900

gaagactggg aactgtggat tcaggcgctg tctgaagcgg gtgttctggc ggaaggtgcg 6960

aaaaccgttg cgtactctta catcggtccg gaaatgacct ggccggttta ctggtctggc 7020

accatcggtg aagcgaaaaa agacgttgaa aaagcggcga aacgtatcac ccagcagtac 7080

ggttgcccgg cgtacccggt tgttgcgaaa gcgctggtta cccaggcgtc ttctgcgatc 7140

ccggttgttc cgctgtacat ctgcctgctg taccgtgtta tgaaagaaaa aggcacccac 7200

gaaggttgca tcgaacagat ggttcgtctg ctgaccacca aactgtaccc ggaaaacggt 7260

gcgccgatcg ttgacgaagc gggtcgtgtt cgtgttgacg actgggaaat ggcggaagac 7320

gttcagcagg cggttaaaga cctgtggtct caggtttcta ccgcgaacct gaaagacatc 7380

tctgacttcg cgggttacca gaccgaattt ctgcgtctgt tcggtttcgg tatcgacggt 7440

gttgactacg accagccggt tgacgttgaa gcggacctgc cgtctgcggc gcagcagtaa 7500

tttaccaaca ctactacgta taaggagagc gttgttatgt ctggtgcggc ggacgttgaa 7560

cgtgtttacg cggcgatgga agaagcggcg ggtctgctgg acgtttcttg cgcgcgtgaa 7620

aaaatctacc cgctgctgac cgttttccag gacaccctga ccgacggtgt tgttgttttc 7680

tctatggcgt ctggtcgtcg ttctaccgaa ctggacttct ctatctctgt tccggtttct 7740

cagggtgacc cgtacgcgac cgttgttaaa gaaggtctgt tccaggcgac cggttctccg 7800

gttgacgaac tgctggcgga caccgttgcg cacctgccgg tttctatgtt cgcgatcgac 7860

ggtgaagtta ccggtggttt caaaaaaacc tacgcgttct tcccgaccga cgacatgccg 7920

ggtgttgcgc agctggcggc gatcccgtct atgccggcgt ctgttgcgga aaacgcggaa 7980

ctgttcgcgc gttacggtct ggacaaagtt cagatgacct ctatggacta caaaaaacgt 8040

caggttaacc tgtacttctc tgacctgaaa caggaatacc ttcagccgga atctgttgtt 8100

gcgctggcgc gtgaactggg tctgcgtgtt ccgggtgaac tgggtctgga attttgcaaa 8160

cgttctttcg cggtttaccc gaccctgaac tgggacaccg gtaaaatcga ccgtctgtgc 8220

ttcgcggcga tctctaccga cccgaccctg gttccgtctg aagacgaacg tgacatcgaa 8280

atgttccgta actacgcgac caaagcgccg tacgcgtacg ttggtgaaaa acgtaccctg 8340

gtttacggtc tgaccctgtc ttctaccgaa gaatactaca aactgggtgc gtactaccac 8400

atcaccgaca tccagcgtca gctgctgaaa gcgttcgacg cgctggaaga ctaatatttt 8460

ttaatattat gaactgaatt taagaggctg cctcccgatt ttatcggaag gcagcctttt 8520

ttataggatc c 8531

<210> 7
<211> 4723
<212> DNA
<213> plasmids

<220>

<223> CRISRP_Loci-pUCZM-7

<400> 7
ccgggaattt gacccttatt ttgaccctct tttttcgagg gtataaaaaa tcctttcgat 60

tcaatatgtt acatatgagc atatttttta gggttatttt gcctttttgg cgagatatcc 120

ctttatttta ggggcaattc tatcttttgc ctctagttca ctgccgcaca ggcagcttag 180

aaaggagacc gaggtctcag ttcactgccg cacaggcagc ttagaaagga tcctctagac 240

tgcagaagct tataaaacga aaggctcagt cgaaagactg ggcctttcgt tttatctgtt 300

gtttgtcggt gaacgctctc ctgagtagga caaatccgcc gggagcggat ttgaacgttg 360

cgaagcaacg gcccggaggg tggcgggcag gacgcccgcc ataaactgcc aggcatcaaa 420

ttaagcagaa ggccatcctg acggatggcc tttgtagaaa agatcaaagg atcttcttga 480

gatccttttt ttctgcgcgt aatctgctgc ttgcaaacaa aaaaaccacc gctaccagcg 540

gtggtttgtt tgccggatca agagctacca actctttttc cgaaggtaac tggcttcagc 600

agagcgcaga taccaaatac tgtccttcta gtgtagccgt agttaggcca ccacttcaag 660

aactctgtag caccgcctac atacctcgct ctgctaatcc tgttaccagt ggctgctgcc 720

agtggcgata agtcgtgtct taccgggttg gactcaagac gatagttacc ggataaggcg 780

cagcggtcgg gctgaacggg gggttcgtgc acacagccca gcttggagcg aacgacctac 840

accgaactga gatacctaca gcgtgagcta tgagaaagcg ccacgcttcc cgaagggaga 900

aaggcggaca ggtatccggt aagcggcagg gtcggaacag gagagcgcac gagggagctt 960

ccagggggaa acgcctggta tctttatagt cctgtcgggt ttcgccacct ctgacttgag 1020

cgtcgatttt tgtgatgctc gtcagggggg cggagcctat ggaaaaacgc cagcaacgcg 1080

gcctttttac ggttcctggc cttttgctgg ccttttgctc attcaaatat gtatccgctc 1140

atgagacaat aaccctgata aatgcttcaa taatattgaa aaaggaagag tatggaaaaa 1200

aaaatcaccg gctataccac cgtggatatt agccagtggc atcgtaaaga acattttgaa 1260

gcgtttcaga gcgtggcgca gtgcacctat aaccagaccg tgcagctgga tatcaccgcg 1320

tttctgaaaa ccgtgaaaaa aaacaaacac aaattctacc cggcgtttat tcatattctg 1380

gcccgtctga tgaacgcgca tccggaattt cgtatggcca tgaaagatgg cgaactggtg 1440

atttgggata gcgtgcatcc gtgctatacc gtgtttcatg aacagaccga aacctttagc 1500

agcctgtgga gcgaatatca tgatgatttt cgccagttcc tgcatattta tagccaggat 1560

gtggcgtgct atggcgaaaa cctggcctat tttccgaaag gcttcatcga aaacatgttc 1620

tttgtgagcg cgaatccgtg ggtgagcttt accagcttcg atctgaacgt ggcgaacatg 1680

gataactttt ttgcgccggt gtttaccatg ggcaaatatt atacccaggg cgataaagtg 1740

ctgatgccgc tggccattca ggtgcatcat gcggtgtgtg atggctttca tgtgggccgt 1800

atgctgaacg aactgcagca gtattgtgat gaatggcagg gcggtgcgta ataactgtca 1860

gaccaagttt actcatatat actttagatt gatttaaaac ttcattttta atttaaaagg 1920

atctaggtga agatcctttt tgataatctc atgaccaaaa tcccttaacg tgagttttcg 1980

ttccactgag cgtcgacggt accatgatta tcggcttctc aaaacatgga cagggcaaag 2040

ctgctccagc cataaaatac ctgctcgatc agcacagaga gggcagagaa aatcatattc 2100

ctgaagtggt gagaggtaat ccagacttaa ccgcatccat tatcgacagc ctgactttca 2160

aacatcacta tactagcgga gttctttctt tcgctgccga agatcatgtt tctgccgagc 2220

aggaaaaaga tattatcaac gatttcgaaa agctggcttt tgctggcctg caagcagacc 2280

agtttaatat cctttgggtg cgccattctc atgcgggtca tcatgaactt catttcatca 2340

ctccaaggat agaactttca tccaagaagt ccttcaacat caagcccccc ggtaagaata 2400

cccaaaagga atttgatgac ttgcggagtg ccatcaatgc caaatatggc tttgctgacc 2460

cagaagaccc gatgagagcg agaaacgtca aaacacctag ctatgaacta aaaatcgcta 2520

ggaatgtcct tagagagcaa aaagcgccct ctgaggacat aagacagctt attgatagcc 2580

ttttaactga aagagccgtc caaggcctga tacatgacag aaaaactctt ctcactaatg 2640

ttgaagagtt aggcttcgaa gttactcgcg caggcaagaa ttatattacc gtcaaagacc 2700

ctgaaagctc aaaacgattt cgtctcaaag gccaactcta tgaacaagac tttacctcta 2760

gccgaacgat tgaaacagca gcagctaaaa gaacaagaga ttattctcga cctgactcaa 2820

cagcagctag acaatttgag gaacaagtta aactccacat tcgaaaacga gctgaattca 2880

atagaaaacg atatcgagcg actgaaccgg agcttaggat ggaaattcct cctatctcgc 2940

tggctgatac cagtcgtgac cagcatcatg atattgtttg gcctgctctt gggcgcagaa 3000

gggataatca agctctatac ccaccagatc aaagctttgc aggaggaaaa agcagagatg 3060

gaagtgaccg tcgaacagct caacaaagca ggtgggaaga tgcaattcag ccattgcgga 3120

gaaaaagaaa ggctttgcat cagagtaaac acacgagaga tcttcgggat aaccagatat 3180

tatctgatcg tgaaagaacg gaaatagaca atgacggaat tggaacaggt tttactgaac 3240

gccttggaaa atttacagca gagattcgat caacaacaga aaagtttatt acacgcgcaa 3300

agcgaattac ggcagatgtt cgaaactacc tcaaccgaga atacttattt acgacagcaa 3360

gtagaaatct tggtcgagcg attgaacagt tttcccagat gaaaacctta agtcgaaagc 3420

tacaaaaacg gagaggtcgt tctcgctctc gataaacttg aaaaggacgc taattccgaa 3480

aagtttttta tctttctgat agtcgatttt agttggcgat ttccgataaa aaagccctga 3540

acttattgaa ctttcctata tttcaataag ttcaataagt tcagggcttt tttattcaaa 3600

aaaatcaatg ccttacgact ttaaaaacca cttttgggga gtgaaaaacc acttttgggg 3660

agtgaaaaac cacttttggg gagtgaaaaa ccacttcaaa aaatatatta agtggttaat 3720

aaagttgtaa tgagaaaaac actacaaata attaaagata gagccggcaa taaagatcaa 3780

aatttacatg caggtgaaat tatagatgga catttttcag accttaaaaa tccgcctagc 3840

ctagctgcac taaaactcta tgagttgctt attaaatacg cagcatataa tatagataaa 3900

aatatatggc ataatatttc tgtatcagaa atgcaaaaaa tcaaaggaat aaagaactat 3960

aactcaacag agctaactca attattaaaa gaacttagag ctgttgttat ggaatataga 4020

gcaaaagata gaactattat tactggtctt ttagatattg ctgcggtcat atctaaccca 4080

gacacaggat atctagagat acaatggaaa tttggagaaa gttttagaga tttaatagct 4140

aagtcagaat attgggcaat tgttgattat agaacaacgt tagagatgac gagccgatat 4200

tcccttcgtt tatatgaaat attagctctt agaataaatt taaaacacaa aaattcagat 4260

acatttactt tagccgagct tagaggaaaa ctgggagttc ctgaaggaaa attaaaaaga 4320

tggtctgatt taagagataa agcgatcaat gttgcggtaa aagaaattaa ccaacggact 4380

actttcgccg taacagcaca accaaaaaaa atgggaagat ccgttcaagc cgtagaattt 4440

aaatgggaac gaaaagagat taaacttcct caaagtatac cattaaaaac atctaaaagc 4500

attgtttttc cattagatgg aagtattaga tatactcaat gggaagatat tgcacgaaaa 4560

aatcttccta agcctttacc tgatttagat atggtagcac aacaatttag aaaatggtta 4620

aaaaataaaa acatttcatc aaacagtatt aatgttttgt ccatttttaa gaaattttgt 4680

gaaaaataca gaatagaatt taataattaa gaattcagta ctc 4723

<210> 8
<211> 28
<212> DNA
<213> plasmids

<220>
<223> CRISPR Plasmid Repeat Sequences

<400> 8
gttcactgcc gcacaggcag cttagaaa 28

<210> 9
<211> 2362
<212> DNA
<213> Zymomonas mobilis

<220>
<223> Origen of replication 1 Zymomonas mobilis (Oril)

<400> 9
cggaaaggtc atcctgaaaa ttctattcgt attctgatca accagatcgt gaatgatcgg 60

gcgatgagcc ggaaacttgt cccgtgatca gaaccggcgc agcctcttcc cttttatcta 120

tttcaaattc aggcctgcca cagcggagaa tcttcgctgt ggcttttttg cgtccaagac 180

cggagcccgc acaatgaccg cctcttcctc ttcagcgccc ctctcggatg cctctgtctt 240

ttctttagag cctgtctctg cgtcttttga acaaacgacg cccgtaacca gtccttttat 300

cgacaggcta ggccgtttca agcgtggcac ttatcgaacc atgttggttg aaacgggtat 360

gccatttgac agcgcaaaga ttatggcaga tcaggccgca gaacaacata aaagagagcg 420

cacattctct aaattaatgc ccttggccaa tggcgctcat cccgaaattt cggcctcaaa 480

taccaatatt gaggctacag cctctgcatc agatttcgac gaaatcgaat attttgacca 540

gatggtatca aacggcgttg atcctagaga tgccgcagat attgctcgca aaacagctat 600

tgcgagacgt cgagaccctc tcgaacgagc cattcgctat aaaacccgcc gtcaaactcc 660

tgatcctgtg catcgggatt cttatgaggt gggcgaacgc gagaagtcgg tctggaagcc 720

ggtcaatccg caagagattg gtgcctatct ggaagcggtt gatcaatatt cgatcaaaac 780

caagggctta agcgataagg ccgttcgtct tctaaagatg ctgtttcgga tggtcgactt 840

taaaactggc cgtctggagc cgaccctcga cacaatatgc gatcgtgtcg gttatgcccg 900

tgcgacggtc gtcaaattac tgcggcaatt acaggattta ggcttcatcc gctggatacg 960

ccggtcaatc aagatcaagg cggacggcaa aggcccccgc cgcaagcaga ccagtaacgc 1020

ctacggcttc ttgtctccca aggcttggcc agatttggcc agacaggttt tcgagcgtgt 1080

gatgcgccgg aggcatgcgc ctattcctga cgatatcgac cacgcccaag aggcggacaa 1140

agccgaaacc aaggctatcg ttgaaaactt gccgcccgaa gatttcgtca aagcaatatc 1200

gggcgataaa gagccaacag agttaacaga ttcgttaata cagctcgcca aatctatcga 1260

gcgggcggaa caacgggaac aagaagaaca ggctaagaag gcagcacagg ccggtcagga 1320

agaggttaaa acatcttccc aaacacctaa ggaggatacc tcaaaaaagc ccccaatctc 1380

gggcaatcaa cgcgagttca attcttatac tctatcctgt tatacttatt tatctaatag 1440

cgaccataca aaactaaaaa ccaaagaaga agataaagac tgcactgctc ctgctaccgc 1500

aggcagtgca gaccctattt atgatcgaaa acctgacagc aatcctttcc tctcaactct 1560

caaagctcgc ggttatcgtc ctgatcaaat caacgcttgc ctcgaaaaca gagataaacg 1620

gaaaaagaaa ggatggatgc ctctgtcacc cgatgaaacc gcccgcctcc ttattcaaac 1680

aaaacagatg tgcccataac aacgaccact atccttctca aagcatatcg tccgttaatc 1740

tctcggcgct gctcgcttcg ctcacaggct cggcgagaaa gaaatttgcc tttcggggga 1800

ctatctaatt cctaacaatt cgcattatcg ataatgtaat aaaaggctaa tctttaaaaa 1860

ccaaattaca gcgattacat catttctatg aatgtgtatt atacataata tgagaagtcg 1920

tgatttgatc aaagaagtta aaaaagccaa tattaccagc ttgacagata taaaataggc 1980

cttggttgat ttcgtgaaag aactggaaga ttgcacaatt cttctttctg gtgatatctt 2040

tgacccgacg gccctcagtc aatgagtgtg cgccgcattt ctgcccattc aatcagggcc 2100

tgcattgaag ggccgagagc gtatcccata ccgctgagct cgtattctac ctttggcggg 2160

acctgcggat agaccgttcg gagcacgatg cgatcctttt ccagttgctt gagctgctgg 2220

atcagcatct tctggttgat gccgtcgatc tgcttttcca gtgaggagaa gcggattggg 2280

ccttttgcgg caaacaactg gcagatgata acaatcttcc attttccttc gagtaccctg 2340

agcgcttctg tcgtcgcctc ag 2362

<210> 10
<211> 2801
<212> DNA
<213> Zymomonas mobilis

<220>
<223> Origen of replication 2 Zymomonas mobilis (Ori2)

<400> 10
gcaccgcaaa ggacatgctg atcgcgagat cggcgcgcta ctcgacactg tcataacaga 60

tcacattcgg agatagctga tgaatactgc gcagaatcag actcttaaaa gagcgttttt 120

gtcatcagaa gccgcagcag ctgactcttc agctgactgc ggcttttcta tgtctgaaaa 180

agatatgcca gattttgaag aaatgacgcc agccagccct tttatcgaca ggctaggccg 240

tttcaagcgc ggcacttatc gaagcatgtt gatgcagacg gggctttctt atgaacgcgc 300

gaaaggcatg gcagacaatg cggcggaaga atttaaaaaa tcaaaagcaa agtcgaaatc 360

tttgccattg gccaagagcg aaatcgctga acttggggct tcagaagcaa cgattgaagt 420

aacagctcat caggtaacat tttctgaatc acaggcacaa ccagaacctt tcgatgctta 480

ctgctgcttg caacagttaa aagatcaggg cgtggacggt gccacagctg cgaagattgc 540

aaatcaggcc tacaagcatc acaaagaagc cgaaaatttt gacgaagcgg tatatttcaa 600

ggaattagtc gatactggca tagatgcggt gatggctgct aatatggctc gtaaaacagc 660

cgaagctaag cgccgcgacc cgctggaaag atacggtatc cgtttaaagc cgcgccgcac 720

gcctgatcct gttcaccgtg actcttatga tgttggcgag cgcgaaaata cggtctggaa 780

gccggtcaat ccgaatgaga tcggggctta tctggaagca gtcgatcaat attcaatcaa 840

gaccggcaat atcagtgata gagccgtccg gctgctcaag atgctgtttc ggatggtgga 900

cttcaagaca ggtcgattag agccgacgct tgatacaatc tgcgaatggg tcggctatgc 960

acgggcaacg gttgtccgct tgctaaaaca gcttcaagag caaggtttta tccgctggat 1020

acgccgttca atcaagatca agacggatgg ggctgggccc cgccgcaaac agaccagtaa 1080

cgcttacggt ttcttgtcgc ctaaatcttg gccggagatg gcaagaaagg ttttcgagcg 1140

cgtgatgcgc cggaagaatg cgccggtctc tgctgatctt gctcatgctc aagaagccga 1200

caaagtcgaa accggtgcca tgattgaaag ccagccgaat gacgactggg tgaaagaaat 1260

cttcgcacag aagcgcgcgc aacatccgac gagtgacgag cagaaaaaca aaaaagaaca 1320

ggccgatgat ctggcagaga ctcttgccag cttcgccaaa tctatcgagc gggcagaaca 1380

acgggaacaa gaagaacagg ccaagaaagc catttcggcc gagcaaaatg aggttaaaac 1440

accctacaaa acactcaagg aggacacctc aaaaacgccc caaatttcgg cagttgagcg 1500

cgagttcaat tttgatactc tatcccgtta tagttatttt aataataacc actccaatca 1560

atttaaaaac caaaaagaga ttaaggactg cactgctcct gctgccgcag gcagtgcaga 1620

cgttgttttg aaaacaaaac ctcaaaccga cactttccta aaaacccttc aaactcgcgg 1680

ttaccgtcaa gaccagacca acgcttgcct cgaaaacaga gataaacgga aaaagaatgg 1740

atggatgcct ctgtcagcgg acgaaaccgc ccgcctcctt atacaaacaa ggcagatgcg 1800

accgtgatac cgacagctat ccttctcaaa gcacaccgcc cattaatctc tcggcgctgc 1860

tcgcttcgct cacaggctcg gcgaaaaaga aatttgcctt ttggggggct attaacttct 1920

aacaactcgc attatcgaaa tgtctttaaa taggtttatt ctctaaaagc gtttcgacat 1980

ggcttttact gttatccatc atccaatctt cagtcggtct tgagcgatat ccaatcaaac 2040

gaacaattct atcccagtca gcttgtagat ctctttcaaa tataggctca tgatgggcta 2100

tccgattacg cagcatccga atttcaccca aatctctata gagactctgt ctctggattt 2160

taatctcttg ctgtggatca agattcggga acacgttcaa caaatagaga tcccaaagcc 2220

gtttatcatg gcgctttgtg aacatagcct gccagaatac aaaattcagt tctggtatta 2280

cctttccggt cgtttccgcc ttcaaacagg ctttgtttac atccttcgat gcgctgtatc 2340

ctgatttgac aactggaagg cttcgtcgga aaccttctga ccacggccac tgatctccat 2400

aggtatagga tagcgcttcc gccacagcat ttcgggtcgc tatttctgtt atatgaaggg 2460

gggctagcaa tgcgccggat agctgggcat tccacacata taaatccaga gcgacgagat 2520

catcttttcc caatgccctg actgtgtctt catatcttcc aagtcgggga gccgataagg 2580

tctttctgat agaggtaatc ttgtcttgaa ttgacagcat ggctacccat ttatatgatc 2640

aaccacatat tcgcctcggg tctttgttgg cttatgcctc cccctgagga caaaagatac 2700

aaaaaagacc tcgcttacgg cggggtcttt ttttatccat tttcctgtat tgtataaagt 2760

ctttgctagc ctgacggaaa acgtaatcct gcggttaatc g 2801

<210> 11
<211> 2707
<212> DNA
<213> Zymomonas mobilis

<220>

<223> Origen of replication 3 Zymomonas mobilis (Ori3)

<400> 11
atgattatcg gcttctcaaa acatggacag ggcaaagctg ctccagccat aaaatacctg 60

ctcgatcagc acagagaggg cagagaaaat catattcctg aagtggtgag aggtaatcca 120

gacttaaccg catccattat cgacagcctg actttcaaac atcactatac tagcggagtt 180

ctttctttcg ctgccgaaga tcatgtttct gccgagcagg aaaaagatat tatcaacgat 240

ttcgaaaagc tggcttttgc tggcctgcaa gcagaccagt ttaatatcct ttgggtgcgc 300

cattctcatg cgggtcatca tgaacttcat ttcatcactc caaggataga actttcatcc 360

aagaagtcct tcaacatcaa gccccccggt aagaataccc aaaaggaatt tgatgacttg 420

cggagtgcca tcaatgccaa atatggcttt gctgacccag aagacccgat gagagcgaga 480

aacgtcaaaa cacctagcta tgaactaaaa atcgctagga atgtccttag agagcaaaaa 540

gcgccctctg aggacataag acagcttatt gatagccttt taactgaaag agccgtccaa 600

ggcctgatac atgacagaaa aactcttctc actaatgttg aagagttagg cttcgaagtt 660

actcgcgcag gcaagaatta tattaccgtc aaagaccctg aaagctcaaa acgatttcgt 720

ctcaaaggcc aactctatga acaagacttt acctctagcc gaacgattga aacagcagca 780

gctaaaagaa caagagatta ttctcgacct gactcaacag cagctagaca atttgaggaa 840

caagttaaac tccacattcg aaaacgagct gaattcaata gaaaacgata tcgagcgact 900

gaaccggagc ttaggatgga aattcctcct atctcgctgg ctgataccag tcgtgaccag 960

catcatgata ttgtttggcc tgctcttggg cgcagaaggg ataatcaagc tctataccca 1020

ccagatcaga gctttgcagg aggaaaaagc agagatggaa gtgaccgtcg aacagctcaa 1080

caaagcaggt gggaagatgc aattcagcca ttgcggagaa aaagaaaggc tttgcatcag 1140

agtaaacaca cgagagatct tcgggataac cagatattat ctgatcgtga aagaacggaa 1200

atagacaatg acggaattgg aacaggtttt actgaacgcc ttggaaaatt tacagcagag 1260

attcgatcaa caacagaaaa gtttattaca cgcgcaaagc gaattacggc agatgttcga 1320

aactacctca accgagaata cttatttacg acagcaagta gaaatcttgg tcgagcgatt 1380

gaacagtttt cccagatgaa aaccttaagt cgaaagctac aaaaacggag aggtcgttct 1440

cgctctcgat aaacttgaaa aggacgctaa ttccgaaaag ttttttatct ttctgatagt 1500

cgattttagt tggcgatttc cgataaaaaa gccctgaact tattgaactt tcctatattt 1560

caataagttc aataagttca gggctttttt attcaaaaaa atcaatgcct tacgacttta 1620

aaaaccactt ttggggagtg aaaaaccact tttggggagt gaaaaaccac ttttggggag 1680

tgaaaaacca cttcaaaaaa tatattaagt ggttaataaa gttgtaatga gaaaaacact 1740

acaaataatt aaagatagag ccggcaataa agatcaaaat ttacatgcag gtgaaattat 1800

agatggacat ttttcagacc ttaaaaatcc gcctagccta gctgcactaa aactctatga 1860

gttgcttatt aaatacgcag catataatat agataaaaat atatggcata atatttctgt 1920

atcagaaatg caaaaaatca aaggaataaa gaactataac tcaacagagc taactcaatt 1980

attaaaagaa cttagagctg ttgttatgga atatagagca aaagatagaa ctattattac 2040

tggtctttta gatattgctg cggtcatatc taacccagac acaggatatc cagagataca 2100

atggaaattt ggagaaagtt ttagagattt aatagctaag tcagaatatt gggcaattgt 2160

tgattataga acaacgttag agatgacgag ccgatattcc cttcgtttat atgaaatatt 2220

agctcttaga ataaatttaa aacacaaaaa ttcagataca tttactttag ccgagcttag 2280

aggaaaactg ggagttcctg aaggaaaatt aaaaagatgg tctgatttaa gagataaagc 2340

gatcaatgtt gcggtaaaag aaattaacca acggactact ttcgccgtaa cagcacaacc 2400

aaaaaaaatg ggaagatccg ttcaagccgt agaatttaaa tgggaacgaa aagagattaa 2460

acttcctcaa agtataccat taaaaacatc taaaagcatt gtttttccat tagatggaag 2520

tattagatat actcaatggg aagatattgc acgaaaaaat cttcctaagc ctttacctga 2580

tttagatatg gtagcacaac aatttagaaa atggttaaaa aataaaaaca tttcatcaaa 2640

cagtattaat gttttgtcca tttttaagaa attttgtgaa aaatacagaa tagaatttaa 2700

taattaa 2707

<210> 12
<211> 668
<212> DNA
<213> Escherichia coli

<220>
<223> Origen of replication E. coli (pBR322)

<400> 12
gtagaaaaga tcaaaggatc ttcttgagat cctttttttc tgcgcgtaat ctgctgcttg 60

caaacaaaaa aaccaccgct accagcggtg gtttgtttgc cggatcaaga gctaccaact 120

ctttttccga aggtaactgg cttcagcaga gcgcagatac caaatactgt ccttctagtg 180

tagccgtagt taggccacca cttcaagaac tctgtagcac cgcctacata cctcgctctg 240

ctaatcctgt taccagtggc tgctgccagt ggcgataagt cgtgtcttac cgggttggac 300

tcaagacgat agttaccgga taaggcgcag cggtcgggct gaacgggggg ttcgtgcaca 360

cagcccagct tggagcgaac gacctacacc gaactgagat acctacagcg tgagctatga 420

gaaagcgcca cgcttcccga agggagaaag gcggacaggt atccggtaag cggcagggtc 480

ggaacaggag agcgcacgag ggagcttcca gggggaaacg cctggtatct ttatagtcct 540

gtcgggtttc gccacctctg acttgagcgt cgatttttgt gatgctcgtc aggggggcgg 600

agcctatgga aaaacgccag caacgcggcc tttttacggt tcctggcctt ttgctggcct 660

tttgctca 668

<210> 13
<211> 589
<212> DNA
<213> Escherichia coli

<220>
<223> colE1

<400> 13
ttgagatcct ttttttctgc gcgtaatctg ctgcttgcaa acaaaaaaac caccgctacc 60

agcggtggtt tgtttgccgg atcaagagct accaactctt tttccgaagg taactggctt 120

cagcagagcg cagataccaa atactgtcct tctagtgtag ccgtagttag gccaccactt 180

caagaactct gtagcaccgc ctacatacct cgctctgcta atcctgttac cagtggctgc 240

tgccagtggc gataagtcgt gtcttaccgg gttggactca agacgatagt taccggataa 300

ggcgcagcgg tcgggctgaa cggggggttc gtgcacacag cccagcttgg agcgaacgac 360

ctacaccgaa ctgagatacc tacagcgtga gctatgagaa agcgccacgc ttcccgaagg 420

gagaaaggcg gacaggtatc cggtaagcgg cagggtcgga acaggagagc gcacgaggga 480

gcttccaggg ggaaacgcct ggtatcttta tagtcctgtc gggtttcgcc acctctgact 540

tgagcgtcga tttttgtgat gctcgtcagg ggggcggagc ctatggaaa 589

<210> 14
<211> 870
<212> DNA
<213> Escherichia coli

<220>
<223> CamR

<400> 14
ttcaaatatg tatccgctca tgagacaata accctgataa atgcttcaat aatattgaaa 60

aaggaagagt atggaaaaaa aaatcaccgg ctataccacc gtggatatta gccagtggca 120

tcgtaaagaa cattttgaag cgtttcagag cgtggcgcag tgcacctata accagaccgt 180

gcagctggat atcaccgcgt ttctgaaaac cgtgaaaaaa aacaaacaca aattctaccc 240

ggcgtttatt catattctgg cccgtctgat gaacgcgcat ccggaatttc gtatggccat 300

gaaagatggc gaactggtga tttgggatag cgtgcatccg tgctataccg tgtttcatga 360

acagaccgaa acctttagca gcctgtggag cgaatatcat gatgattttc gccagttcct 420

gcatatttat agccaggatg tggcgtgcta tggcgaaaac ctggcctatt ttccgaaagg 480

cttcatcgaa aacatgttct ttgtgagcgc gaatccgtgg gtgagcttta ccagcttcga 540

tctgaacgtg gcgaacatgg ataacttttt tgcgccggtg tttaccatgg gcaaatatta 600

tacccagggc gataaagtgc tgatgccgct ggccattcag gtgcatcatg cggtgtgtga 660

tggctttcat gtgggccgta tgctgaacga actgcagcag tattgtgatg aatggcaggg 720

cggtgcgtaa taactgtcag accaagttta ctcatatata ctttagattg atttaaaact 780

tcatttttaa tttaaaagga tctaggtgaa gatccttttt gataatctca tgaccaaaat 840

cccttaacgt gagttttcgt tccactgagc 870

<210> 15
<211> 1068
<212> DNA
<213> Escherichia coli

<220>
<223> AmpR

<400> 15
ttcaaatatg tatccgctca tgagacaata accctgataa atgcttcaat aatattgaaa 60

aaggaagagt atgagtattc aacatttccg tgtcgccctt attccctttt ttgcggcatt 120

ttgccttcct gtttttgctc acccagaaac gctggtgaaa gtaaaagatg ctgaagatca 180

gttgggtgca cgagtgggtt acatcgaact ggatctcaac agcggtaaga tccttgagag 240

ttttcgcccc gaagaacgtt ttccaatgat gagcactttt aaagttctgc tatgtggcgc 300

ggtattatcc cgtattgacg ccgggcaaga gcaactcggt cgccgcatac actattctca 360

gaatgacttg gttgagtact caccagtcac agaaaagcat cttacggatg gcatgacagt 420

aagagaatta tgcagtgctg ccataaccat gagtgataac actgcggcca acttacttct 480

gacaacgatc ggaggaccga aggagctaac cgcttttttg cacaacatgg gggatcatgt 540

aactcgcctt gatcgttggg aaccggagct gaatgaagcc ataccaaacg acgagcgtga 600

caccacgatg cctgtagcaa tggcaacaac gttgcgcaaa ctattaactg gcgaactact 660

tactctagct tcccggcaac aattaataga ctggatggag gcggataaag ttgcaggacc 720

acttctgcgc tcggcccttc cggctggctg gtttattgct gataaatctg gagccggtga 780

gcgtggttct cgcggtatca ttgcagcact ggggccagat ggtaagccct cccgtatcgt 840

agttatctac acgacgggga gtcaggcaac tatggatgaa cgaaatagac agatcgctga 900

gataggtgcc tcactgatta agcattggta actgtcagac caagtttact catatatact 960

ttagattgat ttaaaacttc atttttaatt taaaaggatc taggtgaaga tcctttttga 1020

taatctcatg accaaaatcc cttaacgtga gttttcgttc cactgagc 1068

<210> 16
<211> 1005

<212> DNA
<213> Escherichia coli

<220>
<223> KanR

<400> 16
ttcaaatatg tatccgctca tgagacaata accctgataa atgcttcaat aatattgaaa 60

aaggaagagt atgattgaac aggatggcct gcatgcgggt agcccggcag cgtgggtgga 120

acgtctgttt ggctatgatt gggcgcagca gaccattggc tgctctgatg cggcggtgtt 180

tcgtctgagc gcgcagggtc gtccggtgct gtttgtgaaa accgatctga gcggtgcgct 240

gaacgagctg caggatgaag cggcgcgtct gagctggctg gccaccaccg gtgttccgtg 300

tgcggcggtg ctggatgtgg tgaccgaagc gggccgtgat tggctgctgc tgggcgaagt 360

gccgggtcag gatctgctgt ctagccatct ggcgccggca gaaaaagtga gcattatggc 420

ggatgccatg cgtcgtctgc ataccctgga cccggcgacc tgtccgtttg atcatcaggc 480

gaaacatcgt attgaacgtg cgcgtacccg tatggaagcg ggcctggtgg atcaggatga 540

tctggatgaa gaacatcagg gcctggcacc ggcagagctg tttgcgcgtc tgaaagcgag 600

catgccggat ggcgaagatc tggtggtgac ccatggtgat gcgtgcctgc cgaacattat 660

ggtggaaaat ggccgtttta gcggctttat tgattgcggc cgtctgggcg tggcggatcg 720

ttatcaggat attgcgctgg ccacccgtga tattgcggaa gaactgggcg gcgaatgggc 780

ggatcgtttt ctggtgctgt atggcattgc ggcaccggat agccagcgta ttgcgtttta 840

tcgtctgctg gatgaatttt tctaataact gtcagaccaa gtttactcat atatacttta 900

gattgattta aaacttcatt tttaatttaa aaggatctag gtgaagatcc tttttgataa 960

tctcatgacc aaaatccctt aacgtgagtt ttcgttccac tgagc 1005

<210> 17
<211> 792
<212> DNA
<213> Escherichia coli

<220>
<223> TetR

<400> 17
atgagggaag cggtgatcgc cgaagtatcg actcaactat cagaggtagt tggcgtcatc 60

gagcgccatc tcgaaccgac gttgctggcc gtacatttgt acggctccgc agtggatggc 120

ggcctgaagc cacacagtga tattgatttg ctggttacgg tgaccgtaag gcttgatgaa 180

acaacgcggc gagctttgat caacgacctt ttggaaactt cggcttcccc tggagagagc 240

gagattctcc gcgctgtaga agtcaccatt gttgtgcacg acgacatcat tccgtggcgt 300

tatccagcta agcgcgaact gcaatttgga gaatggcagc gcaatgacat tcttgcaggt 360

atcttcgagc cagccacgat cgacattgat ctggctatct tgctgacaaa agcaagagaa 420

catagcgttg ccttggtagg tccagcggcg gaggaactct ttgatccggt tcctgaacag 480

gatctatttg aggcgctaaa tgaaacctta acgctatgga actcgccgcc cgactgggct 540

ggcgatgagc gaaatgtagt gcttacgttg tcccgcattt ggtacagcgc agtaaccggc 600

aaaatcgcgc cgaaggatgt cgctgccgac tgggcaatgg agcgcctgcc ggcccagtat 660

cagcccgtca tacttgaagc tagacaggct tatcttggac aagaagaaga tcgcttggcc 720

tcgcgcgcag atcagttgga agaatttgtc cactacgtga aaggcgagat caccaaggta 780

gtcggcaaat aa 792

<210> 18
<211> 792
<212> DNA
<213> Escherichia coli

<220>
<223> SpectinomycinR

<400> 18
atgagggaag cggtgatcgc cgaagtatcg actcaactat cagaggtagt tggcgtcatc 60

gagcgccatc tcgaaccgac gttgctggcc gtacatttgt acggctccgc agtggatggc 120

ggcctgaagc cacacagtga tattgatttg ctggttacgg tgaccgtaag gcttgatgaa 180

acaacgcggc gagctttgat caacgacctt ttggaaactt cggcttcccc tggagagagc 240

gagattctcc gcgctgtaga agtcaccatt gttgtgcacg acgacatcat tccgtggcgt 300

tatccagcta agcgcgaact gcaatttgga gaatggcagc gcaatgacat tcttgcaggt 360

atcttcgagc cagccacgat cgacattgat ctggctatct tgctgacaaa agcaagagaa 420

catagcgttg ccttggtagg tccagcggcg gaggaactct ttgatccggt tcctgaacag 480

gatctatttg aggcgctaaa tgaaacctta acgctatgga actcgccgcc cgactgggct 540

ggcgatgagc gaaatgtagt gcttacgttg tcccgcattt ggtacagcgc agtaaccggc 600

aaaatcgcgc cgaaggatgt cgctgccgac tgggcaatgg agcgcctgcc ggcccagtat 660

cagcccgtca tacttgaagc tagacaggct tatcttggac aagaagaaga tcgcttggcc 720

tcgcgcgcag atcagttgga agaatttgtc cactacgtga aaggcgagat caccaaggta 780

gtcggcaaat aa 792

<210> 19
<211> 147
<212> DNA
<213> Zymomonas mobilis

<220>
<223> Leader sequence in the crispr plasmid

<400> 19
aatttgaccc ttattttgac cctctttttt cgagggtata aaaaatcctt tcgattcaat      60

atgttacata tgagcatatt ttttagggtt attttgcctt tttggcgaga tatcccttta     120

ttttaggggc aattctatct tttgcct                                         147


<210> 20
<211> 202
<212> DNA
<213> Escherichia coli


<220>
<223> Terminator

<400> 20
ataaaacgaa aggctcagtc gaaagactgg gcctttcgtt ttatctgttg tttgtcggtg      60

aacgctctcc tgagtaggac aaatccgccg ggagcggatt tgaacgttgc gaagcaacgg     120

cccggagggt ggcgggcagg acgcccgcca taaactgcca ggcatcaaat taagcagaag     180

gccatcctga cggatggcct tt                                              202


<210> 21
<211> 417
<212> DNA
<213> Zymomonas mobilis


<220>
<223> Lambda red gen 1

<400> 21
atggatatta atactgaaac tgagatcaag caaaagcatt cactaacccc ctttcctgtt      60

ttcctaatca gcccggcatt tcgcgggcga tattttcaca gctatttcag gagttcagcc     120

atgaacgctt attacattca ggatcgtctt gaggctcaga gctgggcgcg tcactaccag     180

cagctcgccc gtgaagagaa agaggcagaa ctggcagacg acatggaaaa aggcctgccc     240

cagcacctgt ttgaatcgct atgcatcgat catttgcaac gccacggggc cagcaaaaaa     300

tccattaccc gtgcgtttga tgacgatgtt gagtttcagg agcgcatggc agaacacatc     360

cggtacatgg ttgaaaccat tgctcaccac caggttgata ttgattcaga ggtataa        417


<210> 22
<211> 786
<212> DNA
<213> Zymomonas mobilis


<220>

<223> Lambda red gen 2

<400> 22
atgagtactg cactcgcaac gctggctggg aagctggctg aacgtgtcgg catggattct 60

gtcgacccac aggaactgat caccactctt cgccagacgg catttaaagg tgatgccagc 120

gatgcgcagt tcatcgcatt actgatcgtt gccaaccagt acggccttaa tccgtggacg 180

aaagaaattt acgcctttcc tgataagcag aatggcatcg ttccggtggt gggcgttgat 240

ggctggtccc gcatcatcaa tgaaaaccag cagtttgatg gcatggactt tgagcaggac 300

aatgaatcct gtacatgccg gatttaccgc aaggaccgta atcatccgat ctgcgttacc 360

gaatggatgg atgaatgccg ccgcgaacca ttcaaaactc gcgaaggcag agaaatcacg 420

gggccgtggc agtcgcatcc caaacggatg ttacgtcata aagccatgat tcagtgtgcc 480

cgtctggcct tcggatttgc tggtatctat gacaaggatg aagccgagcg cattgtcgaa 540

aatactgcat acactgcaga acgtcagccg gaacgcgaca tcactccggt taacgatgaa 600

accatgcagg agattaacac tctgctgatc gccctggata aaacatggga tgacgactta 660

ttgccgctct gttcccagat atttcgccgc gacattcgtg catcgtcaga actgacacag 720

gccgaagcag taaaagctct tggattcctg aaacagaaag ccgcagagca gaaggtggca 780

gcatga 786

<210> 23
<211> 681
<212> DNA
<213> Zymomonas mobilis

<220>
<223> Lambda red gen 3

<400> 23
atgacaccgg acattatcct gcagcgtacc gggatcgatg tgagagctgt cgaacagggg 60

gaagatgcgt ggcacaaatt acggctcggc gtcatcaccg cttcagaagt tcacaacgtg 120

atagcaaaac cccgctccgg aaagaagtgg cctgacatga aaatgtccta cttccacacc 180

ctgcttgctg aggtttgcac cggtgtggct ccggaagtta acgctaaagc actggcctgg 240

ggaaaacagt acgagaacga cgccagaacc ctgtttgaat tcacttccgg cgtgaatgtt 300

actgaatccc cgatcatcta tcgcgacgaa agtatgcgta ccgcctgctc tcccgatggt 360

ttatgcagtg acggcaacgg ccttgaactg aaatgcccgt ttacctcccg ggatttcatg 420

aagttccggc tcggtggttt cgaggccata aagtcagctt acatggccca ggtgcagtac 480

agcatgtggg tgacgcgaaa aaatgcctgg tactttgcca actatgaccc gcgtatgaag 540

cgtgaaggcc tgcattatgt cgtgattgag cgggatgaaa agtacatggc gagttttgac 600

gagatcgtgc cggagttcat cgaaaaaatg gacgaggcac tggctgaaat tggttttgta 660

tttggggagc aatggcgatg a                                              681

<210> 24
<211> 4107
<212> DNA
<213> Streptococcus pyogenes

<220>
<223> Cas9

<400> 24
atggataaga aatactcaat aggcttagat atcggcacaa atagcgtcgg atgggcggtg      60

atcactgatg aatataaggt tccgtctaaa aagttcaagg ttctgggaaa tacagaccgc     120

cacagtatca aaaaaaatct tataggggct cttttatttg acagtggaga gacagcggaa     180

gcgactcgtc tcaaacggac agctcgtaga aggtatacac gtcggaagaa tcgtatttgt     240

tatctacagg agattttttc aaatgagatg gcgaaagtag atgatagttt ctttcatcga     300

cttgaagagt cttttttggt ggaagaagac aagaagcatg aacgtcatcc tatttttgga     360

aatatagtag atgaagttgc ttatcatgag aaatatccaa ctatctatca tctgcgaaaa     420

aaattggtag attctactga taaagcggat ttgcgcttaa tctatttggc cttagcgcat     480

atgattaagt ttcgtggtca ttttttgatt gagggagatt taaatcctga taatagtgat     540

gtggacaaac tatttatcca gttggtacaa acctacaatc aattatttga agaaaaccct     600

attaacgcaa gtggagtaga tgctaaagcg attctttctg cacgattgag taaatcaaga     660

cgattagaaa atctcattgc tcagctcccc ggtgagaaga aaaatggctt atttgggaat     720

ctcattgctt tgtcattggg tttgacccct aattttaaat caaattttga tttggcagaa     780

gatgctaaat tacagctttc aaaagatact tacgatgatg atttagataa tttattggcg     840

caaattggag atcaatatgc tgatttgttt ttggcagcta agaatttatc agatgctatt     900

ttactttcag atatcctaag agtaaatact gaaataacta aggctcccct atcagcttca     960

atgattaaac gctacgatga acatcatcaa gacttgactc ttttaaaagc tttagttcga    1020

caacaacttc cagaaaagta taaagaaatc ttttttgatc aatcaaaaaa cggatatgca    1080

ggttatattg atgggggagc tagccaagaa gaattttata aatttatcaa accaatttta    1140

gaaaaaatgg atggtactga ggaattattg gtgaaactaa atcgtgaaga tttgctgcgc    1200

aagcaacgga cctttgacaa cggctctatt ccccatcaaa ttcacttggg tgagctgcat    1260

gctattttga gaagacaaga agacttttat ccatttttaa aagacaatcg tgagaagatt    1320

gaaaaaatct tgacttttcg aattccttat tatgttggtc cattggcgcg tggcaatagt    1380

cgttttgcat ggatgactcg gaagtctgaa gaaacaatta ccccatggaa ttttgaagaa    1440

gttgtcgata aaggtgcttc agctcaatca tttattgaac gcatgacaaa ctttgataaa    1500

aatcttccaa atgaaaaagt actaccaaaa catagtttgc tttatgagta ttttacggtt 1560

tataacgaat tgacaaaggt caaatatgtt actgaaggaa tgcgaaaacc agcatttctt 1620

tcaggtgaac agaagaaagc cattgttgat ttactcttca aaacaaatcg aaaagtaacc 1680

gttaagcaat taaaagaaga ttatttcaaa aaaatagaat gttttgatag tgttgaaatt 1740

tcaggagttg aagatagatt taatgcttca ttaggtacct accatgattt gctaaaaatt 1800

attaaagata aagatttttt ggataatgaa gaaaatgaag atatcttaga ggatattgtt 1860

ttaacattga ccttatttga agatagggag atgattgagg aaagacttaa aacatatgct 1920

cacctctttg atgataaggt gatgaaacag cttaaacgtc gccgttatac tggttgggga 1980

cgtttgtctc gaaaattgat taatggtatt agggataagc aatctggcaa aacaatatta 2040

gattttttga aatcagatgg ttttgccaat cgcaatttta tgcagctgat ccatgatgat 2100

agtttgacat ttaaagaaga cattcaaaaa gcacaagtgt ctggacaagg cgatagttta 2160

catgaacata ttgcaaattt agctggtagc cctgctatta aaaaaggtat tttacagact 2220

gtaaaagttg ttgatgaatt ggtcaaagta atggggcggc ataagccaga aaatatcgtt 2280

attgaaatgg cacgtgaaaa tcagacaact caaaagggcc agaaaaattc gcgagagcgt 2340

atgaaacgaa tcgaagaagg tatcaaagaa ttaggaagtc agattcttaa agagcatcct 2400

gttgaaaata ctcaattgca aaatgaaaag ctctatctct attatctcca aaatggaaga 2460

gacatgtatg tggaccaaga attagatatt aatcgtttaa gtgattatga tgtcgatcac 2520

attgttccac aaagtttcct taaagacgat tcaatagaca ataaggtctt aacgcgttct 2580

gataaaaatc gtggtaaatc ggataacgtt ccaagtgaag aagtagtcaa aaagatgaaa 2640

aactattgga gacaacttct aaacgccaag ttaatcactc aacgtaagtt tgataattta 2700

acgaaagctg aacgtggagg tttgagtgaa cttgataaag ctggttttat caaacgccaa 2760

ttggttgaaa ctcgccaaat cactaagcat gtggcacaaa ttttggatag tcgcatgaat 2820

actaaatacg atgaaaatga taaacttatt cgagaggtta aagtgattac cttaaaatct 2880

aaattagttt ctgacttccg aaaagatttc caattctata aagtacgtga gattaacaat 2940

taccatcatg cccatgatgc gtatctaaat gccgtcgttg gaactgcttt gattaagaaa 3000

tatccaaaac ttgaatcgga gtttgtctat ggtgattata aagtttatga tgttcgtaaa 3060

atgattgcta agtctgagca agaaataggc aaagcaaccg caaaatattt cttttactct 3120

aatatcatga acttcttcaa aacagaaatt acacttgcaa atggagagat tcgcaaacgc 3180

cctctaatcg aaactaatgg ggaaactgga gaaattgtct gggataaagg gcgagatttt 3240

gccacagtgc gcaaagtatt gtccatgccc caagtcaata ttgtcaagaa aacagaagta 3300

cagacaggcg gattctccaa ggagtcaatt ttaccaaaaa gaaattcgga caagcttatt 3360

gctcgtaaaa aagactggga tccaaaaaaa tatggtggtt ttgatagtcc aacggtagct 3420

tattcagtcc tagtggttgc taaggtggaa aaagggaaat cgaagaagtt aaaatccgtt 3480

aaagagttac tagggatcac aattatggaa agaagttcct ttgaaaaaaa tccgattgac 3540

tttttagaag ctaaaggata taaggaagtt aaaaaagact taatcattaa actacctaaa 3600

tatagtcttt ttgagttaga aaacggtcgt aaacggatgc tggctagtgc cggagaatta 3660

caaaaaggaa atgagctggc tctgccaagc aaatatgtga attttttata tttagctagt 3720

cattatgaaa agttgaaggg tagtccagaa gataacgaac aaaaacaatt gtttgtggag 3780

cagcataagc attatttaga tgagattatt gagcaaatca gtgaattttc taagcgtgtt 3840

attttagcag atgccaattt agataaagtt cttagtgcat ataacaaaca tagagacaaa 3900

ccaatacgtg aacaagcaga aaatattatt catttattta cgttgacgaa tcttggagct 3960

cccgctgctt ttaaatattt tgatacaaca attgatcgta aacgatatac gtctacaaaa 4020

gaagttttag atgccactct tatccatcaa tccatcactg gtctttatga aacacgcatt 4080

gatttgagtc agctaggagg tgactaa 4107

<210> 25
<211> 36
<212> DNA
<213> Synthetic plasmid

<220>
<223> Pyruvate Decarboxylase_sgRNA_FW - BsaI

<400> 25
gaaatcaatg caggtgaact gaagaaagcc gctccg 36

<210> 26
<211> 36
<212> DNA
<213> Synthetic plasmid

<220>
<223> Pyruvate Decarboxylase_sgRNA_RV - Bsal

<400> 26
gaaccggagc ggctttcttc agttcacctg cattga 36

<210> 27
<211> 27
<212> DNA
<213> Synthetic plasmid

<220>
<223> Pyruvate Decarboxylase_HDR_FW_1 - SaII

<400> 27

cgtcgacgtt ggttatgtcc tataatc 27

<210> 28
<211> 30
<212> DNA
<213> Synthetic plasmid

<220>
<223> Pyruvate Decarboxylase_HDR_RV_1 - XhoI/SacI

<400> 28
ctcgaggagc tctgcttact ccatatattc 30

<210> 29
<211> 31
<212> DNA
<213> Synthetic plasmid

<220>
<223> Pyruvate Decarboxylase_HDR_FW_2 -SacI/XhoI

<400> 29
gagctcctcg agtttttaaa taaacttaga g 31

<210> 30
<211> 23
<212> DNA
<213> Synthetic plasmid

<220>
<223> Pyruvate Decarboxylase_HDR_RV_2 - KpNI

<400> 30
tggtacctta aggcactgac ttc 23

<210> 31
<211> 36
<212> DNA
<213> Synthetic plasmid

<220>
<223> Lactatedehydrogenase_sgRNA_FW - BsaI

<400> 31
gaaagaaaca attttaagtc gtgttgaaaa aggcga 36

<210> 32
<211> 36
<212> DNA
<213> Synthetic plasmid

<220>
<223> Lactatedehydrogenase_sgRNA_RV - BsaI

<400> 32
gaactcgcct ttttcaacac gacttaaaat tgtttc                                36

<210> 33
<211> 27
<212> DNA
<213> Synthetic plasmid

<220>
<223> Lactatedehydrogenase_HDR_FW_1 - SaII

<400> 33
cgtcgacgaa agatagctat tattatc                                          27

<210> 34
<211> 32
<212> DNA
<213> Synthetic plasmid

<220>
<223> Lactatedehydrogenase_HDR_RV_1 - XhoI/SacI

<400> 34
ctcgaggagc tctatatcga ccttttattt tc                                    32

<210> 35
<211> 29
<212> DNA
<213> Synthetic plasmid

<220>
<223> Lactatedehydrogenase_HDR_FW_2 - SacI/XhoI

<400> 35
gagctcctcg agttaagctg ctataaata                                        29

<210> 36
<211> 24
<212> DNA
<213> Synthetic plasmid

<220>
<223> Lactatedehydrogenase_HDR_RV_2 - KpNI

<400> 36
tggtacctct gtcgccagat cgac                                             24

<210> 37
<211> 36
<212> DNA
<213> Synthetic plasmid

<220>
<223> Phosphoenolpyruvatecarboxylase_sgRNA_FW - BsaI

<400> 37
gaaaatgaaa tttttggccc tgaatccatt acgact                                36

<210> 38
<211> 36
<212> DNA
<213> Synthetic plasmid

<220>
<223> Phosphoenolpyruvatecarboxylase_sgRNA_RV - BsaI

<400> 38
gaacagtcgt aatggattca gggccaaaaa tttcat                                36

<210> 39
<211> 22
<212> DNA
<213> Synthetic plasmid

<220>
<223> Phosphoenolpyruvatecarboxylase_HDR_FW_1 - SalI

<400> 39
cgtcgacttt atcgggaagc tg                                               22

<210> 40
<211> 28
<212> DNA
<213> Synthetic plasmid

<220>
<223> Phosphoenolpyruvatecarboxylase_HDR_RV_1 - XhoI/SacI

<400> 40
ctcgaggagc tcaaaccatc cctttgcc                                         28

<210> 41
<211> 33
<212> DNA
<213> Synthetic plasmid

<220>
<223> Phosphoenolpyruvatecarboxylase_HDR_FW_2 - SacI/XhoI

<400> 41
gagctcctcg agtacatcag ccctatttta aag                                   33

<210> 42
<211> 24
<212> DNA
<213> Synthetic plasmid

<220>
<223> Phosphoenolpyruvatecarboxylase_HDR_RV_2 - KpNI

<400> 42
tggtaccccg ccttgggctg tctg                                              24


<210> 43
<211> 33
<212> DNA
<213> Synthetic plasmid


<220>
<223> KnockIN_G1_FW - SacI

<400> 43
gtcatgagct catggcgctg gtttctatgc gtc                                    33


<210> 44
<211> 32
<212> DNA
<213> Synthetic plasmid


<220>
<223> KnockIN_G1_RV - Xhol

<400> 44
cagtactcga gttatttacg cggggtgtag tc                                     32


<210> 45
<211> 33
<212> DNA
<213> Synthetic plasmid


<220>
<223> KnockIN_G2_FW - Sac

<400> 45
gtcatgagct catgaaatct gttaccatcc tgg                                    33


<210> 46
<211> 31
<212> DNA
<213> Synthetic plasmid


<220>
<223> KnockIN_G2_RV - Xhol

<400> 46
cagtactcga gttacgcaga acggttcgcc g                                      31


<210> 47
<211> 33

<212> DNA
<213> Synthetic plasmid

<220>
<223> KnockIN_G3_FW - SacI

<400> 47
gtcatgagct catgacccgt gaagttgttg ttg                                    33

<210> 48
<211> 34
<212> DNA
<213> Synthetic plasmid

<220>
<223> KnockIN_G3_RV - Xhol

<400> 48
cagtactcga gttagtcttc cagcgcgtcg aacg                                   34

<210> 49
<211> 25
<212> DNA
<213> Synthetic plasmid

<220>
<223> SequencingPrimer_ ORF_FW

<400> 49
cttcctaagc ctttacctga tttag                                             25

<210> 50
<211> 20
<212> DNA
<213> Synthetic plasmid

<220>
<223> SequencingPrimer_ ORF_RV

<400> 50
cgactgagcc tttcgtttta                                                   20

<210> 51
<211> 24
<212> DNA
<213> Synthetic plasmid

<220>
<223> SequencingPrimer_ HDR_FW

<400> 51
tcatgaccaa aatcccttaa cgtg                                              24

<210> 52
<211> 24
<212> DNA
<213> Synthetic plasmid

<220>
<223> SequencingPrimer_HDR _RV

<400> 52
ccatgttttg agaagccgat aatc 24